# EUROPEAN PATENT SPECIFICATION

(11) **EP 3 868 413 B1**
(45) Date of publication and mention of the grant of the patent: **24.07.2024**
(21) Application number: 19873651.4
(22) Date of filing: 17.10.2019
(51) Int. Cl.: A61L 15/20, A61K 9/127, A61K 9/70, A61K 47/24, A61K 47/28, A61K 47/34, A61K 47/36, A61L 15/26, A61L 15/28, A61L 15/42, A61L 15/64

(54) **HEMOSTATIC MATERIAL**
HÄMOSTATISCHES MATERIAL
MATÉRIAU HÉMOSTATIQUE

(30) Priority: 17.10.2018 JP 2018196224
(43) Date of publication of application: 25.08.2021
(73) Proprietor: Toray Industries, Inc., Tokyo 103-8666 (JP); Nanotheta Co, Ltd., Tokyo 169-0051 (JP)
(72) Inventor: TAKEOKA, Shinji, Tokyo 162-8480 (JP); NAKAHARA, Keiko, Tokyo 162-8480 (JP); NISHIURA, Mamoru, Tokyo 162-8480 (JP); OTSUBO, Shinya, Tokyo 169-0051 (JP); KURUMATANI, Hajimu, Tokyo 103-8666 (JP); ARAKANE, Toru, Tokyo 103-8666 (JP); TAKEDA, Masanobu, Otsu-shi, Shiga 520-2141 (JP); NAKAHARA, Makoto, Otsu-shi, Shiga 520-2141 (JP); OYAMA, Kumi, Otsu-shi, Shiga 520-2141 (JP)
(74) Representative: Kador & Partner Part mbB
(86) International application number: PCT/JP2019/040985
(87) International publication number: WO 2020/080496

(56) References cited:
- JP-A- 2007 091 625
- JP-A- 2008 523 149
- JP-A- 2015 128 601
- JP-A- H0 840 928
- JP-A- H10 511 019
- US-A- 4 610 880
- US-A1- 2014 046 277
- SEIICHI FURUTA: "SYMPOSIUM ON THE CLINICAL SIGNIFICANCE OF BLOOD COAGULATION (7) ARTERIOSCLEROSIS WITH REFERENCE TO BLOOD COAGULATION", NIHON NAIKA GAKKAI ZASSHI, vol. 54, no. 8, 1965, pages 882 - 887, XP055702885, ISSN: 0021-5384

## Description

### BACKGROUND OF THE INVENTION

### Field of the Invention

The present invention relates to a hemostatic material.

### Background Art

Platelets play a central role in hemostasis, and adhesion of platelets in blood to blood vessels or aggregation of platelets in blood acts as an important trigger for hemostasis. In order to compensate for decreased platelet counts or platelet dysfunction, or to prepare for massive bleeding, a pseudo-platelet (platelet substitute) is often attempted to be artificially produced. As such a platelet substitute, for example, a lipid microparticle carrying a protein involved in adhesion to blood vessel walls or platelet-platelet aggregation that exists on the platelet membrane surface, a protein that mediates platelet-platelet aggregation, or a peptide corresponding to an active site of such a protein has been attempted to be produced. Since the system of GPIb, which is a glycoprotein existing on a membrane surface, and von Willebrand factor (vWF), which is a plasma protein, or the system of GPIIb/III and fibrinogen plays a central role in adhesion or aggregation of platelets, it is known that a lipid particle having GPIb on the surface (Patent Document 1), a lipid particle carrying a fibrinogen-derived dodecapeptide (H12) on the surface (Patent Document 2, Non-Patent Document 1 and Non-Patent Document 2), etc., can be used as a substitute for platelets.

Meanwhile, it is known that both of a lipid particle including a carboxylic acid-type lipid, a phospholipid and cholesterol and not having the H12 peptide on the surface and a lipid particle including a carboxylic acid-type lipid, a phospholipid and cholesterol and having the H12 peptide on the surface have a platelet aggregation accelerating effect, while the lipid particle not having the H12 peptide on the surface has a smaller platelet aggregation accelerating effect compared with the lipid particle having the H12 peptide on the surface (Non-Patent Document 2).

Further known in the art is drug delivering material for use as a surgical implant, dressing, or suture comprising a fibrous carrier and microparticles of a pharmaceutical coated with a lipid adjuvant for adhering said microparticles to said carrier, characterized in that said fibrous carrier consists essentially of fibers having diameters of between 0.1 micrometers to 100 micrometers coated with an amphiphatic lipid membrane, said adhering microparticles being solid microparticles of between 20 nanometers to 20 micrometers. said lipid is selected from lecithin, phosphatidic acid, phosphatidyl serine, phosphatidyl inositol, cardiolipin, phosphatidyl glycerol, phosphatidyl ethanolamine, sphingomyelin, monoglycerides, long-chain alkylamines, fatty acids cholesterol, triglycerides solid at room temperature, diglycerides solid at room temperature waxes, and combinations thereof (Patent Document 3).

### [Prior Art Document]

### [Patent Document]

[Patent Document 1] WO 01/064743
[Patent Document 2] JP 2005-239549 A
[Patent Document 3] JP H10 511019 A

### [Non-Patent Document]

[Non-Patent Document 1] Proceedings of the 32nd Annual Meeting of the Japanese Society for Biomaterials, p.324
[Non-Patent Document 2] Proceedings of the 33rd Annual Meeting of the Japanese Society for Biomaterials, p.319

### SUMMARY OF THE INVENTION

An object of the present invention is to provide a hemostatic material comprising a lipid that can accelerate adhesion or aggregation of platelets even if the lipid does not carry a protein involved in adhesion or aggregation of platelets, such as GPIb and H12, or a peptide corresponding to an active site thereof.

In order to solve the abovementioned problems, the present invention provides the following inventions.

[1] A hemostatic material, comprising a water-insoluble base and a lipid supported on a surface of the base, wherein the lipid comprises one or two or more anionic lipids,
   wherein the one or two or more anionic lipids comprise one or two or more carboxylic acid-type lipids selected from carboxylic acid-type lipids represented by the following formulas (III) to (VI): 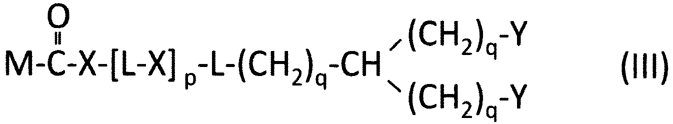 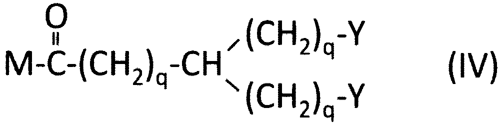 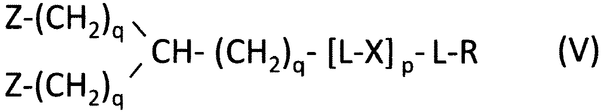 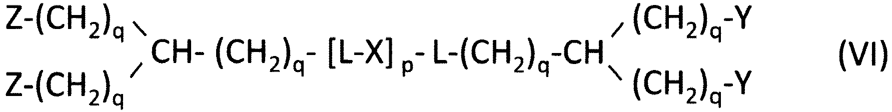
   wherein, in formulas (III) to (VI),
   M is the same or different and represents HO- or M₀-NH-,
   M₀ is the same or different and represents an amino acid residue, an amino acid derivative residue, a peptide residue or a salt thereof, wherein the amino acid residue, the amino acid derivative residue, the peptide residue and the salt thereof can be negatively charged at physiological pH,
   R is the same or different and represents a hydrocarbon group having 8 to 30 carbon atoms,
   L is the same or different and represents -CO-O-, -O-CO-, -CO-NH-, -NH-CO-, -CO-S-, -S-CO- or -S-S-,
   X is the same or different and represents a hydrocarbon group having 1 to 6 carbon atoms,
   p is the same or different and represents an integer of 0 to 4,
   q is the same or different and represents an integer of 0 to 8,
   Y is the same or different and represents a branched chain composed of a branched chain body and one or more groups Y2 that are bonded to the branched chain body, or represents a straight chain composed of one group Y2, wherein the branched chain body is composed of one or more units Y1, wherein each unit Y1 is represented by the following formula (VII): 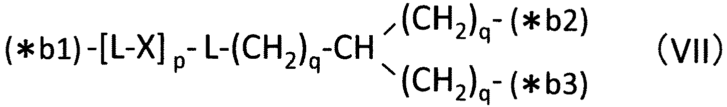 and wherein each group Y2 is represented by the following formula (VIII):

      (*b4)-[L-X]ₚ-L-R (VIII)
   wherein, in formulas (VII) and (VIII),
   R, L, X, p and q are the same as defined above,
   (*b1), (*b2) and (*b3) represent a bond of each unit Y1,
   (*b4) represents a bond of each group Y2,
   the bond (*b1) of each unit Y1 is bonded to (CH₂)_{q} in formula (III), (IV) or (VI), or is bonded to a bond (*b2) or (*b3) of another unit Y1 constituting the branched chain body, and
   the bond (*b4) of each group Y2 is bonded to (CH₂)_{q} in formula (III), (IV) or (VI), or is bonded to a bond (*b2) or (*b3) of any unit Y1 constituting the branched chain body,
   Z is the same or different and represents a branched chain composed of a branched chain body and one or more groups Z2 that are bonded to the branched chain body, or represents a straight chain composed of one group Z2, wherein the branched chain body is composed of one or more units Z1, wherein each unit Z1 is represented by the following formula (IX): 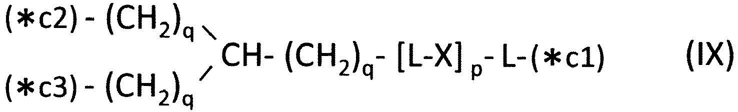 and wherein each group Z2 is selected from groups represented by the following formulas (X) and (XI): 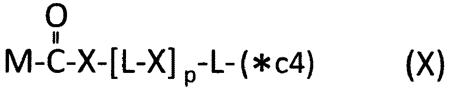 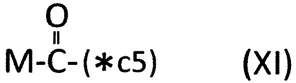
   wherein, in formulas (IX), (X) and (XI),
   M, L, X, p and q are the same as defined above,
   (*c1), (*c2) and (*c3) represent a bond of each unit Z1,
   (*c4) and (*c5) represent a bond of each group Z2,
   the bond (*c1) of each unit Z1 is bonded to (CH₂)_{q} in formula (V) or (VI), or is bonded to a bond (*c2) or (*c3) of another unit Z1 constituting the branched chain body, and
   the bond (*c4) or (*c5) of each group Z2 is bonded to (CH₂)_{q} in formula (V) or (VI), or is bonded to a bond (*c2) or (*c3) of any unit Z1 constituting the branched chain body.
[2] The hemostatic material according to [1], wherein the base is a porous base, and the lipid is supported on a surface of a pore of the porous base.
[3] The hemostatic material according to [2], wherein the lipid accounts for at least a part of the pore of the porous base.
[4] The hemostatic material according to [2] or [3], wherein the porous base is a fiber base.
[5] The hemostatic material according to any one of [1] to [4], wherein the amino acid residue represented by M₀ is an acidic amino acid residue or a neutral amino acid residue.
[6] The hemostatic material according to [5], wherein the acidic amino acid residue is an aspartic acid residue or a glutamic acid residue.
[7] The hemostatic material according to any one of [1] to [4], wherein the residue of the amino acid derivative represented by M₀ is a residue of a basic amino acid derivative, and an introduced derivatization that the basic amino acid derivative comprises is amidation of an amino group of a side chain of a basic amino acid to a group represented by the following formula: -NH-CO-R₁ wherein -NH- is derived from the amino group of the side chain of the basic amino acid, and R₁ represents a hydrocarbon group.
[8] The hemostatic material according to any one of [1] to [4], wherein the peptide residue represented by M₀ is a peptide residue comprising one or two or more acidic amino acid residues.
[9] The hemostatic material according to [8], wherein the peptide residue represented by M₀ is a peptide residue comprising two or more acidic amino acid residues selected from an aspartic acid residue and a glutamic acid residue.
[10] The hemostatic material according to [9], wherein the peptide residue represented by M₀ is a peptide residue consisting of 2 to 12 acidic amino acid residues selected from an aspartic acid residue and a glutamic acid residue
[11] The hemostatic material according to [10], wherein the peptide residue represented by M₀ is a peptide residue represented by the following formula (XII): 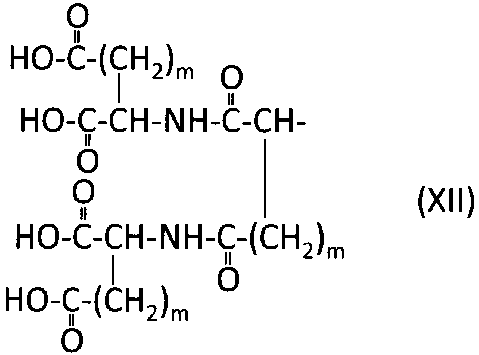 wherein m is the same or different and represents 1 or 2.
[12] The hemostatic material according to any one of [1] to [11], wherein Y is selected from straight and branched chains represented by the following formulas (XIII), (XIV), (XV) and (XVI): 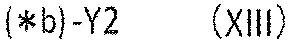 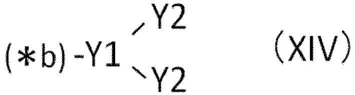 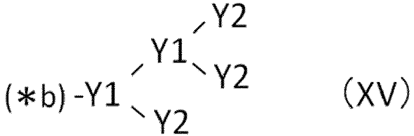 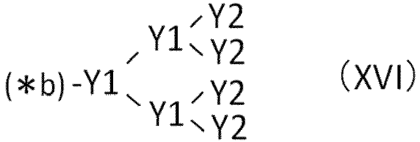
   wherein, in formulas (XIII) to (XVI),
   Y1 represents one unit Y1,
   Y2 represents one group Y2, and
   (*b) represents a bond of the unit Y1 bonded to (CH₂)_{q} in formula (III), (IV) or (VI).
[13] The hemostatic material according to any one of [1] to [12], wherein Z is selected from straight and branched chains represented by the following formulas (XVII), (XVIII), (XIX) and (XX): 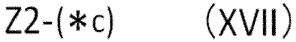 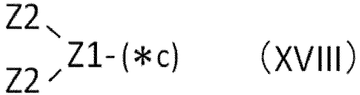 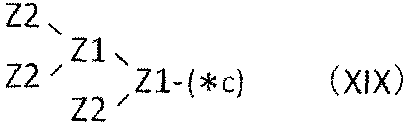 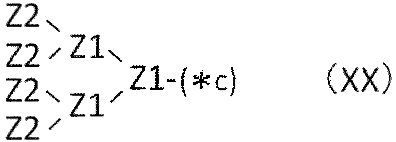
   wherein, in formulas (XVII) to (XX),
   Z1 represents one unit Z1,
   Z2 represents one group Z2, and
   (*c) represents a bond of the unit Z1 bonded to (CH₂)_{q} in formula (V) or (VI).
[14] The hemostatic material according to any one of [1] to [13], wherein the one or two or more anionic lipids comprise one or two or more lipids selected from a phospholipid and a sterol.
[15] The hemostatic material according to [1] to [14], wherein the lipid is supported on a surface of the base in one or two or more forms selected from a lipid particle, a lipid particle aggregate and a lipid membrane.

### Effects of the Invention

According to the present invention, there is provided a hemostatic material comprising a lipid that can accelerate adhesion and/or aggregation of platelets even if the lipid does not carry a protein involved in adhesion or aggregation of platelets, such as GPIb and H12, or a peptide corresponding to an active site of the protein. The lipid of the present invention comprises an anionic lipid that is negatively charged at physiological pH, and thus is negatively charged at physiological pH. Without carrying a known protein constituting the GPIb-vWF system or the GPIIb/III-fibrinogen system, or a peptide that is an active site of the protein, the lipid of the present invention can accelerate adhesion and/or aggregation of platelets by binding to a plurality of platelets. Accordingly, the hemostatic material of the present invention utilizes the platelet adhesion accelerating effect and/or the platelet aggregation accelerating effect of the lipid of the present invention, and thus exerts a potent hemostatic effect, which has not been possessed by conventional hemostatic materials.

### BRIEF DESCRIPTION OF THE DRAWINGS

FIG. 1 is a sectional view schematically showing a hemostatic material according to one embodiment of the present invention.
FIG. 2 is an enlarged view of a region represented by the sign S in FIG. 1.
FIG. 3A shows observation results of fluorescently labeled liposomes in platelet aggregates (fluorescence micrographs of platelet aggregates obtained by using a DiD-labeled liposome dispersion liquid).
FIG. 3B shows observation results of fluorescently labeled liposomes in platelet aggregates (fluorescence micrographs of platelet aggregates obtained by using a DiO-labeled liposome dispersion liquid).
FIG. 3C shows observation results of fluorescently labeled liposomes in platelet aggregates (fluorescence micrographs of platelet aggregates obtained by using a DiD-labeled liposome dispersion liquid).
FIG. 4 shows the amount of bleeding when hemostasis was performed using a hemostatic material fabricated by supporting a liposome on a collagen base.
FIG. 5 shows the hemostasis time when hemostasis was performed using a hemostatic material fabricated by supporting a liposome on a collagen base.
FIG. 6 shows the platelet attachment rate when hemostasis was performed using a hemostatic material fabricated by supporting a liposome on a collagen base.
FIG. 7 shows results on evaluation of the platelet aggregation capacity of a hemostatic material (in vitro).
FIG. 8 shows results on evaluation of the platelet aggregation capacity of a hemostatic material (*in vitro*).
FIG. 9 shows an SEM observation image of a base.
FIG. 10 shows an SEM observation image of DPPA supported on a base.
FIG. 11 shows an SEM observation image of DHSG supported on a base.
FIG. 12 shows an SEM observation image of Asp-DHSG supported on a base.
FIG. 13 shows an SEM observation image of Glu-DHSG supported on a base.
FIG. 14 shows an SEM observation image of AG-DHSG supported on a base.
FIG. 15 shows an SEM observation image of DMPS supported on a base.
FIG. 16 shows an SEM observation image of DSPG supported on a base.
FIG. 17 shows an SEM observation image of DHSG supported on a base (an enlarged view of FIG. 11).
FIG. 18 shows an SEM observation image of Asp-DHSG supported on a base (an enlarged view of FIG. 12).
FIG. 19 shows an SEM observation image of Glu-DHSG supported on a base (an enlarged view of FIG. 13).
FIG. 20 shows an SEM observation image of AG-DHSG supported on a base (an enlarged view of FIG. 14).

### DETAILED DESCRIPTION OF THE INVENTION

The present invention will be described in detail. As used herein, "numerical value A to numerical value B" means numerical value A or more and numerical value B or less.

### Hemostatic Material

The hemostatic material of the present invention comprises a water-insoluble base and a lipid supported on a surface of the base, wherein the lipid comprises one or two or more anionic lipids. The hemostatic material of the present invention may comprise a support member that supports the base, as necessary.

When hemostasis is performed using the hemostatic material of the present invention, for example, the hemostatic material of the present invention is attached to an affected site (bleeding site) so that the surface of the base comes into contact with the affected site. Blood bled from the affected site comes into contact with the lipid supported on the surface of the base. When the lipid supported on the surface of the base comes into contact with blood, the anionic lipid included in the lipid supported on the surface of the base becomes negatively charged. The negatively charged anionic lipid can bind to a plurality of platelets (particularly, activated platelets) and can accelerate adhesion and/or aggregation of platelets, and in turn can accelerate blood coagulation. As a result of this, the hemostatic material of the present invention can accelerate the hemostatic effect of blood.

In an embodiment in which a porous base, particularly a fiber base, is used as a base, the hemostatic effect of the hemostatic material of the present invention is particularly effectively exerted. Specifically, as a result of the fact that a lipid having a platelet adhesion accelerating effect and/or a platelet aggregation accelerating effect is supported on the porous base, in other words, the fact that a lipid has a platelet adhesion accelerating effect and/or a platelet aggregation accelerating effect and such lipid is supported on the porous base, the lipid effectively acts on platelets in blood that permeate pores of the porous base, and thus the hemostatic effect of the hemostatic material of the present invention is particularly effectively exerted.

Conventionally, many hemostatic materials using a substance having a platelet activating effect, such as collagen and gelatin, as a base itself have been used. However, such method requires that the platelet counts or platelet functions of a patient are sufficient. In contrast, in the hemostatic material of the present invention, a base and a lipid supported on the base become a place of adhesion and/or aggregation of platelets, and thus play a role instead of platelets. Therefore, the hemostatic material of the present invention can also be effectively used in a patient who has lower platelet counts due to extensive bleeding and the like, a patient who has lower platelet functions, or conversely, a patient in whom activation of platelets within the living body excessively occurs, resulting in decreased counts of normal platelets in blood, and platelet aggregation becomes difficult to occur, etc.

In an embodiment in which a porous base, particularly a fiber base, is used as a base, a porous base, particularly a fiber base, becomes not only a scaffold that supports a lipid including an anionic lipid, but also a place where activated platelets adhere, and plays a role in stably holding platelet aggregates. Aggregates of platelets (platelet thrombi) aggregated via the lipid supported on the base are held by being entangled in the base, resulting in earlier formation of stable fibrin clots, thus providing a rapid and potent hemostatic effect, which has not been possessed by conventional bases.

Thus, the hemostatic material of the present invention is useful in laparoscopic surgery in which a hemostatic effect is not sufficiently obtained by conventional hemostatic materials due to difficulty of compression, in extracorporeal circulation that requires use of a large amount of anticoagulants such as heparin, and also in a patient who is taking antiplatelet agents.

### <Base>

The hemostatic material of the present invention comprises a water-insoluble base.

The water-insoluble base has a nature of not dissolving for preferably 5 minutes or more, more preferably 10 minutes or more, and still more preferably 20 minutes or more in a state of keeping in contact with blood.

A material of the base is not particularly limited as long as it is insoluble and can support a lipid. The material of the base is preferably a biodegradable material. Biodegradability means a nature of being decomposed, dissolved, absorbed or metabolized within a living body, or a nature of eliminated from within a living body to the outside of the body. Examples of the decomposition reaction include hydrolysis, enzymatic decomposition, microbial decomposition and the like. Examples of the biodegradable material include a biodegradable polymer and the like.

Examples of the biodegradable polymer include homopolymers such as polylactic acid, polyethylene glycol, polyglycolic acid, polycaprolactone and polydioxanone; lactic acid copolymers such as a lactic acid-glycolic acid copolymer and a lactic acid-caprolactone copolymer; aliphatic polyesters such as polyglycerol sebacate, polyhydroxyalkanoate and polybutylene succinate; polysaccharides such as guar gum, pullulan, carrageenan, agarose, cellulose, oxidized cellulose, chitin, chitosan and glucosaminoglycan; proteins such as collagen and gelatin; and denatured products thereof and the like. Regarding these biodegradable polymers, one biodegradable polymer may be used alone, or two or more biodegradable polymers may be used in combination.

It is also of course possible to carry a protein that accelerates blood coagulation on the base. Representative examples of such protein include fibrinogen, vWF, fibronectin, vitronectin, thrombin, blood coagulation factor Xa and the like, and particularly, fibrinogen or thrombin is preferably used.

A monomer in polylactic acid and the lactic acid copolymer may be either L-lactic acid or D-lactic acid, and L-lactic acid is preferable.

The weight average molecular weight of the biodegradable polymer is preferably 3,000 to 2,000,000, and more preferably 30,000 to 1,000,000.

The biodegradable polymer has preferably high purity. Specifically, it is preferable that additives, plasticizers and residues (such as remaining catalysts, remaining monomers, and residual solvents used in molding processing and postprocessing) included in the biodegradable polymer are few. Particularly, regarding substances for which the safety standard value is specified in the medical field, it is preferable that the content is suppressed to less than the standard value.

The base is preferably a base having a larger surface area (specific surface area) per unit mass in order to increase the amount of the lipid supported on the surface of the base. Examples of the base having a larger specific surface area include a porous base and the like.

The porous base is a base having many pores. The surface of the porous base includes an inner surface (pore surface) in addition to an outer surface. The pore may be a through pore that penetrates the base, or may be a non-through pore that does not penetrates the base. The porous base may have one or both of the through pore and the non-through pore. A plurality of pores may be communicated. In particular, when the pore is a through pore, particularly preferable results are obtained. The pore may be any one of a micropore, a mesopore and a macropore. The size of the pore (pore diameter) is not particularly limited as long as the lipid can be supported on the surface of the pore, and can be appropriately adjusted according to the form of the lipid (e.g., particle diameter, etc., when the form of the lipid is a particle, and membrane thickness, etc., when the form of the lipid is a membrane). The size of the pore is preferably a size such that capillary action that makes blood permeate the inside of the porous base occurs when the porous base comes into contact with blood. The pore of the porous base is hereinafter sometimes referred to as void of the porous base.

The specific surface area of the porous base is preferably 0.3 to 15.0 m²/g, more preferably 0.5 to 10.0 m²/g, and still more preferably 0.7 to 7.0 m²/g. The specific surface area can be measured by, for example, the BET method.

The porosity of the porous base is preferably 30 to 99.9%, more preferably 50 to 99.8%, and still more preferably 60 to 99.7%. The porosity can be measured, for example, as follows. A cross section in the thickness direction is cut with an ion milling system (e.g., model IM4000 manufactured by Hitachi High-Technologies Corporation, an equivalent product thereof, etc.), and observed with a scanning electron microscope (SEM). The void part and the non-void part in contact with the cross section are binarized, and the area ratio of the area of the void part to the whole area can be defined as porosity (%).

Examples of the porous base include a fiber base, a sponge and the like, and of these, a fiber base is preferable.

The fiber base is a formed structure of a fiber material. The fiber base is preferably a fiber sheet. Examples of the fiber base include paper, a nonwoven fabric, a woven fabric, a knitted fabric and the like. The nonwoven fabric also includes a nonwoven fabric in which fibers are interlaced by interlacing treatment such as needlepunch and water stream, and a web-like nonwoven fabric that is not subjected to interlacing treatment. Examples of the fiber constituting the fiber base include a biodegradable polymer fiber, a synthetic resin fiber and the like. The description on the biodegradable polymer constituting the biodegradable polymer fiber is the same as mentioned above. Examples of the synthetic resin constituting the synthetic resin fiber include polyolefin resins such as a polyethylene resin, a polypropylene resin, a polymethylpentene resin and an olefin-based thermoplastic elastomer; vinyl-based resins such as a polyvinyl chloride resin, a polyvinylidene chloride resin, a polyvinyl alcohol resin, a vinyl chloride-vinyl acetate copolymer resin, an ethylene-vinyl acetate copolymer resin and an ethylene-vinyl alcohol copolymer resin; polyester resins such as a polyethylene terephthalate resin, a polybutylene terephthalate resin, a polyethylene naphthalate-isophthalate copolymer resin and a polyester-based thermoplastic elastomer; acrylic resins such as a polymethyl methacrylate resin, a polyethyl methacrylate resin and a polybutyl methacrylate resin; polyamide resins typified by nylon 6 or nylon 66, etc.; cellulose-based resins such as a cellulose triacetate resin and cellophane; a polystyrene resin; a polycarbonate resin; a polyarylate resin; a polyimide resin and the like.

The diameter of the fiber constituting the fiber base is preferably 0.2 to 10 µm, more preferably 0.3 to 6.0 µm, and still more preferably 0.5 to 3.0 µm. The diameter of the fiber is a diameter of a fiber cross section. The shape of the fiber cross section is not limited to a circle, and may be an ellipse, etc. When the fiber cross section is an ellipse, the mean of the length in the long axis direction and the length in the short axis direction of the ellipse is defined as the diameter of the fiber cross section. When the fiber cross section is not a circle or an ellipse, the diameter of the fiber cross section may be calculated by approximating the fiber cross section to a circle or an ellipse. The diameter of the fiber cross section can be measured by, for example, processing a photograph taken by a scanning electron microscope with image processing software (WINROOF (registered trademark)).

The fiber base can be formed by, for example, the electrospinning method, the spunbond method, the melt-blown method and the like. Of these methods, the electrospinning method (electrostatic spinning method, the electrospray method) or the melt-blown method is preferably used. The electrospinning method is a method in which a high voltage is applied to a polymer solution obtained by dissolving a polymer in a solvent, and then the charged polymer solution is ejected to perform spinning. The step includes a step of dissolving a polymer in a solvent to prepare a polymer solution, a step of applying a high voltage to the polymer solution, a step of ejecting the polymer solution and a step of evaporating the solvent from the ejected polymer solution to form a fiber.

The basis weight (weight per unit area) of the fiber base is preferably 5 to 200 g/m², more preferably 10 to 100 g/m², and still more preferably 15 to 50 g/m². The basis weight is measured in accordance with JIS L 1913:1998 6.2.

The thickness of the fiber base is preferably 0.03 to 10 mm, more preferably 0.05 to 5 mm, and still more preferably 0.1 to 3 mm. The thickness of the fiber base is measured at no load. When the thickness of the fiber base is different between regions, it is preferable that both of the minimum thickness and the maximum thickness are within the above range.

### <Support Member>

The hemostatic material of the present invention may comprise a support member that supports the base. The support member is a member that is provided as necessary. In terms of improving the ease in handling of the hemostatic material of the present invention, the hemostatic material of the present invention preferably comprises a support member that supports the base.

The support member is preferably water-insoluble. In the case in which the support member is water-insoluble, the form of the hemostatic material of the present invention is easily held when the hemostatic material of the present invention is used. The water-insoluble support member has a nature of not dissolving for preferably 5 minutes or more, more preferably 10 minutes or more, and still more preferably 20 minutes or more in a state of keeping in contact with blood.

The support member preferably has a liquid absorption property. The liquid absorption property is preferably a nature combining absorbability that absorbs a liquid such as blood with a liquid retention property that retains the absorbed liquid. The fact that the support member has a liquid absorption property is useful for improving the hemostatic capacity of the hemostatic material of the present invention.

When the hemostatic material of the present invention comprises the support member, the base is laminate on the support member directly or via other layers. When the support member has a liquid absorption property, it is preferable that the base and the support member are communicated so that blood permeating from the base side is absorbed by the support member.

The support member may be biodegradable or non-biodegradable. Examples of the biodegradable material include a biodegradable polymer and the like. The description on the biodegradable polymer is the same as mentioned above. Examples of the non-biodegradable material include celluloses, a synthetic resin and the like. Examples of the celluloses include celluloses (cellulose or cellulose derivatives) such as cellulose, carboxymethylcellulose, cellulose acylate (e.g., cellulose triacetate, cellulose diacetate, etc.) and lignocellulose. The description on the synthetic resin is the same as mentioned above.

Examples of the support member (particularly, support member having a liquid absorption property) include a fiber base, a sponge, a high-absorbent resin such as cross-linked sodium polyacrylate and the like. The description on the fiber base is the same as mentioned above.

It is also of course possible to carry a protein that accelerates blood coagulation on the support member. Representative examples of such protein include fibrinogen, vWF, fibronectin, vitronectin, thrombin, blood coagulation factor Xa and the like, and particularly, fibrinogen or thrombin is preferably used.

The thickness of the support member is preferably 0.05 to 30 mm, more preferably 0.1 to 10 mm, and still more preferably 0.1 to 5 mm. The thickness of the support member is measured under no load. When the thickness of the support member is different between regions, it is preferable that both of the minimum thickness and the maximum thickness are within the above range.

### <Lipid>

The hemostatic material of the present invention comprises a lipid supported on a surface of the base, wherein the lipid comprises one or two or more anionic lipids. When the lipid supported on the surface of the base comes into contact with blood, the anionic lipid included in the lipid supported on the surface of the base becomes negatively charged. The negatively charged anionic lipid can bind to a plurality of platelets (particularly, activated platelets) and can accelerate adhesion and/or aggregation of platelets, and in turn can accelerate blood coagulation. As a result of this, the hemostatic material of the present invention can accelerate the hemostatic effect of blood. The hemostatic effect of the hemostatic material of the present invention is particularly effectively exerted as a result of the fact that a lipid having a platelet adhesion accelerating effect and/or a platelet aggregation accelerating effect is supported on a porous base, particularly a fiber base, in other words, the fact that a lipid has a platelet adhesion accelerating effect and/or a platelet aggregation accelerating effect and the lipid is supported on a porous base, particularly a fiber base.

The platelet adhesion accelerating effect is an effect of accelerating adhesion of platelets to any site or member (e.g., a base on which a lipid is supported, particularly a porous base). In other words, the anionic lipid can accelerate adhesion of platelets in a site or member in which the anionic lipid exists. The platelet aggregation accelerating effect is an effect of accelerating the platelet-platelet attachment (aggregation). In other words, the anionic lipid can accelerate the platelet-platelet attachment (aggregation) in a site or member in which the anionic lipid exists. In actual thrombus formation, there are many cases in which adhesion and aggregation of platelets occur almost at the same time and cannot be distinguished.

In the hemostatic material of the present invention, the lipid is supported on a surface of the base, for example, in one or two or more forms selected from a lipid particle, a lipid particle aggregate and a lipid membrane. A lipid particle aggregate is hereinafter sometimes referred to as an assembly of lipid particles.

When the base is a porous base, particularly a fiber base, it is preferable that the lipid exists so as to account for at least a part of the pore of the porous base, for example, in one or two or more forms selected from a lipid particle, a lipid particle aggregate and a lipid membrane. Existence of the lipid accounting for at least a part of the pore of the porous base is particularly effective when a high-dose lipid is supported on the base.

In one embodiment, the lipid is supported on a surface of the base in a form of a lipid particle and/or a lipid particle aggregate. For example, a part of the lipid is supported on a surface of the base in a form of a lipid particle, and the other part of the lipid is supported on a surface of the base in a form of a lipid particle aggregate.

In another embodiment, the lipid is supported on a surface of the base in a form of a lipid membrane. Irregularities may be formed on the surface of the lipid membrane. When the lipid is composed of: a lipid membrane having a flat surface; and a lipid particle supported on the flat surface of the lipid membrane and/or a lipid particle aggregate supported on the flat surface of the lipid membrane, the lipid corresponds to a lipid membrane having irregularities on the surface.

In further another embodiment, the lipid is supported on a surface of the base in a form of a lipid particle, a lipid particle aggregate and a lipid membrane. For example, a part of the lipid is supported on a surface of the base in a form of a lipid particle, another part of the lipid is supported on a surface of the base in a form of a lipid particle aggregate, and the other part of the lipid is supported on a surface of the base in a form of a lipid membrane.

When the form of the lipid is a lipid particle and/or a lipid particle aggregate, since the surface of the lipid that can come into contact with platelets is increased, the abovementioned effect is more effectively exerted. For example, when a part of or the whole of the lipid particle supported on the surface of the base and/or the lipid particle aggregate supported on the surface of the base come(s) into contact with blood, and then is/are released from the base to act on platelets, the abovementioned effect is more effectively exerted.

When the form of the lipid is a lipid membrane, the lipid membrane may or may not maintain the form of a membrane after coming into contact with blood. When the lipid membrane maintains the form of a membrane after coming into contact with blood, the abovementioned effect is exerted by the anionic lipid included in the lipid membrane. Examples of the case in which the lipid membrane does not maintain the form of a membrane after coming into contact with blood include the case in which a part of or the whole of the lipid membrane is hydrated by moisture in blood to form a lipid particle and the lipid particle thus formed is released from the base. When a part of the lipid membrane is hydrated by moisture in blood to form a lipid particle and the lipid particle thus formed is released from the base, the abovementioned effect is exerted by the anionic lipid included in the lipid particle and the anionic lipid included in the remaining lipid membrane. When the whole of the lipid membrane is hydrated by moisture in blood to form a lipid particle and the lipid particle thus formed is released from the base, the abovementioned effect is exerted by the anionic lipid included in the lipid particle. A lipid particle is formed from a part of or the whole of the lipid membrane, and the lipid particle thus formed is released from the base, resulting in an increased opportunity for the anionic lipid to act on platelets, and thus the abovementioned effect is more effectively exerted. The description on the lipid particle used herein is also applied to a lipid particle supported on the surface of the base as well as a lipid particle formed from a lipid membrane supported on the surface of the base unless otherwise specified.

The lipid is supported on the surface of the base so that the lipid can come into contact with blood. When the base is a porous base, the lipid is supported on a surface of a pore of the porous base. When the base is a fiber base, the lipid is supported on a surface of a pore of the fiber base. The pore of the fiber base is formed by intertanglement of a plurality of fibers, and the surface of the pore of the fiber base is formed by surfaces of a plurality of fibers. When the lipid has a form of a lipid membrane, the lipid membrane is supported on the surface of the pore of the fiber base, for example, so as to spread between a plurality of fibers. A method for supporting the lipid on the base is not particularly limited. Examples of the method for supporting the lipid on the base include physical adsorption, covalent bond, hydrogen bond, coordinate bond, electrostatic interaction, hydrophobic interaction, van der Waals force and the like. It is not necessary that all lipids included in the hemostatic material of the present invention are directly supported on the surface of the base (i.e., keep in contact with the surface of the base). When the lipids assemble by the lipid-lipid interaction force to take a form of a particle or a membrane, a part of the lipids may be directly supported on the surface of the base. The hemostatic material of the present invention may include an assembly of lipid particles formed by binding of two or more lipid particles, and the assembly of lipid particles may include a lipid particle bound to other lipid particles in a state of being directly supported on the surface of the base (i.e., a state of keeping in contact with the surface of the base) as well as a lipid particle bound to other lipid particles in a state of not being directly supported on the surface of the base (i.e., a state of not keeping in contact with the surface of the base). When the base is a porous base, particularly a fiber base, it is also possible that the lipid particle, the assembly of the lipid particle or the lipid membrane accounts for a part of the void of the base, and existence of the lipid particle, the assembly of the lipid particle or the lipid membrane accounting for a part of the void of the base is particularly effective when a high-dose lipid is supported on the base. The lipid particle and/or the assembly of the lipid particle may be supported on the surface of the lipid membrane supported on the surface of the base.

A supported amount of the lipid on the base is not particularly limited as long as the platelet adhesion effect and/or the platelet aggregation effect of the lipid (by extension, the hemostatic effect of the hemostatic material of the present invention) is/are exerted. The supported amount of the lipid on the base is a sum of the supported amount of the lipid directly supported on the surface of the base and the supported amount of the lipid indirectly supported on the surface of the base via the lipid directly supported on the surface of the base. For example, when the lipid has a form of a lipid particle, the supported amount of the lipid particle on the base is a sum of the supported amount of the lipid particle directly supported on the surface of the base and the supported amount of the lipid particle indirectly supported on the surface of the base via one or two or more other lipid particles. In other words, when the hemostatic material of the present invention includes an assembly of lipid particles formed by binding of two or more lipid particles, the supported amount of the lipid particle on the base also includes the supported amount of the assembly of lipid particles on the base. When the base is a porous base (particularly a fiber sheet), the supported amount of the lipid is preferably 1 to 1,000 g/m², more preferably 2 to 500 g/m², and still more preferably 5 to 100 g/m² per planar view area of the porous base. The planar view area is an area of the porous base in a planar view. When the base is a porous base (particularly a fiber sheet), the supported amount of the lipid is preferably 0.1 to 300% by weight, more preferably 1 to 200% by weight, and still more preferably 5 to 100% by weight, based on the weight of the porous base.

When the hemostatic material of the present invention comprises a support member that supports the base, the hemostatic material of the present invention may include a lipid supported on the surface of the base as well as a lipid supported on the surface of the support member. The form of the lipid supported on the surface of the support member is the same as the form of the lipid supported on the surface of the base. The lipid supported on the surface of the support member is supported on the surface of the support member, for example, in one or two or more forms selected from a lipid particle, a lipid particle aggregate and a lipid membrane. The description on the lipid supported on the surface of the support member is the same as the description on the lipid supported on the surface of the base. When the support member is a porous base, it is also possible that the lipid particle, the assembly of the lipid particle or the lipid membrane accounts for a part of the void of the support member, and existence of the lipid particle, the assembly of the lipid particle or the lipid membrane accounting for a part of the void of the support member is particularly effective when a high-dose lipid is supported on the support member.

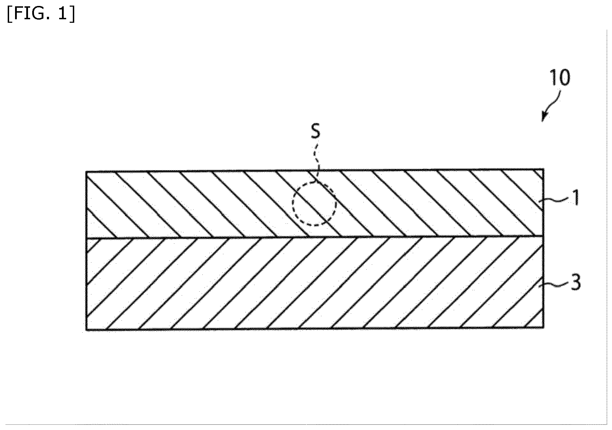

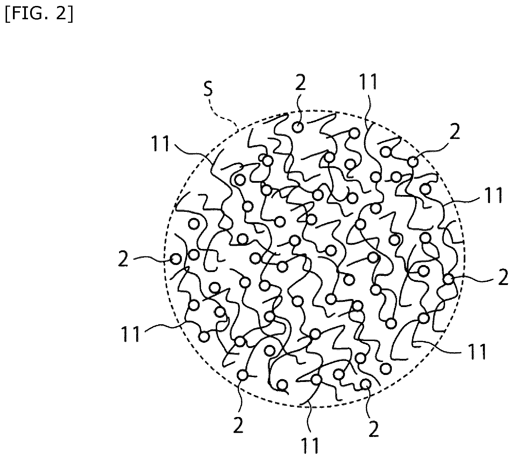

One embodiment of the hemostatic material of the present invention is shown in FIG. 1 and FIG. 2. FIG. 1 is a sectional view schematically showing a hemostatic material according to one embodiment of the present invention, and FIG. 2 is an enlarged view of a region represented by the sign S in FIG. 1.

As shown in FIG. 1 and FIG. 2, a hemostatic material 10 according to one embodiment of the present invention comprises a water-insoluble base 1, many lipid particles 2 supported on the surface of the base 1, and a support member 3 that supports the base 1. The hemostatic material 10 may include an assembly of lipid particles formed by binding of two or more lipid particles 2. The assembly of lipid particles may include a lipid particle 2 bound to other lipid particles 2 in a state of being directly supported on the surface of the base 1 (i.e., a state of keeping in contact with the surface of the base 1) as well as a lipid particle 2 bound to other lipid particles 2 in a state of not being directly supported on the surface of the base 1 (i.e., a state of not keeping in contact with the surface of the base 1). The hemostatic material 10 has a surface formed by the base 1 and a surface formed by the support member 3. When hemostasis is performed using the hemostatic material 10, the hemostatic material 10 is attached to an affected site so that the surface formed by the base 1 comes into contact with the affected site (bleeding site). In the hemostatic material 10, a fiber sheet is used as the base 1, and a water-insoluble support member is used as the support member 3. When hemostasis is performed using the hemostatic material 10, one surface of the fiber sheet is used as a surface that comes into contact with an affected site (bleeding site). On the other surface of the fiber sheet, the support member 3 is provided. The support member 3 is a member that is provided as necessary, and the hemostatic material of the present invention also includes an embodiment in which the support member 3 is omitted.

A lipid particle is a particle comprising a lipid. A lipid membrane is a membrane comprising a lipid. The lipid is an organic molecule having a hydrophilic moiety and a hydrophobic moiety, and the lipid includes a simple lipid, a complex lipid, a derived lipid and the like. The lipid may be modified by a hydrophilic polymer, etc. Examples of the hydrophilic polymer include polyethylene glycol (PEG), polyglycerin, polypropylene glycol, polyvinyl alcohol, styrene-maleic anhydride alternating copolymer, polyvinylpyrrolidone, synthetic polyamino acid and the like.

The lipid (e.g., a lipid constituting a lipid particle, a lipid constituting a lipid membrane, etc.) comprises one or two or more anionic lipids. The anionic lipid has a group that is negatively charged at physiological pH as a part of a hydrophilic moiety. Therefore, when the anionic lipid comes into contact with blood and is hydrated by moisture in the blood, it becomes negatively charged. Examples of the group that is negatively charged at physiological pH include a phosphoric acid group, a carboxyl group, a sulfo group, a nitro group, a salt thereof and the like. The physiological pH is usually pH 5.5 to 9.0, preferably pH 6.5 to 8.0, and more preferably pH 7.0 to 7.8. Examples of an anionic lipid include a carboxylic acid-type lipid, an acidic phospholipid, a fatty acid, a ganglioside, an acidic amino acid-based surfactant and the like.

The shape of the lipid particle is not particularly limited. Examples of the shape of the lipid particle includes a spherical shape (a true spherical shape, an elliptic spherical shape, etc.), an indefinite shape and the like. When the lipid particle is a crystallite such as a nanocrystal and a microcrystal, the crystallite has a definite crystal shape.

The mean particle diameter of the lipid particle is not particularly limited. The mean particle diameter of the lipid particle is preferably 30 to 5,000 nm, more preferably 50 to 1,000 nm, and still more preferably 70 to 400 nm. The mean particle diameter as used herein is a numerical value measured by dynamic light scattering. Dynamic light scattering can be performed using Zetasizer nano (manufactured by Malvern Panalytical Ltd.). At that time, it is possible to use a dispersion liquid having the concentration of the lipid particle of 0.1 mg/mL that was prepared using PBS as a dispersion medium. The measurement temperature is, for example, 25°C. The scattering angle is, for example, 90 degrees. The particle diameter can be adjusted by, for example, using the extrusion method, the French press method and the like.

The lipid particle may be a monodisperse particle or a polydisperse particle, and is preferably a monodisperse particle. In order to obtain a monodisperse lipid particle, it is preferable to adjust the particle diameter of the lipid particle to a certain range by treatment such as homogenization and extrusion.

The form of the lipid particle is not particularly limited. Examples of the form of the lipid particle include a liposome, a micelle, a nanosphere, a microsphere (e.g., a lipid microsphere), a nanocrystal, a microcrystal and the like. Of these forms, a liposome is preferable. Examples of the liposome include a multilamellar vesicle (MLV), a small unilamellar vesicle (SUV), a large unilamellar vesicle and the like. The lipid particle also includes a lipid particle in which the inside of the particle is solid (i.e., the inside of the particle is packed with components) not having a lipid bilayer structure (lamella structure) like a liposome. Examples of such form include a form having a core of a hydrophobic polymer (preferably, a hydrophobic biodegradable polymer) and a lipid layer covering the core.

The form of the lipid particle can be confirmed by electron microscopy (e.g., cryo-transmission electron microscopy (CryoTEM method)), structural analysis using X-rays (e.g., small-angle X-ray scattering (SAXS) measurement) and the like.

The liposome is a lipid vesicle formed from a lipid bilayer membrane including a lipid molecule, specifically, a closed vesicle having space (internal phase) separated from the external environment by a lipid bilayer membrane occurring based on the polarity of a hydrophobic group and a hydrophilic group of a lipid molecule. The internal phase of the liposome includes a dispersion medium (e.g., an aqueous medium such as water) used during the production of the liposome. When the lipid bilayer membrane is defined as one layer, the number of layers of the lipid bilayer membrane possessed by the liposome is preferably 1 to 4, and more preferably 1 to 2.

The number of layers of the lipid bilayer membrane can be controlled by a pore diameter of a filter and a dispersion medium (pH, temperature, ionic strength) of a vesicle. Examples of the method for measuring the number of layers include the freeze-fracture method, small-angle X-ray scattering, electron spin resonance (ESR) using a spin-labeled lipid, a measurement method using ³¹P-NMR, a measurement method using 6-p-toluidino-2-naphthalenesulfonic acid (TNS) and the like.

The liposome may include a drug in the internal phase. The drug included in the internal phase of the liposome is preferably a drug that is physiologically or pharmacologically effective by being accumulated in a vascular injury site, and examples thereof include a platelet aggregation initiator, a blood coagulant, a vasoconstrictor, an anti-inflammatory agent and the like. Among these, a drug that enhances in particular the thrombus formation (e.g., a platelet aggregation initiator, a blood coagulant, etc.) is particularly preferably used. Encapsulation of a water-soluble drug can be performed using, for example, the hydration method, the extrusion method, the ethanol injection method, the reverse phase evaporation method, the freeze-thawing method and the like. Encapsulation of a lipophilic drug can be performed using, for example, the Bangham method, the mechanochemical method, the supercritical carbon dioxide method, the film loading method and the like. Encapsulation of a dissociative drug can be performed using, for example, the pH gradient (remote loading) method, the counterion concentration gradient method and the like.

Examples of the platelet aggregation initiator include adenosine diphosphate (ADP), collagen, a collagen-derived peptide, convulxin, serotonin, epinephrine, vasopressin, carbazochrome, a blood coagulation factor (e.g., FVIII, FIX), thrombin, an antiplasmin agent (e.g., epsilon-aminocaproic acid, tranexamic acid), protamine sulfate, ethamsylate, phytonadione, conjugated estrogen (e.g., sodium estrone sulfate, sodium equilin sulfate) and the like.

Examples of the blood coagulation accelerating agent include fibrinogen, thrombin, a blood coagulation factor (e.g., FXa), protamine sulfate and the like.

Examples of the vasoconstrictor include noradrenaline, norfenefrine, phenylephrine, metaraminol, methoxamine, prostaglandin F₁α, prostaglandin F₂α, thromboxane A₂ and the like.

Examples of the anti-inflammatory agent include a steroidal anti-inflammatory agent (e.g., dexamethasone, hydrocortisone, prednisolone, betamethasone, triamcinolone, methylprednisolone), a nonsteroidal anti-inflammatory agent (e.g., indomethacin, acemetacin, flurbiprofen, aspirin, ibuprofen, flufenamic acid, ketoprofen) and the like.

The lipid particle and the lipid membrane may include one or two or more components other than a lipid. Examples of the other components include a surfactant, a protein, a peptide, an antioxidant, an antiseptic, a pH adjuster, triglyceride, a biodegradable polymer such as polylactic acid, a dispersion medium used for production of the lipid particle or the lipid membrane and the like.

Examples of the surfactant include an anionic surfactant, an amphoteric surfactant, a nonionic surfactant and the like.

Examples of the anionic surfactant include α-acyl sulfonate, alkyl sulfonate, alkyl aryl sulfonate, alkyl naphthalene sulfonate, alkyl sulfate, alkyl ether sulfate, alkylamide sulfate, polyoxyethylene alkyl ether sulfate, polyoxyethylene alkylamide ether sulfate, alkyl phosphate, alkylamide phosphate, alkyloylalkyl taurine salt, N-acyl amino acid salt, sulfosuccinate, perfluoroalkyl phosphoric acid ester and the like.

Examples of the amphoteric surfactant include glycine type, aminopropionic acid type, carboxybetaine type, sulfobetaine type, sulfonic acid type, sulfuric acid type, phosphoric acid type and the like. Specific examples thereof include 2-alkyl-N-carboxymethyl-N-hydroxyethyl imidazolinium betaine, coconut oil fatty acid amide propyl betaine and the like.

Examples of the nonionic surfactant include fatty acid alkanolamide, polyoxyethylene hardened castor oil, polyoxyethylene sorbitan fatty acid ester, polyoxyethylene alkyl ether, polyoxyethylene alkyl ester, sucrose fatty acid ester, polyglycerin fatty acid ester, alkyl amine oxide and the like.

Examples of the antioxidant include ascorbic acid, uric acid, a tocopherol homolog such as vitamin E and the like. As the tocopherol, four types of isomers including α-, β-, γ- and σ-isomers exist, and all of them are included in the lipid particle and the lipid membrane.

Examples of the antiseptic include propyl p-hydroxybenzoate, ethyl p-hydroxybenzoate, methyl p-hydroxybenzoate, bronopol and the like.

Examples of the pH adjuster include a phosphate buffer and the like.

The thickness of the lipid membrane is preferably 10 to 1,000 nm, more preferably 30 to 500 nm, and still more preferably 60 to 240 nm. The method for measuring the lipid membrane is as follows. The lipid membrane is observed with a scanning electron microscope (SEM). The thickness of five points optionally selected in the SEM observation image is measured, and the mean is regarded as the thickness of the lipid membrane.

The lipid of the present invention may or may not carry a protein involved in adhesion or aggregation of platelets, such as GPIb and H12, or a peptide corresponding to an active site of the protein. Without carrying a protein involved in adhesion or aggregation of platelets, such as GPIb and H12, or a peptide corresponding to an active site of the protein, the lipid of the present invention can accelerate adhesion and/or aggregation of platelets. Therefore, regarding the lipid of the present invention, it is preferable that the surface is not chemically modified by GPIb, H12, etc., in terms of reducing the production step, the production cost and the like.

It is preferable that the surfaces of the lipid particle, the lipid particle aggregate and the lipid membrane are negatively charged at physiological pH. As a result of this, when the surfaces of the lipid particle, the lipid particle aggregate and the lipid membrane come into contact with blood and are hydrated by moisture in the blood, they becomes negatively charged.

In the lipid particle before coming into contact with blood, a hydrophilic moiety of the anionic lipid may or may not be located in the surface side of the lipid particle. When the hydrophilic moiety of the anionic lipid is located in the surface side of the lipid particle in the lipid particle before coming into contact with blood, in the lipid particle after coming into contact with blood, the hydrophilic moiety of the anionic lipid is also located in the surface side of the lipid particle. Even when the hydrophilic moiety of the anionic lipid is not located in the surface side of the lipid particle in the lipid particle before coming into contact with blood, after coming into contact with blood, the lipid particle is reconstituted, and as a result, the hydrophilic moiety of the anionic lipid can be located in the surface side of the lipid particle. As a result of the fact that the hydrophilic moiety of the anionic lipid is located in the surface side of the lipid particle, the surface of the lipid particle becomes likely to be negatively charged at physiological pH (i.e., when it comes into contact with blood and is hydrated by moisture in the blood, it becomes likely to be negatively charged).

In the lipid membrane before coming into contact with blood, the hydrophilic moiety of the anionic lipid may or may not be located in the surface side of the lipid membrane. When the hydrophilic moiety of the anionic lipid is located in the surface side of the lipid membrane in the lipid membrane before coming into contact with blood, in the lipid membrane remaining after coming into contact with blood, the hydrophilic moiety of the anionic lipid is also located in the surface side of the lipid particle. Even when the hydrophilic moiety of the anionic lipid is not located in the surface side of the lipid particle in the lipid membrane before coming into contact with blood, after coming into contact with blood, the lipid membrane is reconstituted, and as a result, the hydrophilic moiety of the anionic lipid can be located in the surface side of the lipid membrane. As a result of the fact that the hydrophilic moiety of the anionic lipid is located in the surface side of the lipid membrane, the surface of the lipid membrane becomes likely to be negatively charged at physiological pH (i.e., when it comes into contact with blood and is hydrated by moisture in the blood, it becomes likely to be negatively charged). Even when the hydrophilic moiety of the anionic is or is not located in the surface side of the lipid membrane in the lipid membrane before coming into contact with blood, in the lipid particle formed from the lipid membrane, the hydrophilic moiety of the anionic lipid is located in the surface side of the lipid particle.

The degree of negative charge at physiological pH of the surface of the lipid particle or the surface of the lipid particle aggregate can be evaluated based on a zeta potential (surface potential) of the lipid particle or the lipid particle aggregate under a physiological condition. The physiological condition is a condition in which usually pH is 5.5 to 9.0, preferably pH is 6.5 to 8.0, and more preferably pH is 7.0 to 7.8, and the ionic strength is usually 0.05 to 0.30, preferably 0.10 to 0.20, and more preferably 0.14 to 0.16.

The zeta potential (surface potential) of the lipid particle or the lipid particle aggregate under a physiological condition is preferably -12 mV or less, more preferably -15 mV or less, and still more preferably -18 mV or less. The lower limit of the zeta potential of the lipid particle or the lipid particle aggregate under a physiological condition is not particularly limited. The zeta potential of the lipid particle or the lipid particle aggregate under a physiological condition is preferably -80 mV or more, more preferably -50 mV or more, and still more preferably -45 mV or more. The upper limit and the lower limit mentioned herein can be appropriately combined. The zeta potential as used herein is a numerical value measured by electrophoretic light scattering. Electrophoretic light scattering can be performed using Zetasizer nano (manufactured by Malvern Panalytical Ltd.). At that time, it is possible to use a dispersion liquid having the concentration of the lipid particle of 0.1 mg/mL that was prepared using PBS as a dispersion medium. The measurement condition is, for example, a condition in which pH is 7.4, the ionic strength is 0.153, and the temperature is 25°C.

A method for producing the lipid particle or the lipid particle aggregate can be appropriately selected according to the form of the lipid particle or the lipid particle aggregate. Examples of the method for producing the lipid particle or the lipid particle aggregate include the thin film method, the reverse phase evaporation method, the ethanol injection method, the ether injection method, the dehydration-rehydration method, the surfactant dialysis method, the surfactant removal method, the hydration method, the freeze-thawing method, the ultrasonic wave method, the extrusion method, the high-pressure emulsification method and the like.

When the lipid particle or the lipid particle aggregate is produced, the dispersion medium in which the lipid particle is dispersed can be used, for example, buffers such as a phosphate buffer, a citrate buffer and phosphate-buffered saline, water, physiological saline, a cell culture medium and the like.

The lipid membrane can be supported on the surface of a hemostatic material by attaching an appropriate amount of a solution of a lipid at an appropriate concentration to a base, followed by drying. As a solvent in which a lipid is dissolved, for example, it is possible to use organic solvents such as ethyl alcohol, isopropyl alcohol, tert-butyl alcohol and diethyl ether. As a method for attaching a solution to a base, for example, it is possible to use the spraying method, the falling drop method, the dipping method, the applying method and the like. As a drying method, for example, it is possible to use freeze-drying, natural drying, drying by heating, drying under reduced pressure and the like. The concentration of a lipid can be selected in a range of 0.1 mg/mL to 100 mg/mL according to the solubility in the solvent and the membrane thickness of the lipid membrane obtained. The amount of a solution attached to a base can be appropriately adjusted according to the surface area of the base (when a support member exists, the surface area of the support member is included), the membrane thickness of the lipid membrane and the like. Usually, when an anionic lipid is dissolved in an organic solvent, it is dissolved as an acid type. When the anionic lipid is a salt type such as a sodium salt, since the solubility in an organic solvent is greatly decreased, an undissolved lipid is dispersed as an amorphous fine powder or a lipid particle in a form of a crystallite. When this is supported on a base in the same manner as the abovementioned method, a lipid particle is supported on the surface of the base. In this case, the lipid particle may be supported in a state in which a lipid membrane derived from a lipid dissolved in an organic solvent coexists, or may be supported in a state in which an aggregate of the lipid particle further coexists.

The surfaces of the lipid particle, the lipid particle aggregate and the lipid membrane may be modified by a hydrophilic polymer, etc. Examples of the hydrophilic polymer include polyethylene glycol (PEG), polyglycerin, polypropylene glycol, polyvinyl alcohol, styrene-maleic anhydride alternating copolymer, polyvinylpyrrolidone, synthetic polyamino acid and the like. Regarding these hydrophilic polymers, one hydrophilic polymer may be used alone, or two or more hydrophilic polymers may be used in combination.

The one or two or more anionic lipids included in the lipid supported on the surface of the base include one or two or more carboxylic acid-type lipids.

The lipid according to the invention comprises one or two or more carboxylic acid-type lipids selected from carboxylic acid-type lipids (III) to (VI). Hereinafter, the carboxylic acid-type lipids (III) to (VI) are sometimes collectively referred to as "carboxylic acid-type lipid of the present invention."

The carboxylic acid-type lipid of the present invention has a hydrophilic moiety and a hydrophobic moiety, and the hydrophilic moiety has a carboxyl group or a salt thereof. The carboxylic acid-type lipid of the present invention is an anionic lipid, and a carboxyl group or a salt thereof existing in the hydrophilic moiety is ionized at physiological pH and negatively charged. Therefore, when the lipid particle, the lipid particle aggregate or the lipid membrane comes into contact with blood and is hydrated by moisture in the blood, the surface of the lipid particle, the lipid particle aggregate or the lipid membrane is negatively charged. As a result of this, at least a part of the lipid particle, the lipid particle aggregate or the lipid membrane can bind to a plurality of platelets (particularly, activated platelets) via an electrostatic interaction and can accelerate aggregation of platelets, and in turn can accelerate blood coagulation. This mention does not deny the fact that the platelet adhesion accelerating effect and/or the platelet aggregation accelerating effect evoked by the lipid particle, the lipid particle aggregate or the lipid membrane of the present invention can be involved in an interaction other than an electrostatic interaction such as the van der Waals force.

In the lipid particle, the lipid particle aggregate or the lipid membrane, the content of the carboxylic acid-type lipid of the present invention is not particularly limited as long as the surface of the lipid particle, the lipid particle aggregate or the lipid membrane is negatively charged at physiological pH. The content of the carboxylic acid-type lipid of the present invention is preferably 5 mol% or more, more preferably 10 mol% or more, still more preferably 30 mol% or more, yet more preferably 50 mol% or more, further preferably 60 mol% or more, and still further preferably 70 mol% or more, based on the total lipid amount included in the lipid particle, the lipid particle aggregate or the lipid membrane. The upper limit of the content of the carboxylic acid-type lipid of the present invention is 100 mol% based on the total lipid amount included in the lipid particle, the lipid particle aggregate or the lipid membrane (in this case, all lipids included in the lipid particle, the lipid particle aggregate or the lipid membrane are the carboxylic acid-type lipids of the present invention).

### <Carboxylic Acid-Type Lipid (I) not falling under the scope of the claims>

The carboxylic acid-type lipid (I) not falling under the scope of the claims is represented by the following formula (I). When two or more same symbols (e.g., L, X, etc.) exist in formula (I), the meanings of these same symbols may be the same or different as long as they are within the range of the definition of the symbols.

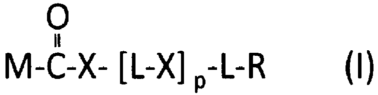

In formula (I), M represents OH- or M₀-NH-.

In formula (I), M₀ represents an amino acid residue, an amino acid derivative residue, a peptide residue or a salt thereof that can be negatively charged at physiological pH.

The physiological pH is usually pH 5.5 to 9.0, preferably pH 6.5 to 8.0, and more preferably pH 7.0 to 7.8.

The fact that an amino acid residue, an amino acid derivative residue, a peptide residue or a salt thereof represented by M₀ can be negatively charged at physiological pH means that an amino acid residue, an amino acid derivative residue, a peptide residue or a salt thereof represented by M₀ can be negatively charged when coming into contact with blood.

An amino acid residue, an amino acid derivative residue, a peptide residue or a salt thereof represented by M₀ may have, in addition to a functional group that can be negatively charged at physiological pH, a functional group that can be positively charged at physiological pH as long as it can be negatively charged at physiological pH as a whole. For example, when the number of functional groups (e.g., a carboxyl group or a salt thereof) that can be negatively charged at physiological pH is higher than the number of functional groups (e.g., an amino group) that can be positively charged at physiological pH, an amino acid residue, an amino acid derivative residue, a peptide residue or a salt thereof represented by M₀ can be negatively charged at physiological pH as a whole.

Amino acid is an organic compound having a carboxyl group and an amino group in the same molecule. Amino acid is preferably aliphatic amino acid. Aliphatic amino acid may be any one of α-amino acid, β-amino acid, γ-amino acid, δ-amino acid and ε-amino acid, and is preferably α-amino acid. α-Amino acid may be any one of D-form and L-form, and is preferably L-form. Amino acid may be natural amino acid or non-natural amino acid, and is preferably natural amino acid. Natural amino acid is preferably any one of 20 types of amino acids included in a protein. Examples of the other amino acids include cystine, hydroxyproline, hydroxylysine, thyroxine, O-phosphoserine, desmosine, β-alanine, δ-aminovaleric acid, sarcosine (N-methylglycine), γ-aminobutyric acid (GABA), tricholomic acid, kainic acid, opine and the like.

Examples of the α-amino acid include glycine, alanine, valine, leucine, isoleucine, serine, threonine, tyrosine, cysteine, methionine, aspartic acid, asparagine, glutamic acid, glutamine, arginine, lysine, histidine, phenylalanine, tryptophan and the like, examples of the β-amino acid include β-alanine and the like, examples of the γ-amino acid include γ-amino-n-butyric acid (GABA), carnitine and the like, examples of the δ-amino acid include 5-aminolevulinic acid, 5-aminovaleric acid and the like, and examples of the ε-amino acid include 6-aminohexanoic acid and the like.

Examples of the non-natural amino acid include amino acid in which a main chain structure is different from that of natural amino acid (e.g., α,α-disubstituted amino acid (e.g., α-methylalanine, etc.), N-alkyl-α-amino acid, D-amino acid, β-amino acid, a-hydroxy acid, etc.), amino acid in which a side chain structure is different from that of natural amino acid (e.g., norleucine, homohistidine, etc.), amino acid in which a side chain has excessive methylene (e.g., homoamino acid, etc.) and amino acid in which a functional group (e.g., a thiol group) in a side chain is substituted with a sulfonic acid group (e.g., cysteic acid, etc.). In addition, aminoalkanesulfonic acid having a sulfonic acid group and an amino group in the same molecule (e.g., aminoethanesulfonic acid (taurine), etc.) is included in the non-natural amino acid.

An amino acid residue represented by M₀ means a moiety obtained by removing an amino group from amino acid unless otherwise specified. An amino group removed from α-amino acid, β-amino acid, γ-amino acid, δ-amino acid and ε-amino acid may be an amino group bonded to a-carbon, β-carbon, γ-carbon, δ-carbon and ε-carbon, respectively, or may be an amino group included in a side chain, and is preferably an amino group bonded to a-carbon, β-carbon, γ-carbon, δ-carbon and ε-carbon. When M₀ represents an amino acid residue, -NH- of a structure represented by M₀-NH-CO- is derived from an amino group of amino acid. Thus, the amino acid residue represented by M₀ is defined as a moiety obtained by removing an amino group from amino acid.

The amino acid residue or a salt thereof represented by M₀ is not particularly limited as long as it can be negatively charged at physiological pH as a whole. The amino acid residue or a salt thereof represented by M₀ is preferably an acidic amino acid residue, a neutral amino acid residue or a salt thereof, and more preferably an acidic amino acid residue or a salt thereof.

An acidic amino acid residue or a salt thereof has two carboxyl groups or salts thereof, and these carboxyl groups or salts thereof can be ionized at physiological pH and negatively charged. Therefore, the acidic amino acid residue or a salt thereof can be negatively charged at physiological pH as a whole. The acidic amino acid residue or a salt thereof is preferably an aspartic acid residue, a glutamic acid residue or a salt thereof.

A neutral amino acid residue or a salt thereof has one carboxyl group or a salt thereof, and this carboxyl group or salt thereof can be ionized at physiological pH and negatively charged. Meanwhile, a functional group included in a side chain of a neutral amino acid residue or a salt thereof is uncharged at physiological pH. Therefore, the neutral amino acid residue or a salt thereof can be negatively charged at physiological pH as a whole. Examples of the neutral amino acid residue include a glycine residue, an alanine residue, a phenylalanine residue, a leucine residue, an isoleucine residue, a methionine residue, a valine residue, an asparagine residue, a glutamine residue and the like. Examples of a preferable neutral amino acid residue include a glycine residue, an alanine residue and the like.

An amino acid derivative represented by M₀ is produced by introducing a chemical modification into a side chain of amino acid, and has the same structure as that of amino acid except that a chemical modification is introduced into a side chain. An amino acid derivative residue represented by M₀ means a moiety obtained by removing an amino group from an amino acid derivative unless otherwise specified. An amino group removed from a derivative of α-amino acid, β-amino acid, γ-amino acid, δ-amino acid and ε-amino acid may be an amino group bonded to a-carbon, β-carbon, γ-carbon, δ-carbon and ε-carbon, respectively, or may be an amino group included in a side chain, and is preferably an amino group bonded to a-carbon, β-carbon, γ-carbon, δ-carbon and ε-carbon, respectively. When M₀ represents an amino acid derivative residue, -NH- of a structure represented by Mₒ-NH-CO- is derived from an amino group of an amino acid derivative. Thus, the amino acid derivative residue represented by M₀ is defined as a moiety obtained by removing an amino group from an amino acid derivative.

The amino acid derivative residue represented by M₀ is not particularly limited as long as it can be negatively charged at physiological pH as a whole. Examples of the amino acid derivative residue represented by M₀ include a residue of a basic amino acid derivative. Examples of the introduced derivatization that a basic amino acid derivative comprises include amidation of an amino group of a side chain of a basic amino acid to a group represented by the following formula: -NH-CO-R₁ wherein -NH- is derived from the amino group of the side chain of the basic amino acid, and R₁ represents a hydrocarbon group, and the like. The basic amino acid has a carboxyl group bonded to a-carbon, an amino group bonded to a-carbon and an amino group included in a side chain bonded to a-carbon. As a result of derivatization of an amino group of a side chain into -NH-CO-R₁, the residue of a basic amino acid derivative can be negatively charged at physiological pH as a whole.

Examples of the basic amino acid include lysine, arginine, histidine and the like.

As a carboxylic acid used for amidation of an amino group of a side chain of a basic amino acid, for example, it is possible to use aliphatic carboxylic acid represented by the formula: R₁-COOH wherein R₁ is the same as defined above.

A hydrocarbon group represented by R₁ is preferably an aliphatic hydrocarbon group. The aliphatic hydrocarbon group may be linear or branched, and is preferably linear. The aliphatic hydrocarbon group may be saturated or unsaturated, and is preferably saturated. The number of carbon atoms of the aliphatic hydrocarbon group is usually 1 to 10, preferably 1 to 6, more preferably 1 to 4, and still more preferably 1 to 2. The unsaturated bond may be a carbon-carbon double bond or a carbon-carbon triple bond, and is preferably a carbon-carbon double bond.

Examples of the aliphatic hydrocarbon group represented by R₁ include an alkyl group, an alkenyl group, alkynyl group and the like, and it is preferably an alkyl group or an alkenyl group, and more preferably an alkyl group. Specific examples of the aliphatic hydrocarbon group represented by R₁ include a methyl group, an ethyl group, a propyl group, an isopropyl group, a butyl group, a sec-butyl group, a tert-butyl group, an isobutyl group, a pentyl group, a tert-pentyl group, an isopentyl group, a hexyl group, an isohexyl group, a heptyl group, an octyl group, a 2-ethylhexyl group, a nonyl group, a decyl group, an ethylene group, a propylene group, a butene group, an isobutene group, an isoprene group, a pentene group, a hexene group, a heptene group, an octene group, a nonene group, a decene group and the like. Specific preferable examples of the aliphatic hydrocarbon group represented by R₁ include a methyl group, an ethyl group and the like.

A peptide residue represented by M₀ means a moiety obtained by removing an amino group from a peptide unless otherwise specified. An amino group removed from a peptide may be an amino group bonded to a-carbon, β-carbon, γ-carbon, δ-carbon or ε-carbon, or may be an amino group included in a side chain, and is preferably an amino group bonded to a-carbon, β-carbon, γ-carbon, δ-carbon or ε-carbon. When M₀ represents a peptide residue, -NH- of a structure represented by Mₒ-NH-CO- is derived from an amino group of a peptide. Thus, the peptide residue represented by M₀ is defined as a moiety obtained by removing an amino group from a peptide.

The type and the number of amino acid residues constituting the peptide residue represented by M₀ is not particularly limited as long as the peptide residue can be negatively charged at physiological pH as a whole. The amino acid residue as used herein is a usual meaning (a moiety obtained by removing H of an amino group and/or OH of a carboxyl group from amino acid), and differs from the abovementioned meaning (a moiety obtained by removing an amino group from amino acid).

The peptide residue represented by M₀ can be composed of one or two or more amino acid residues selected from an acidic amino acid residue, a neutral amino acid residue and a basic amino acid residue, and is preferably composed of one or two or more amino acid residues selected from an acidic amino acid residue and a neutral amino acid residue, more preferably composed of one or two or more amino acid residues selected from an acidic amino acid residue, and still more preferably composed of one or two amino acid residues selected from aspartic acid and glutamic acid.

In the peptide residue represented by M₀, the difference between the number of functional groups (e.g., a carboxyl group or a salt thereof) that can be negatively charged at physiological pH and the number of functional groups (e.g., an amino group) that can be positively charged at physiological pH (the number of functional groups that can be negatively charged at physiological pH - the number of functional groups that can be positively charged at physiological pH) is preferably 1 or more, more preferably 2 or more, and still more preferably 3 or more. The upper limit of the difference is not particularly limited, and is preferably 10, more preferably 8, and still more preferably 4. In the peptide residue represented by M₀, the number of functional groups (e.g., an amino group) that can be positively charged at physiological pH is preferably 4 or less, more preferably 2 or less, and still more preferably 0.

The number of amino acid residues constituting the peptide residue represented by M₀ is usually 2 to 12, preferably 2 to 7, more preferably 2 to 5, and still more preferably 2 to 4.

The peptide residue represented by M₀ is preferably a peptide residue including one or two or more acidic amino acid residues, more preferably a peptide residue including two or more acidic amino acid residues, and still more preferably a peptide residue including two or more acidic amino acid residues selected from an aspartic acid residue and a glutamic acid residue. The peptide residue including one or two or more acidic amino acid residues may or may not include a neutral amino acid residue. The peptide residue including one or two or more acidic amino acid residues may or may not include a basic amino acid residue, and preferably does not include a basic amino acid residue. In other words, the peptide residue including one or two or more acidic amino acid residues is preferably composed of an acidic amino acid residue and a neutral amino acid residue, and more preferably composed of an acidic amino acid residue.

Examples of the peptide residue including two or more acidic amino acid residues selected from an aspartic acid residue and a glutamic acid residue include a peptide residue represented by the following formula (XII) (hereinafter sometimes referred to as "AG residue"). The AG residue is a peptide residue composed of three acidic amino acid residues selected from an aspartic acid residue and a glutamic acid residue.

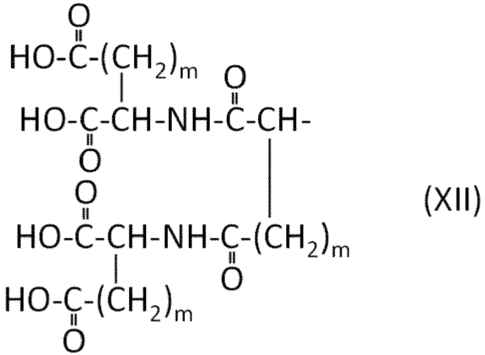

In formula (XII), m represents 1 or 2. Integers represented by a plurality of m's existing in formula (XII) may be the same or different.

A salt of an amino acid residue, an amino acid derivative residue or a peptide residue represented by M₀ is usually a salt formed by a carboxyl group, and specific examples thereof include a calcium salt, a magnesium salt, a potassium salt and the like.

In formula (I), R represents a hydrocarbon group. R is a monovalent functional group.

The number of carbon atoms of the hydrocarbon group represented by R is 8 to 30, preferably 10 to 24, more preferably 12 to 22, and still more preferably 14 to 18.

The hydrocarbon group represented by R is preferably an aliphatic hydrocarbon group. The aliphatic hydrocarbon group may be linear or branched, and is preferably linear. The aliphatic hydrocarbon group may be saturated or unsaturated, and is preferably saturated. The number of carbon atoms of the aliphatic hydrocarbon group is preferably 10 to 24, more preferably 12 to 22, and still more preferably 14 to 18. When the aliphatic hydrocarbon group has an unsaturated bond, the number of unsaturated bonds is usually 1 to 6, preferably 1 to 4, more preferably 1 to 3, and still more preferably 1 to 2. The unsaturated bond may be a carbon-carbon double bond or a carbon-carbon triple bond, and is preferably a carbon-carbon double bond.

Examples of the aliphatic hydrocarbon group represented by R include an alkyl group, an alkenyl group, alkynyl group and the like, and it is preferably an alkyl group or an alkenyl group, and more preferably an alkyl group. Specific examples of the aliphatic hydrocarbon group represented by R include a dodecyl group, a tridecyl group, a tetradecyl group, a pentadecyl group, a hexadecyl group, a heptadecyl group, an octadecyl group, a nonadecyl group, an icosyl group, a henicosyl group, a docosyl group, a dodecenyl group, a tricosyl group, a tridecenyl group, a tetradecenyl group, a pentadecenyl group, a hexadecenyl group, a heptadecenyl group, an octadecenyl group, a nonadecenyl group, an icocenyl group, a henicosenyl group, a docosenyl group, a tricosenyl group, a tridecadienyl group, a tetradecadienyl group, a pentadecadienyl group, a hexadecadienyl group, a heptadecadienyl group, an octadecadienyl group, a nonadecadienyl group, an icosadienyl group, a henicosadienyl group, a docosadienyl group, an octadecatrienyl group, an icosatrienyl group, an icosatetraenyl group, an icosapentaenyl group, a docosahexaenyl group, a methyldodecyl group, a methyltridecyl group, a methyltetradecyl group, a methylpentadecyl group, a methylheptadecyl group, a methyloctadecyl group, a methylnonadecyl group, a methylicosyl group, a methylhenicosyl group, a methyldocosyl group, an ethylundecyl group, an ethyldodecyl group, an ethyltridecyl group, an ethyltetradecyl group, an ethylpentadecyl group, an ethylheptadecyl group, an ethyloctadecyl group, an ethylnonadecyl group, an ethylicosyl group, an ethylhenicosyl group, a hexylheptyl group, a hexylnonyl group, a heptyloctyl group, a heptyldecyl group, an octylnonyl group, an octylundecyl group, a nonyldecyl group, a decylundecyl group, an undecyldodecyl group, a hexamethylundecyl group and the like. Examples of a preferable aliphatic hydrocarbon group include a tetradecyl group, a hexadecyl group, a heptadecyl group, an octadecyl group and the like.

In formula (I), L represents -CO-O-, -O-CO-, -CO-NH-, -NH-CO-, -CO-S-, -S-CO- or -S-S-. L represents preferably -CO-O-, -O-CO-, -CO-NH- or -NH-CO-. When a plurality of L's exist in formula (I) (when p is 1 or more), the meanings of these multiple L's may be the same or different as long as they are within the range of the definition of L. L is a divalent functional group, and the right and left of L correspond to the right and left of formula (I). In other words, (*al)-L-(*a2) is bonded to a group existing on the left side of L in formula (I) via a bond on the left side (*a1), and is bonded to a group existing on the right side of L in formula (I) via a bond on the right side (*a2). Therefore, -CO-O- is distinguished from -O-CO-, -CO-NH- is distinguished from -NH-CO-, and -CO-S- is distinguished from -S-CO-.

In formula (I), X represents a hydrocarbon group, a neutral amino acid residue or a polyalkylene glycol residue. When a plurality of X's exist in formula (I) (when p is 1 or more), the meanings of these multiple X's may be the same or different as long as they are within the range of the definition of X. X is a divalent functional group.

The number of carbon atoms of the hydrocarbon group represented by X is 1 to 6, preferably 1 to 5, more preferably 1 to 4, and still more preferably 1 to 2.

The hydrocarbon group represented by X is preferably an aliphatic hydrocarbon group. The aliphatic hydrocarbon group may be linear or branched, and is preferably linear. The aliphatic hydrocarbon group may be saturated or unsaturated, and is preferably saturated. The unsaturated bond may be a carbon-carbon double bond or a carbon-carbon triple bond, and is preferably a carbon-carbon double bond. Examples of the aliphatic hydrocarbon group represented by X include an alkylene group, an alkenylene group, an alkynylene group and the like, and it is preferably an alkylene group or an alkenylene group, and more preferably an alkylene group. The number of carbon atoms of the alkylene group is usually 1 to 6, preferably 1 to 5, more preferably 1 to 4, and still more preferably 1 to 2. The number of carbon atoms of the alkenylene group is usually 2 to 6, preferably 2 to 5, more preferably 2 to 4, and still more preferably 2 to 3. The number of carbon atoms of the alkynylene group is usually 2 to 6, preferably 2 to 5, more preferably 2 to 4, and still more preferably 2 to 3.

Examples of the alkylene group include -CH₂-, -(CH₂)₂-, -(CH₂)₃-, -CH(CH₃)CH₂-, -CH₂CH(CH₃)-, -(CH₂)₄-, -CH(CH₃)(CH₂)₂-, -(CH₂)₂CH(CH₃)-, -C(CH₃)₂CH₂-, -CH₂C(CH₃)₂-, -(CH₂)₃-, -CH(CH₃)(CH₂)₃-, -(CH₂)₃CH(CH₃)-, -CH(C₂H₅)(CH₂)₂-, -(CH₂)₂CH(C₂H₅)-, -CH₂CH(CH₃)(CH₂)₂-, -(CH₂)₂CH(CH₃)CH₂-, -CH₂CH(C₂H₅)CH₂-, -C(CH₃)₂(CH₂)₂-, -(CH₂)₂C(CH₃)₂-, -CH₂C(CH₃)₂CH₂-, -(CH₂)₆-, -CH(CH₃)(CH₂)₄-, -(CH₂)₄CH(CH₃)-, -CH(C₂H₅)(CH₂)₃-, -CH₂CH(CH₃)(CH₂)₃-, -(CH₂)₃CH(CH₃)CH₂-, -(CH₂)₂CH(CH₃)(CH₂)₂-, -(CH₂)₃CH(C₂Hₛ)-, -C(CH₃)₂(CH₂)₃-, -(CH₂)₃C(CH₃)₂-, -CH₂C(CH₃)₂(CH₂)₂-, -(CH₂)₂C(CH₃)₂CH₂-, -C(CH₃)₂C(CH₃)₂- and the like. Examples of a preferable alkylene group include -CH₂-, -(CH₂)₂- and the like.

Examples of the alkenylene group include -CH=CH-, -CH=CH-CH₂-, -CH₂-CH=CH-, -CH=CH-CH₂-CH₂-, -CH₂-CH=CH-CH₂-, -CH₂-CH₂-CH=CH-, -CH=CH-CH=CH-, -C(CH₃)=CH-CH₂-, -CH=C(CH₃)-CH₂-, -CH=CH-CH(CH₃)-, -CH(CH₃)-CH=CH-, -CH₂-C(CH₃)=CH-, -CH₂-CH=C(CH₃)-, -CH=CH-CH₂-CH₂-CH₂-, -CH₂-CH=CH-CH₂-CH₂-, -CH₂-CH₂-CH=CH-CH₂-, -CH₂-CH₂-CH₂-CH=CH-, -CH=CH-CH₂-CH₂-CH₂-CH₂-, -CH₂-CH=CH-CH₂-CH₂-CH₂-, -CH₂-CH₂-CH=CH-CH₂-CH₂-, -CH₂-CH₂-CH₂-CH=CH-CH₂-, -CH₂-CH₂-CH₂-CH₂-CH=CH- and the like. Examples of a preferable alkenylene group include -CH=CH-, -CH=CH-CH₂- and the like.

Examples of the alkynylene group include -C≡C-, -C≡C-CH₂-, -CH₂-C≡C-, -C≡C-CH₂-CH₂-, -CH₂-C≡C-CH₂-, -CH₂-CH₂-C≡C-, -C≡C-CH₂-CH₂-CH₂-, -CH₂-C≡C-CH₂-CH₂-, -CH₂-CH₂-C≡C-CH₂-, -CH₂-CH₂-CH₂-C≡C-, -C≡C-CH₂-CH₂-CH₂-CH₂-, -CH₂-C≡C-CH₂-CH₂-CH₂-, -CH₂-CH₂-C≡C-CH₂-CH₂-, -CH₂-CH₂-CH₂-C≡C-CH₂-, -CH₂-CH₂-CH₂-CH₂-C≡C- and the like. Examples of a preferable alkynylene group include -C≡C-, -C=C-CH₂- and the like.

A neutral amino acid residue represented by X means a moiety obtained by removing a carboxyl group and an amino group from a neutral amino acid unless otherwise specified. The carboxyl group and the amino group of the neutral amino acid are used for formation of an adjacent moiety (-CO- or -L-) of X of formula (I). For example, when the carboxylic acid-type lipid (I) not falling under the scope of the claims has a structure of -CO-X-L- (e.g., when p is 0), the carboxyl group of the neutral amino acid residue is used for formation of -CO- of a structure of -CO-X-L-, and the amino group of the neutral amino acid is used for formation of -L- of a structure of -CO-X-L-. When the carboxylic acid-type lipid (I) not falling under the scope of the claims has a structure of -L-X-L- (e.g., when p is an integer of 1 or more), the carboxyl group of the neutral amino acid residue is used for formation of one -L- of a structure of -L-X-L-, and the amino group of the neutral amino acid is used for formation of the other -L- of a structure of -L-X-L-. Thus, the neutral amino acid residue represented by X is defined as a moiety obtained by removing a carboxyl group and an amino group from a neutral amino acid.

The neutral amino acid residue represented by X is preferably a neutral amino acid residue not having a reactive functional group (e.g., a hydroxyl group, a thiol group, etc.) in a side chain. Examples of a preferable neutral amino acid residue include a glycine residue, an alanine residue, a phenylalanine residue, a leucine residue, an isoleucine residue, a valine residue, a methionine residue, an asparagine residue, a glutamine residue and the like, and examples of a more preferable neutral amino acid residue include a glycine residue, an alanine residue, an asparagine residue, a glutamine residue and the like. When the neutral amino acid residue represented by X is a neutral amino acid residue in which a side chain is a hydrocarbon group, overlapping occurs with the case in which X is a hydrocarbon group. In terms of excluding the overlapping with the case in which X is a hydrocarbon group, the neutral amino acid residue represented by X is preferably a neutral amino acid residue in which a side chain is not a hydrocarbon group. Examples of the neutral amino acid residue in which a side chain is not a hydrocarbon group include a methionine residue, an asparagine residue, a glutamine residue and the like.

A polyalkylene glycol residue represented by X means a moiety obtained by removing functional groups (e.g., a carboxyl group, an amino group, a hydroxyl group, a thiol group, etc.) at both terminals from polyalkylene glycol or a polyalkylene glycol derivative unless otherwise specified. The polyalkylene glycol derivative is one in which one or both of hydroxyl groups at both terminals of polyalkylene glycol is/are substituted with another/other functional group(s) (e.g., a carboxyl group, an amino group, a thiol group, etc.). The functional groups (e.g., a carboxyl group, an amino group, a hydroxyl group, a thiol group and the like) at both terminals of polyalkylene glycol or the polyalkylene glycol derivative are used for formation of an adjacent moiety (-CO- or -L-) of X of formula (I). For example, when the functional groups at both terminals of a polyalkylene glycol derivative are a carboxyl group and a hydroxyl group and the carboxylic acid-type lipid (I) not falling under the scope of the claims has a structure of -CO-X-L- (e.g., when p is 0), the carboxyl group of the polyalkylene glycol derivative is used for formation of -CO- of a structure of -CO-X-L-, and the hydroxyl group of the polyalkylene glycol derivative is used for formation of -L- of a structure of -CO-X-L-. When the carboxylic acid-type lipid (I) not falling under the scope of the claims has a structure of -L-X-L- (e.g., when p is an integer of 1 or more), a functional group at one terminal of polyalkylene glycol or the polyalkylene glycol derivative is used for formation of one -L- of a structure of -L-X-L-, and a functional group at the other terminal is used for formation of the other -L- of a structure of -L-X-L-. Thus, the polyalkylene glycol residue represented by X is defined as a moiety obtained by removing functional groups at both terminals from polyalkylene glycol or a polyalkylene glycol derivative.

Examples of an alkylene glycol unit constituting polyalkylene glycol include ethylene glycol, propylene glycol, butylene glycol and the like. The alkylene glycol unit constituting polyalkylene glycol may be one or two or more.

Examples of the polyalkylene glycol include polyethylene glycol, polypropylene glycol, polytrimethylene glycol, polybutylene glycol, polytetramethylene glycol, polyoxyethyleneoxypropylene glycol and the like. The molecular weight of the polyalkylene glycol is preferably 400 to 40,000, more preferably 1,000 to 10,000, and still more preferably 2,000 to 5,000.

In formula (I), p represents an integer of 0 to 4, preferably an integer of 0 to 3, more preferably an integer of 0 to 2, and still more preferably an integer of 0 to 1.

### <Carboxylic Acid-Type Lipid (II) not falling under the scope of the claims>

The carboxylic acid-type lipid (II) not falling under the scope of the claims is represented by the following formula (II).

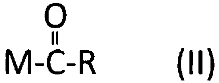

In formula (II), M and R are the same as defined above.

### <Carboxylic Acid-Type Lipid (III)>

The carboxylic acid-type lipid (III) is represented by the following formula (III). The meanings of a plurality of same symbols (e.g., L's, X's, q's, Y's, etc.) existing in formula (III) may be the same or different as long as they are within the range of the definition of the symbols.

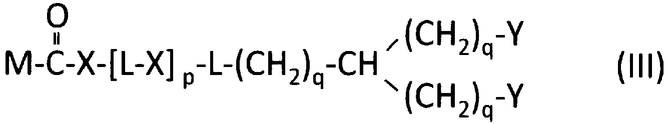

In formula (III), M, L, X and p are the same as defined above. The meaning of Y will be mentioned later. When a plurality of L's exist in formula (III), the meanings of these multiple L's may be the same or different as long as they are within the range of the definition of L. When a plurality of X's exist in formula (III), the meanings of these multiple X's may be the same or different as long as they are within the range of the definition of X.

In formula (III), q represents an integer of 0 to 8, preferably an integer of 0 to 6, more preferably an integer of 0 to 4, and still more preferably an integer of 0 to 2. Integers represented by a plurality of q's existing in formula (III) may be the same or different. The same applies to integers represented by a plurality of q's existing in other formulas.

### <Carboxylic Acid-Type Lipid (IV)>

The carboxylic acid-type lipid (IV) is represented by the following formula (IV). The meanings of a plurality of same symbols (e.g., q's, Y's, etc.) existing in formula (IV) may be the same or different as long as they are within the range of the definition of the symbols.

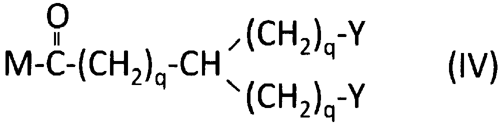

In formula (IV), M and q are the same as defined above. The meaning of Y will be mentioned later. Integers represented by a plurality of q's existing in formula (IV) may be the same or different.

### <Carboxylic Acid-Type Lipid (V)>

The carboxylic acid-type lipid (V) is represented by the following formula (V). The meanings of a plurality of same symbols (e.g., L's, X's, q's, Z's, etc.) existing in formula (V) may be the same or different as long as they are within the range of the definition of the symbols.

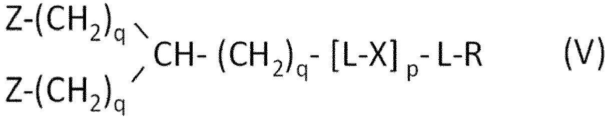

In formula (V), R, L, X, p and q are the same as defined above. The meaning of Z will be mentioned later. When a plurality of L's exist in formula (V), the meanings of these multiple L's may be the same or different as long as they are within the range of the definition of L. When a plurality of X's exist in formula (V), the meanings of these multiple X's may be the same or different as long as they are within the range of the definition of X. Integers represented by a plurality of q's existing in formula (V) may be the same or different.

### <Carboxylic Acid-Type Lipid (VI)>

The carboxylic acid-type lipid (VI) is represented by the following formula (VI). The meanings of a plurality of same symbols (e.g., L's, X's, q's, Y's, Z's, etc.) existing in formula (VI) may be the same or different as long as they are within the range of the definition of the symbols.

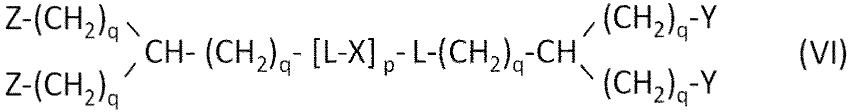

In formula (VI), L, X, p and q are the same as defined above. The meanings of Y and Z will be mentioned later. When a plurality of L's exist in formula (VI), the meanings of these multiple L's may be the same or different as long as they are within the range of the definition of L. When a plurality of X's exist in formula (VI), the meanings of these multiple X's may be the same or different as long as they are within the range of the definition of X. Integers represented by a plurality of q's existing in formula (VI) may be the same or different.

### <Branched Moiety>

In formulas (III), (IV), (V) and (VI), a branched moiety represented by the following formula (BP) is derived from, for example, a trifunctional compound (i.e., a residue of a trifunctional compound). A residue of a trifunctional compound means a moiety obtained by removing three reactive functional groups from a trifunctional compound unless otherwise specified. Three reactive functional groups of a trifunctional compound are used for formation of a moiety adjacent to the branched moiety (-CO- or -L-). Thus, the residue of a trifunctional compound is defined as a moiety obtained by removing three reactive functional groups from a trifunctional compound.

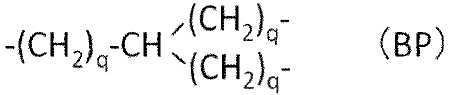

The trifunctional compound has first, second and third functional groups independently selected from a carboxyl group, an amino group, a hydroxyl group and a thiol group. The first and second functional groups may be the same or different, and are preferably different. The third functional group may be the same as or different from one or both of the first and second functional groups, and is preferably different from one or both of the first and second functional groups. Examples of the trifunctional compound include trifunctional amino acid and the like. The trifunctional amino acid is amino acid having a first functional group that is a carboxyl group, a second functional group that is an amino group and a third functional group selected from a carboxyl group, an amino group, a hydroxyl group and a thiol group. The third functional group is preferably different from one or both of the first and second functional groups. Examples of the trifunctional amino acid include amino acid having a carboxyl group and an amino group bonded to a-carbon and having a carboxyl group, an amino group, a hydroxyl group or a thiol group in a side chain. Examples of such amino acid include aspartic acid, glutamic acid, lysine, serine and the like.

In one embodiment, all of three q in the branched moiety are 0. In another embodiment, of three q in the branched moiety, one q is an integer of 1 or more, for example, 1, 2, 3 or 4, and the other two q are 0. In further another embodiment, of three q in the branched moiety, two q are the same or different and are an integer of 1 or more, for example, 1, 2, 3 or 4, and the other one q is 0. In still further another embodiment, three q in the branched moiety are the same or different and are an integer of 1 or more, for example, 1, 2, 3 or 4.

When the branched moiety is derived from aspartic acid, of three q, one q is 1, and the other two q are 0.

When the branched moiety is derived from glutamic acid, of three q, one q is 2, and the other two q are 0.

When the branched moiety is derived from lysine, of three q, one q is 4, and the other two q are 0.

When the branched moiety is derived from serine, of three q, one q is 1, and the other two q are 0.

### <Meaning of Y>

In formulas (III), (IV) and (VI), Y represents a branched chain composed of a branched chain body composed of one or more units Y1 and one or more groups Y2 bonded to the branched chain body, or represents a straight chain composed of one group Y2. The meanings of a plurality of Y's existing in each of formulas (III), (IV) and (VI) may be the same or different as long as they are within the range of the definition of Y. When a plurality of same symbols (e.g., R's, L's, X's, p's, q's, etc.) exist in a structure formula of each Y, the meanings of these same symbols may be the same or different as long as they are within the range of the definition of the symbols.

Each unit Y1 is represented by the following formula (VII). When Y includes two or more units Y1, the meanings of these units Y1 may be the same or different as long as they are within the range of the definition of Y1. When a plurality of same symbols (e.g., L's, X's, q's, etc.) exist in a structure formula of each Y1, the meanings of these same symbols may be the same or different as long as they are within the range of the definition of the symbols.

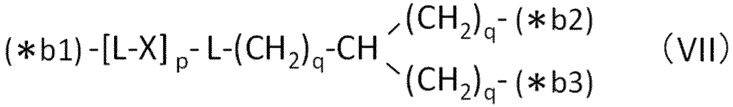

In formula (VII), L, X, p and q are the same as defined above. When a plurality of L's exist in formula (VII), the meanings of these multiple L's may be the same or different as long as they are within the range of the definition of L. When a plurality of X's exist in formula (VII), the meanings of these multiple X's may be the same or different as long as they are within the range of the definition of X. Integers represented by a plurality of q's existing in formula (VII) may be the same or different.

In formula (VII), (*b1), (*b2) and (*b3) represent a bond of each unit Y1.

Each group Y2 is represented by the following formula (VIII). When Y includes two or more groups Y2, the meanings of these groups Y2 may be the same or different as long as they are within the range of the definition of Y2. When a plurality of same symbols (e.g., L's, X's, etc.) exist in a structure formula of each Y2, the meanings of these same symbols may be the same or different as long as they are within the range of the definition of the symbols.

(*b4)-[L-X]p-L-R (VIII)

In formula (VIII), R, L, X and p are the same as defined above. When a plurality of L's exist in formula (VIII), the meanings of these multiple L's may be the same or different as long as they are within the range of the definition of L. When a plurality of X's exist in formula (VIII), the meanings of these multiple X's may be the same or different as long as they are within the range of the definition of X. When the carboxylic acid-type lipid of the present invention has a plurality of groups Y2, the meanings of R's included in the plurality of groups Y2 (R's in formula (VIII)) may be the same or different as long as they are within the range of the definition of R.

In formula (VIII), (*b4) represents a bond of each group Y2.

In one embodiment, Y represents a branched chain composed of a branched chain body composed of one or more units Y1 and one or more groups Y2 bonded to the branched chain body.

When the branched chain body is composed of one unit Y1, a bond (*b1) of the unit Y1 is bonded to (CH₂)_{q} in formula (III), (IV) or (VI). When the branched chain body is composed of one unit Y1, two groups Y2 are bonded to the branched chain body. A bond (*b4) of each group Y2 is bonded to a bond (*b2) or (*b3) of a unit Y1 constituting the branched chain body, and each group Y2 constitutes a terminal part of Y.

When the branched chain body is composed of two or more units Y1, a bond (*b1) of each unit Y1 is bonded to (CH₂)_{q} in formula (III), (IV) or (VI), or is bonded to a bond (*b2) or (*b3) of another unit Y1 constituting the branched chain body. In other words, when the branched chain body is composed of two or more units Y1, the branched chain body includes, in addition to one unit Y1 bonded to (CH₂)_{q} in formula (III), (IV) or (VI), one or more units Y1 in which a bond (*b1) is bonded to a bond (*b2) or (*b3) of another unit Y1. When the branched chain body is composed of two or more units Y1, (the number of units Y1 + 1) groups Y2 are bonded to the branched chain body. A bond (*b4) of each group Y2 is bonded to a bond (*b2) or (*b3) of any unit Y1 constituting the branched chain body, and each group Y2 constitutes a terminal part of Y.

In the embodiment in which Y represents a branched chain composed of a branched chain body composed of one or more units Y1 and one or more groups Y2 bonded to the branched chain body, the number of units Y1 included in Y is not particularly limited as long as it is 1 or more. The number of units Y1 included in Y is usually 1 to 4, preferably 1 to 3, more preferably 1 to 2, and still more preferably 1. The number of groups Y2 included in Y is determined according to the number of units Y1 included in Y. When the number of units Y1 is 1 or more, the number of groups Y2 bonded to the branched chain body is (the number of units Y1 + 1).

In another embodiment, Y represents a straight chain composed of one group Y2. In this embodiment, a bond (*b4) of the group Y2 is bonded to (CH₂)_{q} in formula (III), (IV) or (VI).

In the embodiment in which Y represents a straight chain composed of a group Y2, Y does not include a unit Y1 (the number of units Y1 is 0), and the number of groups Y2 included in Y is 1.

Each Y can be selected from, for example, straight and branched chains represented by the following formulas (XIII), (XIV), (XV) and (XVI).

(_{*}b)-Y2 (XIII)

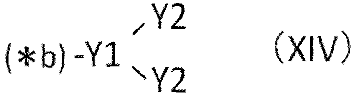

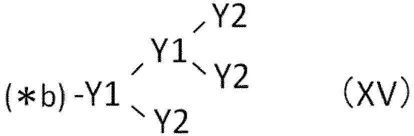

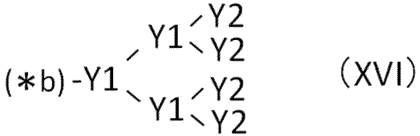

In formulas (XIII) to (XVI), Y1 represents one unit Y1, Y2 represents one group Y2, and (*b) represents a bond of the unit Y1 bonded to (CH₂)_{q} in formula (III), (IV) or (VI).

### <Meaning of Z>

In formulas (V) and (VI), Z represents a branched chain composed of a branched chain body composed of one or more units Z1 and one or more groups Z2 bonded to the branched chain body, or represents a straight chain composed of one group Z2. The meanings of a plurality of Z's existing in each of formulas (V) and (VI) may be the same or different as long as they are within the range of the definition of Z. When a plurality of same symbols (e.g., M's, L's, X's, p's, q's, etc.) exist in a structure formula of each Z, the meanings of these same symbols may be the same or different as long as they are within the range of the definition of the symbols.

Each unit Z1 is represented by the following formula (IX). When Z includes two or more units Z1, the meanings of these units Z1 may be the same or different as long as they are within the range of the definition of Z1. When a plurality of same symbols (e.g., L's, X's, q's, etc.) exist in a structure formula of each Z1, the meanings of these same symbols may be the same or different as long as they are within the range of the definition of the symbols.

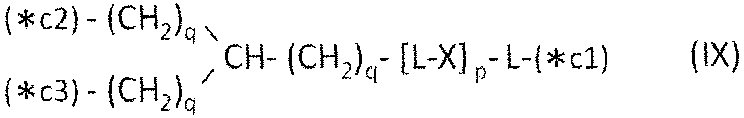

In formula (IX), L, X, p and q are the same as defined above. When a plurality of L's exist in formula (IX), the meanings of these multiple L's may be the same or different as long as they are within the range of the definition of L. When a plurality of X's exist in formula (IX), the meanings of these multiple X's may be the same or different as long as they are within the range of the definition of X. Integers represented by a plurality of q's existing in formula (IX) may be the same or different.

In formula (IX), (*c1), (*c2) and (*c3) represent a bond of each unit Z1.

Each group Z2 is selected from groups represented by the following formulas (X) and (XI).

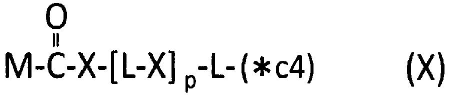

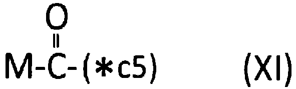

In formula (X), M, L, X and p are the same as defined above, and (*c4) represents a bond of each group Z2. In formula (XI), M is the same as defined above, and (*c5) represents a bond of each group Z2. When a plurality of L's exist in formula (X), the meanings of these multiple L's may be the same or different as long as they are within the range of the definition of L. When a plurality of X's exist in formula (X), the meanings of these multiple X's may be the same or different as long as they are within the range of the definition of X. When the carboxylic acid-type lipid of the present invention has a plurality of groups Z2, the meanings of R's included in the plurality of groups Z2 (R's in formula (X) or (XI)) may be the same or different as long as they are within the range of the definition of R.

In one embodiment, Z represents a branched chain composed of a branched chain body composed of one or more units Z1 and one or more groups Z2 bonded to the branched chain body.

When the branched chain body is composed of one unit Z1, a bond (*c1) of the unit Z1 is bonded to (CH₂)_{q} in formula (V) or (VI). When the branched chain body is composed of one unit Z1, two groups Z2 are bonded to the branched chain body. A bond (*c4) or (*c5) of each group Z2 is bonded to a bond (*c2) or (*c3) of a unit Z1 constituting the branched chain body, and each group Z2 constitutes a terminal part of Z.

When the branched chain body is composed of two or more units Z1, a bond (*c1) of each unit Z1 is bonded to (CH₂)_{q} in formula (V) or (VI), or is bonded to a bond (*c2) or (*c3) of another unit Z1 constituting the branched chain body. In other words, when the branched chain body is composed of two or more units Z1, the branched chain body includes, in addition to one unit Z1 bonded to (CH₂)_{q} in formula (V) or (VI), one or more units Z1 in which a bond (*c1) is bonded to a bond (*c2) or (*c3) of another unit Z1. When the branched chain body is composed of two or more units Z1, (the number of units Z1 + 1) groups Z2 are bonded to the branched chain body. A bond (*c4) or (*c5) of each group Z2 is bonded to a bond (*c2) or (*c3) of any unit Z1 constituting the branched chain body, and each group Z2 constitutes a terminal part of Z.

In the embodiment in which Z represents a branched chain composed of a branched chain body composed of one or more units Z1 and one or more groups Z2 bonded to the branched chain body, the number of units Z1 included in Z is not particularly limited as long as it is 1 or more. The number of units Z1 included in Z is usually 1 to 4, preferably 1 to 3, more preferably 1 to 2, and still more preferably 1. The number of groups Z2 included in Z is determined according to the number of units Z1 included in Z. When the number of units Z1 is 1 or more, the number of groups Z2 bonded to the branched chain body is (the number of units Z1 + 1).

In another embodiment, Z represents a straight chain composed of one group Z2. In this embodiment, a bond (*c4) or (*c5) of the group Z2 is bonded to (CH₂)_{q} in formula (V) or (VI).

In the embodiment in which Z represents a straight chain composed of a group Z2, Z does not include a unit Z1 (the number of units Z1 is 0), and the number of groups Z2 included in Z is 1.

Each Z can be selected from, for example, straight and branched chains represented by the following formulas (XVII), (XVIII), (XIX) and (XX).

Z2-(*c) (XVII)

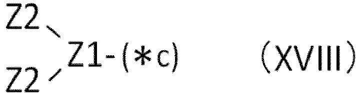

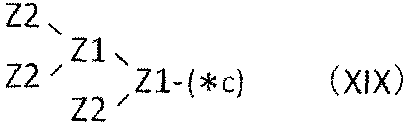

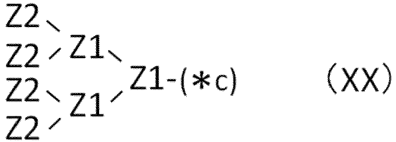

In formulas (XVII) to (XX), Z1 represents one unit Z1, Z2 represents one group Z2, and (*c) represents a bond of the unit Z1 bonded to (CH₂)_{q} in formula (V) or (VI).

### <Method for Producing Carboxylic Acid-Type Lipid (I) not falling under the scope of the claims>

One embodiment of a method for producing the carboxylic acid-type lipid (I) not falling under the scope of the claims will be described. When a plurality of same symbols (e.g., L's, X's, etc.) exist in a structure formula of one certain compound, the meanings of these same symbols may be the same or different as long as they are within the range of the definition of the symbols. When the same symbols (e.g., L, X, etc.) exist in structure formulas of two or more compounds, the meanings of these same symbols may be the same or different as long as they are within the range of the definition of the symbols.

### (Step 1A)

When M is M₀-NH₂, a compound (1) represented by the following formula:

M₀-NH₂

wherein M₀ is the same as defined above,
is provided.

The compound (1) can be produced in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. The compound (1) may be a commercially available product.

### (Step 2A)

A compound (2) represented by the following formula:

HOOC-X-A₁

wherein X is the same as defined above, and A₁ represents a carboxyl group, an amino group, a hydroxyl group or a thiol group, is provided.

The compound (2) can be produced in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. The compound (2) may be a commercially available product.

When X is a hydrocarbon group, A₁ is selected from a carboxyl group, an amino group, a hydroxyl group and a thiol group.

When X is a hydrocarbon group and A₁ is a carboxyl group, examples of the compound (2) include aliphatic dicarboxylic acid and the like. Examples of the aliphatic dicarboxylic acid include malonic acid, succinic acid, glutaric acid, 2-methylglutaric acid, 3-methylglutaric acid, adipic acid, pimelic acid, suberic acid, azelaic acid, sebacic acid, dodecanedioic acid, maleic acid, fumaric acid, citraconic acid, mesaconic acid, 2-pentenedioic acid, itaconic acid, allylmalonic acid, isopropylidenesuccinic acid, 2,2,4-trimethyladipic acid, 2,4,4-trimethyladipic acid, 2,4-hexadienedioic acid, acetylenedicarboxylic acid and the like. The aliphatic dicarboxylic acid may be acid anhydride.

When X is a hydrocarbon group and A₁ is an amino group, examples of the compound (2) include neutral amino acid in which a side chain is a hydrocarbon group and the like. Examples of the neutral amino acid in which a side chain is a hydrocarbon group include glycine, alanine, phenylalanine, leucine, isoleucine, valine and the like.

When X is a hydrocarbon group and A₁ is a hydroxyl group, examples of the compound (2) include aliphatic hydroxycarboxylic acid and the like. Examples of the aliphatic hydroxycarboxylic acid include glycolic acid, lactic acid, hydroxybutyric acid, hydroxyvaleric acid, hydroxycaproic acid, hydroxycapric acid and the like.

When X is a hydrocarbon group and A₁ is a thiol group, examples of the compound (2) include aliphatic carboxylic acid thiol and the like. Examples of the aliphatic carboxylic acid thiol include 2-mercaptopropionic acid, 3-mercaptopropionic acid, 3-mercaptobutanoic acid, 2-mercaptoisobutyric acid, 3-mercaptoisobutyric acid, 3-mercapto-3-methylbutyric acid, 2-mercaptovaleric acid, 3-mercaptoisovaleric acid, 4-mercaptovaleric acid, 3-phenyl-3mercaptopropionic acid and the like.

When X is a neutral amino acid residue, A₁ is an amino group, and the compound (2) is neutral amino acid. Examples of the neutral amino acid include a glycine residue, an alanine residue, a phenylalanine residue, a leucine residue, an isoleucine residue, a valine residue, a methionine residue, an asparagine residue, a glutamine residue and the like. In terms of excluding the overlapping with the case in which X is a hydrocarbon group, the neutral amino acid is preferably neutral amino acid in which a side chain is not a hydrocarbon group. Examples of the neutral amino acid in which a side chain is not a hydrocarbon group include methionine, asparagine, glutamine and the like.

When X is a polyalkylene glycol residue, A₁ is a carboxyl group, an amino group, a hydroxyl group or a thiol group, and the compound (2) is a polyalkylene glycol derivative having a carboxyl group at one terminal and having a carboxyl group, a hydroxyl group, an amino group or a thiol group at the other terminal. Polyalkylene glycol derivatives in which various functional groups are introduced into one or both terminals are commercially available.

### (Step 3A)

A compound (3) represented by the following formula (3):

D₁-X-[L-X]ₚ₋₁-Eₚ (3)

wherein L and X are the same as defined above, and p represents an integer of 1 or more, and D₁ and Eₚ each independently represent a carboxyl group, an amino group, a hydroxyl group or a thiol group,
is provided, as necessary.

A functional group D₁ is a functional group that can be reacted with the functional group A₁ of the compound (2), and is selected from a carboxyl group, an amino group, a hydroxyl group and a thiol group. When A₁ is a carboxyl group, D₁ is an amino group, a hydroxyl group or a thiol group. When A₁ is an amino group, D₁ is a carboxyl group. When A₁ is a hydroxyl group, D₁ is a carboxyl group. When A₁ is a thiol group, D₁ is a carboxyl group or a thiol group.

The compound (3) can be produced in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. The compound (3) may be a commercially available product.

One embodiment of a method for producing the compound (3) will be described.

According to an integer represented by p, a compound (3-1) represented by the formula: D₁-X-E₁, a compound (3-2) represented by the formula: D₂-X-E₂, a compound (3-3) represented by the formula: D₃-X-E₃, ..., a compound (3-p) represented by the formula: Dₚ-X-Eₚ are provided. For example, when p is 1, the compound (3-1) is provided, when p is 2, the compound (3-1) and the compound (3-2) are provided, and when p is 3, the compound (3-1), the compound (3-2) and the compound (3-3) are provided.

A functional group D₁ and a functional group Eₚ are the same as defined above.

Functional groups E₁ to Eₚ₋₁ are each independently selected from a carboxyl group, an amino group, a hydroxyl group and a thiol group.

Functional groups D₂ to Dₚ each are functional groups that can be reacted with the functional groups E₁ to Eₚ₋₁, and are each independently selected from a carboxyl group, an amino group, a hydroxyl group and a thiol group. For example, the functional group D₂ is a functional group that can be reacted with the functional group E₁, the functional group D₃ is a functional group that can be reacted with the functional group E₂, and the functional group Dₚ is a functional group that can be reacted with the functional group Eₚ₋₁. Specific examples of a combination of functional groups that can be reacted are the same as the specific examples of the combination of the functional group A₁ and the functional group D₁.

The compounds (3-1) to (3-p) are generalized by a compound (3-k) represented by the formula: Dₖ-X-Eₖ (k = 1, 2, ..., p), and the compound (3-k) will be described.

When X is a hydrocarbon group, Dₖ and Eₖ are each independently selected from a carboxyl group, an amino group, a hydroxyl group and a thiol group.

When X is a hydrocarbon group, Dₖ is a carboxyl group, and Eₖ is a carboxyl group, examples of the compound (3-k) include aliphatic dicarboxylic acid and the like. Specific examples of the aliphatic dicarboxylic acid are the same as mentioned above. The aliphatic dicarboxylic acid may be acid anhydride.

When X is a hydrocarbon group, Dₖ is a carboxyl group, and Eₖ is an amino group, examples of the compound (3-k) include neutral amino acid in which a side chain is a hydrocarbon group, and the like. Specific examples of the neutral amino acid in which a side chain is a hydrocarbon group are the same as mentioned above.

When X is a hydrocarbon group, Dₖ is a carboxyl group, and Eₖ is a hydroxyl group, examples of the compound (3-k) include aliphatic hydroxycarboxylic acid and the like. Specific examples of the aliphatic hydroxycarboxylic acid are the same as mentioned above.

When X is a hydrocarbon group, Dₖ is a carboxyl group, and Eₖ is a thiol group, examples of the compound (3-k) include aliphatic carboxylic acid thiol and the like. Specific examples of the aliphatic carboxylic acid thiol are the same as mentioned above.

When X is a hydrocarbon group, Dₖ is an amino group, and Eₖ is a carboxyl group, examples of the compound (3-k) include neutral amino acid in which a side chain is a hydrocarbon group and the like. Specific examples of the neutral amino acid in which a side chain is a hydrocarbon group are the same as mentioned above.

When X is a hydrocarbon group, Dₖ is an amino group, and Eₖ is an amino group, examples of the compound (3-k) include aliphatic diamine and the like. Examples of the aliphatic diamine include 1,4-butanediamine, 1,5-pentanediamine, 1,2-ethanediamine, 1,3-propanediamine, 1,6-hexanediamine and the like.

When X is a hydrocarbon group, Dₖ is an amino group, and Eₖ is a hydroxyl group, examples of the compound (3-k) include aliphatic hydroxy amine and the like. Examples of the aliphatic hydroxy amine include monoethanolamine, diethanolamine, triethanolamine, monopropanolamine, dipropanolamine, tripropanolamine, monobutanolamine, dibutanolamine, tributanolamine, N-methyl-diethanolamine, N,N-dimethylmonoethanolamine, aminomethyl propanol and the like.

When X is a hydrocarbon group, Dₖ is an amino group, and Eₖ is a thiol group, examples of the compound (3-k) include aliphatic amine having a thiol group, and the like. Examples of the aliphatic amine having a thiol group include cysteamine, N-alkylcysteamine, 3-aminopropanethiol, 4-aminobutanethiol and the like.

When X is a hydrocarbon group, Dₖ is a hydroxyl group, and Eₖ is a carboxyl group, examples of the compound (3-k) include aliphatic hydroxycarboxylic acid and the like. Specific examples of the aliphatic hydroxycarboxylic acid are the same as mentioned above.

When X is a hydrocarbon group, Dₖ is a hydroxyl group, and Eₖ is an amino group, examples of the compound (3-k) include aliphatic hydroxy amine and the like. Specific examples of the aliphatic hydroxy amine are the same as mentioned above.

When X is a hydrocarbon group, Dₖ is a hydroxyl group, and Eₖ is a hydroxyl group, examples of the compound (3-k) include aliphatic diol and the like. Examples of the aliphatic diol include ethylene glycol, 1,3-propylene glycol, 1,2-propylene glycol, 1,2-butylene glycol, 1,4-butylene glycol, isopentanediol, 1,2-pentanediol, 1,3-pentanediol, 1,4-pentanediol, 1,5-pentanediol, 1,2-hexanediol, 1,3-hexanediol, 1,4-hexanediol, 1,5-hexanediol, 1,6-hexanediol, 3-methyl-1,5-pentanediol, 2-methyl-2,4-pentanediol, 1,2-heptanediol, 1,3-heptanediol, 1,4-heptanediol, 1,5-heptanediol, 1,6-heptanediol, 1,7-heptanediol, 2,4-heptanediol, 3,4-heptanediol, 1,2-octanediol, 2,3-octanediol, 2-ethyl-1,3-hexanediol, 2-butyl-2-ethyl-1,3-propanediol, 2,5-dimethyl-2,5-hexanediol and the like.

When X is a hydrocarbon group, Dₖ is a hydroxyl group, and Eₖ is a thiol group, examples of the compound (3-k) include aliphatic alcohol having a thiol group, and the like. Examples of the aliphatic alcohol having a thiol group include 2-mercaptoethanol, 3-mercapto-1-propanol, 3-mercapto-2-propanol, 4-mercapto-1-butanol, 4-mercapto-2-butanol, 4-mercapto-3-butanol, 1-mercapto-1,1-methanediol, 1-mercapto-1,1-ethanediol, 3-mercapto-1,2-propanediol (a-thioglycerol), 2-mercapto-1,2-propanediol, 2-mercapto-2-methyl-1,3-propanediol, 2-mercapto-2-ethyl-1,3-propanediol, 1-mercapto-2,2-propanediol, 2-mercaptoethyl-2-methyl-1,3-propanediol, 2-mercaptoethyl-2-ethyl-1,3-propanediol and the like.

When X is a hydrocarbon group, Dₖ is a thiol group, and Eₖ is a carboxyl group, examples of the compound (3-k) include aliphatic carboxylic acid thiol and the like. Specific examples of the aliphatic carboxylic acid thiol are the same as mentioned above.

When X is a hydrocarbon group, Dₖ is a thiol group, and Eₖ is an amino group, examples of the compound (3-k) include aliphatic amine having a thiol group, and the like. Specific examples of the aliphatic amine having a thiol group are the same as mentioned above.

When X is a hydrocarbon group, Dₖ is a thiol group, and Eₖ is a hydroxyl group, examples of the compound (3-k) include aliphatic alcohol having a thiol group, and the like. Specific examples of the aliphatic alcohol having a thiol group are the same as mentioned above.

When X is a hydrocarbon group, Dₖ is a thiol group, and Eₖ is a thiol group, examples of the compound (3-k) include aliphatic dithiol and the like. Examples of the aliphatic dithiol include 1,4-butanedithiol, ethanedithiol and the like.

When X is a neutral amino acid residue, one of Dₖ and Eₖ is a carboxyl group, the other is an amino group, and the compound (3-k) is a neutral amino acid. Examples of the neutral amino acid include a glycine residue, an alanine residue, a phenylalanine residue, a leucine residue, an isoleucine residue, a valine residue, a methionine residue, an asparagine residue, a glutamine residue and the like. In terms of excluding the overlapping with the case in which X is a hydrocarbon group, the neutral amino acid is preferably neutral amino acid in which a side chain is not a hydrocarbon group. Examples of the neutral amino acid in which a side chain is not a hydrocarbon group include methionine, asparagine, glutamine and the like.

When X is a polyalkylene glycol residue, Dₖ and Eₖ are each independently selected from a carboxyl group, an amino group, a hydroxyl group and a thiol group, and the compound (3-k) is polyalkylene glycol having hydroxyl groups at both terminals, a polyalkylene glycol derivative having a hydroxyl group at one terminal and having a carboxyl group, an amino group or a thiol group at the other terminal, or a polyalkylene glycol derivative having a carboxyl group, an amino group or a thiol group each independently at both terminals. Polyalkylene glycol derivatives in which various functional groups are introduced into one or both terminals are commercially available.

The functional group E₁ of the compound (3-1) is reacted with the functional group D₂ of the compound (3-2) in accordance with a conventional method to produce a compound represented by the formula: D₁-X-L-X-E₂, and then the functional group E₂ of the produced compound is reacted with the functional group D₃ of the compound (3-3) in accordance with a conventional method to produce a compound represented by the formula: D₁-X-L-X-L-X-E₃. The same step is repeated to produce a compound represented by the formula: D₁-X-[L-X]ₚ₋₂-Eₚ₋₁, and the functional group Eₚ₋₁ of the produced compound is reacted with the functional group Dₚ of the compound (3-p) in accordance with a conventional method to produce a compound (3) represented by the formula: D₁-X-[L-X]ₚ₋₁-Eₚ. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method.

When E₁ is a carboxyl group and D₂ is an amino group, L formed by the reaction of both groups is -CO-NH-. When E₁ is a carboxyl group and D₂ is a hydroxyl group, L formed by the reaction of both groups is -CO-O-. When E₁ is a carboxyl group and D₂ is a thiol group, L formed by the reaction of both groups is -CO-S-. When E₁ is an amino group and D₂ is a carboxyl group, L formed by the reaction of both groups is -NH-CO-. When E₁ is a hydroxyl group and D₂ is a carboxyl group, L formed by the reaction of both groups is -O-CO-. When E₁ is a thiol group and D₂ is a carboxyl group, L formed by the reaction of both groups is -S-CO-. When E₁ is a thiol group and D₂ is a thiol group, L formed by the reaction of both groups is -S-S-. Specific examples of L formed by the reaction of other two functional groups are the same as the specific examples of L formed by the reaction of the functional group E₁ and the functional group D₂.

### (Step 4A)

A compound (4) represented by the following formula (4):

A₂-R (4)

wherein R is the same as defined above, and A₂ represents a carboxyl group, an amino group, a hydroxyl group or a thiol group, is provided.

A functional group A₂ is a functional group that can be reacted with the functional group A₁ of the compound (2) or the functional group Eₚ of the compound (3), and is selected from a carboxyl group, an amino group, a hydroxyl group and a thiol group. Specific examples of a combination of functional groups that can be reacted are the same as the specific examples of the combination of the functional group A₁ and the functional group D₁.

The compound (4) can be produced in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. The compound (4) may be a commercially available product.

When A₂ is a carboxyl group, examples of the compound (4) include linear or branched saturated or unsaturated aliphatic carboxylic acid and the like. Examples of the aliphatic carboxylic acid include acetic acid, propionic acid, butyric acid, valeric acid, isovaleric acid, caproic acid, enanthic acid, caprylic acid, undecanoic acid, lauric acid, tridecanoic acid, myristic acid, pentadecanoic acid, palmitic acid, heptadecanoic acid, stearic acid, nonadecanoic acid, arachic acid, behenic acid, palmitoleic acid, oleic acid, linoleic acid, linoleic acid, arachidonic acid and the like.

When A₂ is an amino group, examples of the compound (4) include linear or branched saturated or unsaturated aliphatic amine and the like. The aliphatic amine may be any one of primary aliphatic amine and secondary aliphatic amine, and is preferably primary aliphatic amine. Examples of the aliphatic amine include dodecylamine, tridecylamine, tetradecylamine, pentadecylamine, hexadecylamine, heptadecylamine, octadecylamine, docosylamine, oleylamine, N-methyl-dodecylamine, N-methyl-tetradecylamine, N-methyl-hexadecylamine, N-ethyl-dodecylamine, N-ethyl-tetradecylamine, N-ethyl-hexadecylamine, N-propyl-dodecylamine, N-propyl-tetradecylamine, N-propyl-hexadecylamine, dioleylamine and the like.

When A₂ is a hydroxyl group, examples of the compound (4) include linear or branched saturated or unsaturated aliphatic alcohol and the like. The aliphatic alcohol may be any one of primary aliphatic alcohol, secondary aliphatic alcohol and tertiary aliphatic alcohol, and is preferably primary aliphatic alcohol. Examples of the aliphatic alcohol include lauryl alcohol, cetyl alcohol, stearyl alcohol, behenyl alcohol, 1,1-dodecenol, 1-oley alcohol, linolenyl alcohol and the like. The compound (4) may be dialkyl glycerol in which aliphatic alcohol is ether bonded to position 1 and position 3 or position 1 and position 2 of glycerin.

When A₂ is a thiol group, examples of the compound (4) include linear or branched saturated or unsaturated aliphatic thiol and the like. Examples of the aliphatic thiol include methanethiol, ethanethiol, propanethiol, butanethiol, pentanethiol, hexanethiol, heptanethiol, octanethiol, nonanethiol, decanethiol, undecanethiol, hexadecanethiol, octadecanethiol and the like.

### (Step 5A)

When p in formula (I) is 0, a carboxylic acid-type lipid (I) not falling under the scope of the claims in which M is M₀-NH- is produced: by reacting the functional group A₁ of the compound (2) with the functional group A₂ of the compound (4) in accordance with a conventional method to produce a carboxylic acid-type lipid (I) not falling under the scope of the claims in which M is HO- , and then reacting the carboxyl group of the carboxylic acid-type lipid (I) not falling under the scope of the claims in which M is HO-, with the amino group of the compound (1); or by reacting the amino group of the compound (1) with the carboxyl group of the compound (2) in accordance with a conventional method to produce a compound represented by the formula: M₀-NH-CO-X-A₁, and then reacting the functional group A₁ of the produced compound with the functional group A₂ of the compound (4) in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. Specific examples of L formed by the reaction of two functional groups are the same as the specific examples of L formed by the reaction of the functional group E₁ and the functional group D₂.

When p in formula (I) is 1 or more, a carboxylic acid-type lipid (I) not falling under the scope of the claims in which M is HO- is produced: by reacting the functional group Eₚ of the compound (3) with the functional group A₂ of the compound (4) in accordance with a conventional method to produce a compound represented by the formula: D₁-X-[L-X]ₚ₋₁-L-R, and then reacting the functional group D₁ of the produced compound with the functional group A₁ of the compound (2) in accordance with a conventional method; or by reacting the functional group A₁ of the compound (2) with the functional group D₁ of the compound (3) in accordance with a conventional method to produce a compound represented by the formula: HOOC-X-[L-X]ₚ-Eₚ, and then reacting the functional group Eₚ of the produced compound with the functional group A₂ of the compound (4) in accordance with a conventional method. A carboxylic acid-type lipid (I) not falling under the scope of the claims in which M is M₀-NH- is produced by reacting the carboxyl group of the carboxylic acid-type lipid (I) not falling under the scope of the claims in which M is HO-, with the amino group of the compound (1). In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. Specific examples of L formed by the reaction of two functional groups are the same as the specific examples of L formed by the reaction of the functional group E₁ and the functional group D₂.

As mentioned above, the carboxylic acid-type lipid (I) not falling under the scope of the claims can be produced by a method including step 1A to step 5A. In each step, the order of reaction can be appropriately changed as long as a desired compound can be produced.

### <Method for Producing Carboxylic Acid-Type Lipid (II) not falling under the scope of the claims >

One embodiment of a method for producing the carboxylic acid-type lipid (II) not falling under the scope of the claims will be described.

### (Step 1B)

When M is M₀-NH-, a compound (1) is provided.

### (Step 2B)

A compound (4) in which A₂ is a carboxyl group is provided.

### (Step 3B)

The compound (4) is a carboxylic acid-type lipid (II) in which M is HO-.

A carboxylic acid-type lipid (II) not falling under the scope of the claims in which M is M₀-NH- is produced by reacting the amino group of the compound (1) with the carboxyl group of the compound (4) in which A₂ is a carboxyl group in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method.

As mentioned above, the carboxylic acid-type lipid (II) not falling under the scope of the claims can be produced by a method including step 1B to step 3B. In each step, the order of reaction can be appropriately changed as long as a desired compound can be produced.

### <Method for Producing Carboxylic Acid-Type Lipid (III)>

One embodiment of a method for producing the carboxylic acid-type lipid (III) will be described. When two or more same symbols (e.g., L, X, p, q, etc.) exist in a structure formula of one certain compound, the meanings of these same symbols may be the same or different as long as they are within the range of the definition of the symbols. When the same symbols (e.g., L, X, p, q, etc.) exist in structure formulas of two or more compounds, the meanings of these same symbols may be the same or different as long as they are within the range of the definition of the symbols.

### (Step 1C)

When M is M₀-NH-, a compound (1) is provided.

### (Step 2C)

A compound (2) is provided.

### (Step 3C)

A compound (3) is provided, as necessary.

### (Step 4C)

A compound (5) represented by the following formula (5) is provided.

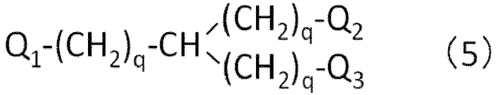

In formula (5), q is the same as defined above. A plurality of q's existing in formula (5) may represent the same integers or may represent different integers.

In formula (5), Q₁ is a functional group that can be reacted with the functional group A₁ of the compound (2) or the functional group Eₚ of the compound (3), and is selected from a carboxyl group, an amino group, a hydroxyl group and a thiol group. Specific examples of a combination of functional groups that can be reacted are the same as the specific examples of the combination of the functional group A₁ and the functional group D₁.

In formula (5), Q₂ and Q₃ each independently represent a carboxyl group, an amino group, a hydroxyl group or a thiol group. Q₂ and Q₃ may be the same or different.

In formula (5), Q₁ may be the same as or different from one or both of Q₂ and Q₃. When Q₁ is different from one or both of Q₂ and Q₃, it becomes easy to select a protecting group for protection of Q₁. From this point of view, it is preferable that Q₁ is different from one or both of Q₂ and Q₃.

The compound (5) is not particularly limited as long as it is a trifunctional compound. The compound (5) is preferably trifunctional amino acid. The trifunctional amino acid is amino acid having a first functional group that is a carboxyl group, a second functional group that is an amino group and a third functional group selected from a carboxyl group, an amino group, a hydroxyl group and a thiol group. The third functional group is preferably different from one or both of the first and second functional groups. Examples of the trifunctional amino acid include amino acid having a carboxyl group and an amino group bonded to α-carbon and having a carboxyl group, an amino group, a hydroxyl group or a thiol group in a side chain. Examples of such amino acid include lysine, aspartic acid, glutamic acid, serine and the like.

### (Step 5C)

A compound (6) represented by the following formula (6) is provided, as necessary.

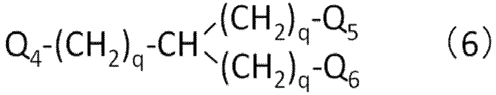

In formula (6), q is the same as defined above. A plurality of q's existing in formula (6) may represent the same integers or may represent different integers.

In formula (6), Q₄ is a functional group that can be reacted with Q₂ or Q₃ of the compound (5), Q₅ or Q₆ of another compound (6) or a functional group Gₚ of a compound (7) mentioned later, and is selected from a carboxyl group, an amino group, a hydroxyl group and a thiol group. Specific examples of a combination of functional groups that can be reacted are the same as the specific examples of the combination of the functional group A₁ and the functional group D1.

In formula (6), Q₅ and Q₆ each independently represent a carboxyl group, an amino group, a hydroxyl group or a thiol group. Q₅ and Q₆ may be the same or different.

In formula (6), Q₄ may be the same as or different from one or both of Q₅ and Q₆. When Q₄ is different from one or both of Q₅ and Q₆, it becomes easy to select a protecting group for protection of Q₄. From this point of view, it is preferable that Q₄ is different from one or both of Q₅ and Q₆.

The compound (6) is not particularly limited as long as it is a trifunctional compound. The compound (6) is preferably trifunctional amino acid. The description on the trifunctional amino acid is the same as mentioned above.

### (Step 6C)

A compound (7) represented by the following formula (7):

F₁-X-[L-X]ₚ₋₁-Gₚ (7)

wherein L and X are the same as defined above, and p represents an integer of 1 or more, and F₁ and Gₚ each independently represent a carboxyl group, an amino group, a hydroxyl group or a thiol group,
is provided, as necessary.

A functional group F₁ is a functional group that can be reacted with the functional group Q₂ or Q₃ of the compound (5) or the functional group Q₅ or Q₆ of the compound (6), and is selected from a carboxyl group, an amino group, a hydroxyl group and a thiol group. Specific examples of a combination of functional groups that can be reacted are the same as the specific examples of the combination of the functional group A₁ and the functional group D₁.

The compound (7) can be produced in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. The compound (7) may be a commercially available product.

One embodiment of a method for producing the compound (7) is the same as one embodiment of a method for producing the compound (3).

### (Step 7C)

A compound (8) represented by the following formula (8):

H₁-X-[L-X]ₚ₋₁-Iₚ (8)

wherein L and X are the same as defined above, and p represents an integer of 1 or more, and H₁ and Iₚ each independently represent a carboxyl group, an amino group, a hydroxyl group or a thiol group,
is provided, as necessary.

A functional group H₁ is a functional group that can be reacted with the functional group Q₂ or Q₃ of the compound (5) or the functional group Q₅ or Q₆ of the compound (6), and is selected from a carboxyl group, an amino group, a hydroxyl group and a thiol group. Specific examples of a combination of functional groups that can be reacted are the same as the specific examples of the combination of the functional group A₁ and the functional group D₁.

The compound (8) can be produced in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection by a protecting group can be performed in accordance with a conventional method. The compound (8) may be a commercially available product.

One embodiment of a method for producing the compound (8) is the same as one embodiment of a method for producing the compound (3).

### (Step 8C)

A compound (9) represented by the following formula (9):

A₃-R (9)

wherein R is the same as defined above, and A₃ represents a carboxyl group, an amino group, a hydroxyl group or a thiol group, is provided.

A functional group A₃ is a functional group that can be reacted with the functional group Q₂ or Q₃ of the compound (5), the functional group Q₅ or Q₆ of the compound (6) or the functional group Iₚ of the compound (8), and is selected from a carboxyl group, an amino group, a hydroxyl group and a thiol group. Specific examples of a combination of functional groups that can be reacted are the same as the specific examples of the combination of the functional group A₁ and the functional group D₁.

The compound (9) can be produced in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. The compound (9) may be a commercially available product. Specific examples of the compound (9) are the same as the specific examples of the compound (4).

### (Step 9C)

A compound (5-Y) represented by the following formula (5-Y) is produced by introducing a straight chain or a branched chain into the functional groups Q₂ and Q₃ of the compound (5). In formula (5-Y), Y is the same as defined above.

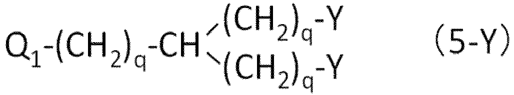

The case in which a straight chain or a branched chain is introduced into the functional group Q₂ of the compound (5) will be described, and a straight chain or a branched chain can also be similarly introduced into the functional group Q₃ of the compound (5).

When a straight chain is introduced into the functional group Q₂ of the compound (5), a compound (10) represented by the following formula (10) is produced.

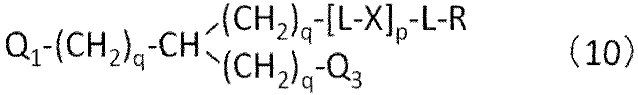

When p in formula (10) is 0, a compound (10) is produced by reacting the functional group Q₂ of the compound (5) with the functional group A₃ of the compound (9) in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. Specific examples of L formed by the reaction of two functional groups are the same as the specific examples of L formed by the reaction of the functional group E₁ and the functional group D₂.

When p in formula (10) is 1 or more, a compound (10) is produced: by reacting the functional group Q₂ of the compound (5) with the functional group H₁ of the compound (8) in accordance with a conventional method, and then reacting the functional group Iₚ of the obtained compound with the functional group A₃ of the compound (9) in accordance with a conventional method; or by reacting the functional group Iₚ of the compound (8) with the functional group A₃ of the compound (9) in accordance with a conventional method, and then reacting the functional group H₁ of the obtained compound with the functional group Q₂ of the compound (5) in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. Specific examples of L formed by the reaction of two functional groups are the same as the specific examples of L formed by the reaction of the functional group E₁ and the functional group D₂.

When a branched chain is introduced into the functional group Q₂ of the compound (5), a compound (11) represented by the following formula (11) is produced.

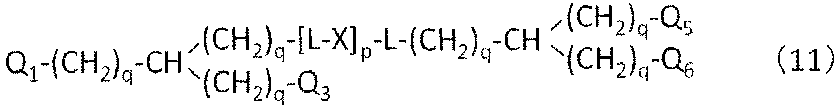

When p in formula (11) is 0, a compound (11) is produced by reacting the functional group Q₂ of the compound (5) with the functional group Q₄ of the compound (6) in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. Specific examples of L formed by the reaction of two functional groups are the same as the specific examples of L formed by the reaction of the functional group E₁ and the functional group D₂.

When p in formula (11) is 1 or more, a compound (11) is produced: by reacting the functional group Q₂ of the compound (5) with the functional group F₁ of the compound (7) in accordance with a conventional method, and then reacting the functional group Gₚ of the obtained compound with the functional group Q₄ of the compound (6) in accordance with a conventional method; or by reacting the functional group Gₚ of the compound (7) with the functional group Q₄ of the compound (6) in accordance with a conventional method, and then reacting the functional group F₁ of the obtained compound with the functional group Q₂ of the compound (5) in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. Specific examples of L formed by the reaction of two functional groups are the same as the specific examples of L formed by the reaction of the functional group E₁ and the functional group D₂.

When a branched chain is introduced into the functional group Q₂ of the compound (5), after production of the compound (11), a straight chain or a branched chain is introduced into the functional groups Q₅ and Q₆ of the compound (11).

The case in which a straight chain or a branched chain is introduced into the functional group Q₅ of the compound (11) will be described, and a straight chain or a branched chain can also be similarly introduced into the functional group Q₆ of the compound (11).

When a straight chain is introduced into the functional group Q₅ of the compound (11), a compound (12) represented by the following formula (12) is produced.

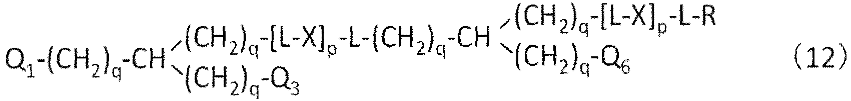

When p in formula (12) (p in -(CH₂)_{q}-[L-X]ₚ-L-R) is 0, a compound (12) is produced by reacting the functional group Q₅ of the compound (11) with the functional group A₃ of the compound (9) in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. Specific examples of L formed by the reaction of two functional groups are the same as the specific examples of L formed by the reaction of the functional group E₁ and the functional group D₂.

When p in formula (12) (p in -(CH₂)_{q}-[L-X]ₚ-L-R) is 1 or more, a compound (12) is produced: by reacting the functional group Q₅ of the compound (11) with the functional group H₁ of the compound (8) in accordance with a conventional method, and then reacting the functional group Iₚ of the obtained compound with the functional group A₃ of the compound (9) in accordance with a conventional method; or by reacting the functional group Iₚ of the compound (8) with the functional group A₃ of the compound (9) in accordance with a conventional method, and then reacting the functional group H₁ of the obtained compound with the functional group Q₅ of the compound (11) in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. Specific examples of L formed by the reaction of two functional groups are the same as the specific examples of L formed by the reaction of the functional group E₁ and the functional group D₂.

When a branched chain is introduced into the functional group Q₅ of the compound (11), a compound (13) represented by the following formula (13) is produced.

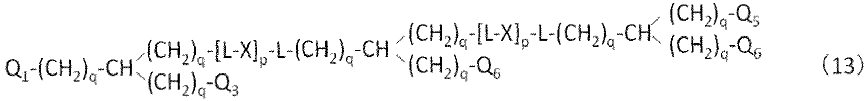

When p in formula (13) (p in -(CH₂)_{q}-[L-X]ₚ-L-(CH₂)_{q}-CH(-(CH₂)_{q}-Q₅)(-(CH₂)_{q}-Q₆)) is 0, a compound (13) is produced by reacting the functional group Q₅ of the compound (11) with the functional group Q₄ of the compound (6) in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. Specific examples of L formed by the reaction of two functional groups are the same as the specific examples of L formed by the reaction of the functional group E₁ and the functional group D₂.

When p in formula (13) (p in -(CH₂)_{q}-[L-X]ₚ-L-(CH₂)_{q}-CH(-(CH₂)_{q}-Q₅)(-(CH₂)_{q}-Q₆)) is 1 or more, a compound (13) is produced: by reacting the functional group Q₅ of the compound (11) with the functional group F₁ of the compound (7) in accordance with a conventional method, and then reacting the functional group Gₚ of the obtained compound with the functional group Q₄ of the compound (6) in accordance with a conventional method; or by reacting the functional group Gₚ of the compound (7) with the functional group Q₄ of the compound (6) in accordance with a conventional method, and then reacting the functional group F₁ of the obtained compound with the functional group Q₅ of the compound (11) in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. Specific examples of L formed by the reaction of two functional groups are the same as the specific examples of L formed by the reaction of the functional group E₁ and the functional group D₂.

A straight chain or a branched chain is introduced into newly introduced functional groups Q₅ and Q₆ in the same manner as mentioned above. By repeating this operation desired times, a branched chain having a desired number of branches is introduced into the functional group Q₂ of the compound (5). A straight chain is introduced into lastly introduced functional groups Q₅ and Q₆ in the same manner as mentioned above. As a result, a compound (5-Y) is produced.

### (Step 10C)

When p in formula (III) is 0, a carboxylic acid-type lipid (III) in which M is M₀-NH- is produced: by reacting the functional group Q₁ of the compound (5-Y) with the functional group A₁ of the compound (2) in accordance with a conventional method to produce a carboxylic acid-type lipid (III) in which M is HO-, and then reacting the carboxyl group of the carboxylic acid-type lipid (III) in which M is HO- with the amino group of the compound (1) in accordance with a conventional method; or by reacting the amino group of the compound (1) with the carboxyl group of the compound (2) in accordance with a conventional method, and then reacting the functional group A₁ of the obtained compound with the functional group Q₁ of the compound (5-Y) in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. Specific examples of L formed by the reaction of two functional groups are the same as the specific examples of L formed by the reaction of the functional group E₁ and the functional group D₂.

When p in formula (III) is 1 or more, a carboxylic acid-type lipid (III) in which M is M₀-NH- is produced: by reacting the functional group Q₁ of the compound (5-Y) with the functional group Eₚ of the compound (3) in accordance with a conventional method, then reacting the functional group D₁ of the obtained compound with the functional group A₁ of the compound (2) in accordance with a conventional method to produce a carboxylic acid-type lipid (III) in which M is HO-, and then reacting the carboxyl group of the carboxylic acid-type lipid (III) in which M is HO- with the amino group of the compound (1) in accordance with a conventional method; or by reacting the amino group of the compound (1) with the carboxyl group of the compound (2) in accordance with a conventional method, then reacting the functional group A₁ of the obtained compound with the functional group D₁ of the compound (3) in accordance with a conventional method, and then reacting the functional group Eₚ of the obtained compound with the functional group Q₁ of the compound (5-Y) in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. Specific examples of L formed by the reaction of two functional groups are the same as the specific examples of L formed by the reaction of the functional group E₁ and the functional group D₂.

As mentioned above, the carboxylic acid-type lipid (III) can be produced by a method including step 1C to step 10C. In each step, the order of reaction can be appropriately changed as long as a desired compound can be produced.

### <Method for Producing Carboxylic Acid-Type Lipid (IV)>

One embodiment of a method for producing the carboxylic acid-type lipid (IV) will be described.

### (Step 1D)

When M is M₀-NH-, compound (1) is provided.

### (Step 2D)

A compound (5) in which Q₁ is a carboxyl group is provided.

### (Step 3D)

A carboxylic acid-type lipid (IV) in which M is HO- is produced by introducing a straight chain or a branched chain into the functional groups Q₂ or Q₃ of the compound (5) in the same manner as in step 9C. Then, a carboxylic acid-type lipid (IV) is produced by reacting the functional group Q₁ (carboxyl group) of the carboxylic acid-type lipid (IV) in which M is HO- with the amino group of the compound (1) in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method.

A compound (5-Y) having a functional group Q₁ (carboxyl group) (carboxylic acid-type lipid (IV) in which M is HO-) is produced by introducing a straight chain or a branched chain into the functional groups Q₂ and Q₃ of the compound (5) in the same manner as in step 9C. A carboxylic acid-type lipid (IV) in which M is M₀-NH- is produced by reacting the functional group Q₁ (carboxyl group) of the produced compound (5-Y) with the amino group of the compound (1) in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method.

As mentioned above, the carboxylic acid-type lipid (IV) can be produced by a method including step 1D to step 3D. In each step, the order of reaction can be appropriately changed as long as a desired compound can be produced.

### <Method for Producing Carboxylic Acid-Type Lipid (V)>

One embodiment of a method for producing the carboxylic acid-type lipid (V) will be described. When two or more same symbols (e.g., L, X, p, q, etc.) exist in a structure formula of one certain compound, the meanings of these same symbols may be the same or different as long as they are within the range of the definition of the symbols. When the same symbols (e.g., L, X, p, q, etc.) exist in structure formulas of two or more compounds, the meanings of these same symbols may be the same or different as long as they are within the range of the definition of the symbols.

### (Step 1E)

When M is M₀-NH-, a compound (1) is provided.

### (Step 2E)

A compound (2) is provided.

### (Step 3E)

A compound (3) is provided, as necessary.

### (Step 4E)

A compound (14) represented by the following formula (14) is provided.

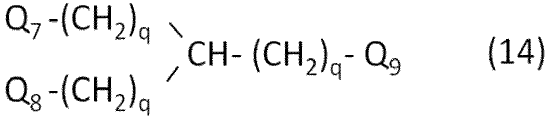

In formula (14), q is the same as defined above. A plurality of q's existing in formula (14) may represent the same integers or may represent different integers.

In formula (14), Q₇ and Q₈ are each independently functional groups that can be reacted with the amino group of the compound (1), the functional group A₁ of the compound (2), the functional group Eₚ of the compound (3), a functional group Q₁₂ of a compound (15) mentioned later or a functional group Kₚ of a compound (16) mentioned later, and are selected from a carboxyl group, an amino group, a hydroxyl group and a thiol group. Specific examples of a combination of functional groups that can be reacted are the same as the specific examples of the combination of the functional group A₁ and the functional group D₁. Q₇ and Q₈ may be the same or different.

In formula (14), Q₉ represents a carboxyl group, an amino group, a hydroxyl group or a thiol group.

In formula (14), Q₉ may be the same as or different from one or both of Q₇ and Q₈. When Q₉ is different from one or both of Q₇ and Q₈, it becomes easy to select a protecting group for protection of Q₉. From this point of view, it is preferable that Q₉ is different from one or both of Q₇ and Q₈.

The compound (14) is not particularly limited as long as it is a trifunctional compound. The compound (14) is preferably trifunctional amino acid. The description on the trifunctional amino acid is the same as mentioned above.

### (Step 5E)

A compound (15) represented by the following formula (15) is provided, as necessary.

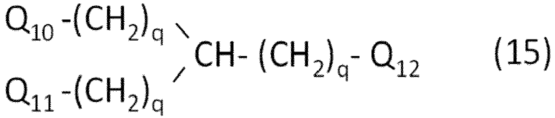

In formula (15), q is the same as defined above. A plurality of q's existing in formula (15) may represent the same integers or may represent different integers.

In formula (15), Q₁₀ and Q₁₁ are each independently functional groups that can be reacted with the amino group of the compound (1), the functional group A₁ of the compound (2), the functional group Eₚ of the compound (3), a functional group Q₁₂ of another compound (15) or a functional group Kₚ of a compound (16) mentioned later, and are selected from a carboxyl group, an amino group, a hydroxyl group and a thiol group. Specific examples of a combination of functional groups that can be reacted are the same as the specific examples of the combination of the functional group A₁ and the functional group D₁. Q₁₀ and Q₁₁ may be the same or different.

In formula (15), Q₁₂ represents a carboxyl group, an amino group, a hydroxyl group or a thiol group.

In formula (15), Q₁₂ may be the same as or different from one or both of Q₁₀ and Q₁₁. When Q₁₂ is different from one or both of Q₁₀ and Q₁₁, it becomes easy to select a protecting group for protection of Q₁₂. From this point of view, it is preferable that Q₁₂ is different from one or both of Q₁₀ and Q₁₁.

The compound (15) is not particularly limited as long as it is a trifunctional compound. The compound (15) is preferably trifunctional amino acid. The description on the trifunctional amino acid is the same as mentioned above.

### (Step 6E)

A compound (16) represented by the following formula (16):

J₁-X-[L-X]ₚ₋₁-Kₚ (16)

wherein L and X are the same as defined above, and p represents an integer of 1 or more, and J₁ and Kₚ each independently represent a carboxyl group, an amino group, a hydroxyl group or a thiol group,
is provided, as necessary.

A functional group J₁ is a functional group that can be reacted with the functional group Q₁₂ of the compound (15), and is selected from a carboxyl group, an amino group, a hydroxyl group and a thiol group. Specific examples of a combination of functional groups that can be reacted are the same as the specific examples of the combination of the functional group A₁ and the functional group D₁.

The compound (16) can be produced in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. The compound (16) may be a commercially available product.

One embodiment of a method for producing the compound (16) is the same as one embodiment of a method for producing the compound (3).

### (Step 7E)

A compound (17) represented by the following formula (17):

T₁-X-[L-X]ₚ₋₁-Uₚ (17)

wherein L and X are the same as defined above, and p represents an integer of 1 or more, and T₁ and Uₚ each independently represent a carboxyl group, an amino group, a hydroxyl group or a thiol group,
is provided, as necessary.

A functional group T₁ is a functional group that can be reacted with the functional group Q₉ of the compound (14), and is selected from a carboxyl group, an amino group, a hydroxyl group and a thiol group. Specific examples of a combination of functional groups that can be reacted are the same as the specific examples of the combination of the functional group A₁ and the functional group D₁.

The compound (17) can be produced in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. The compound (17) may be a commercially available product.

One embodiment of a method for producing the compound (17) is the same as one embodiment of a method for producing the compound (3).

### (Step 8E)

A compound (18) represented by the following formula (18):

A₄-R (18)

wherein R is the same as defined above, and A₄ represents a carboxyl group, an amino group, a hydroxyl group or a thiol group, is provided.

A functional group A₄ is a functional group that can be reacted with the functional group Q₉ of the compound (14) or the functional group Uₚ of the compound (17), and is selected from a carboxyl group, an amino group, a hydroxyl group and a thiol group. Specific examples of a combination of functional groups that can be reacted are the same as the specific examples of the combination of the functional group A₁ and the functional group D₁.

The compound (18) can be produced in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. The compound (18) may be a commercially available product. Specific examples of the compound (18) are the same as the specific examples of the compound (4).

### (Step 9E)

A compound (14-Z) represented by the following formula (14-Z) is produced by introducing a straight chain or a branched chain into the functional groups Q₇ and Q₈ of the compound (14). In formula (14-Z), Z is the same as defined above.

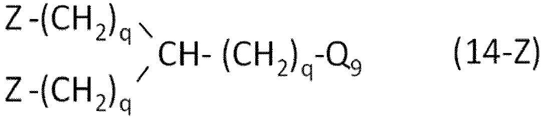

The case in which a straight chain or a branched chain is introduced into the functional group Q₇ of the compound (14) will be described, and a straight chain or a branched chain can also be similarly introduced into the functional group Q₈ of the compound (14).

When a straight chain represented by formula (X) is introduced into the functional group Q₇ of the compound (14), a compound (19) represented by the following formula (19) is produced.

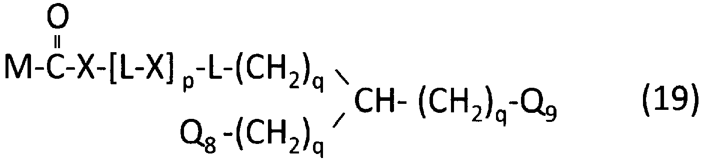

When p in formula (19) is 0, a compound (19) in which M is M₀-NH- is produced: by reacting the functional group Q₇ of the compound (14) with the functional group A₁ of the compound (2) in accordance with a conventional method to produce a compound (19) in which M is HO-, and then reacting the carboxyl group of the compound (19) in which M is HO- with the amino group of the compound (1) in accordance with a conventional method; or by reacting the amino group of the compound (1) with the carboxyl group of the compound (2) in accordance with a conventional method, and then reacting the functional group A₁ of the obtained compound with the functional group Q₇ of the compound (14) in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. Specific examples of L formed by the reaction of two functional groups are the same as the specific examples of L formed by the reaction of the functional group E₁ and the functional group D₂.

When p in formula (19) is 1 or more, a compound (19) in which M is M₀-NH- is produced: by reacting the functional group Q₇ of the compound (14) with the functional group Eₚ of the compound (3) in accordance with a conventional method, then reacting the functional group D₁ of the obtained compound with the functional group A₁ of the compound (2) in accordance with a conventional method to produce a compound (19) in which M is HO-, and then reacting the carboxyl group of the compound (19) in which M is HO-with the amino group of the compound (1) in accordance with a conventional method; or by reacting the amino group of the compound (1) with the carboxyl group of the compound (2) in accordance with a conventional method, then reacting the functional group A₁ of the obtained compound with the functional group D₁ of the compound (3) in accordance with a conventional method, and then reacting the functional group Eₚ of the obtained compound with the functional group Q₇ of the compound (14) in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. Specific examples of L formed by the reaction of two functional groups are the same as the specific examples of L formed by the reaction of the functional group E₁ and the functional group D₂.

When a straight chain represented by formula (XI) is introduced into the functional group Q₇ of the compound (14), a compound (20) represented by the following formula (20) is produced.

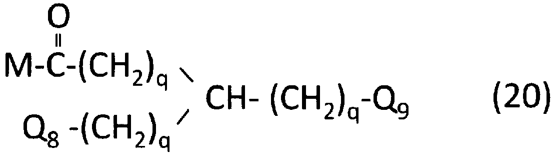

When a straight chain represented by formula (XI) is introduced into the functional group Q₇ of the compound (14), the functional group Q₇ of the compound (14) is a carboxyl group. When a straight chain represented by formula (XI) is introduced into the functional group Q₈ of the compound (14), the functional group Q₈ of the compound (14) is a carboxyl group. A compound (20) is produced by reacting the functional group Q₇ (carboxyl group) of the compound (14) with the amino group of the compound (1) in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method.

When a branched chain is introduced into the functional group Q₇ of the compound (14), a compound (21) represented by the following formula (21) is produced.

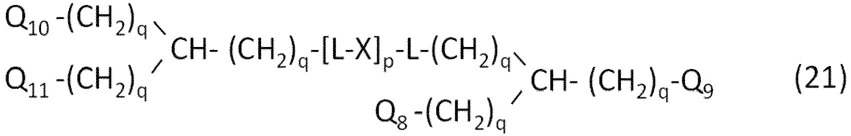

When p in formula (21) is 0, a compound (21) is produced by reacting the functional group Q₇ of the compound (14) with the functional group Q₁₂ of the compound (15). In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. Specific examples of L formed by the reaction of two functional groups are the same as the specific examples of L formed by the reaction of the functional group E₁ and the functional group D₂.

When p in formula (21) is 1 or more, a compound (21) is produced: by reacting the functional group Q₇ of the compound (14) with the functional group Kₚ of the compound (16) in accordance with a conventional method, and then reacting the functional group J₁ of the obtained compound with the functional group Q₁₂ of the compound (15) in accordance with a conventional method; or by reacting the functional group J₁ of the compound (16) with the functional group Q₁₂ of the compound (15) in accordance with a conventional method, and then reacting the functional group Kₚ of the obtained compound with the functional group Q₇ of the compound (14). In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. Specific examples of L formed by the reaction of two functional groups are the same as the specific examples of L formed by the reaction of the functional group E₁ and the functional group D₂.

When a branched chain is introduced into the functional group Q₇ of the compound (14), after production of the compound (21), a straight chain or a branched chain is introduced into the functional groups Q₁₀ and Q₁₁ of the compound (21).

The case in which a straight chain or a branched chain is introduced into the functional group Q₁₀ of the compound (21) will be described, and a straight chain or a branched chain can also be similarly introduced into the functional group Q₁₁ of the compound (21).

When a straight chain represented by formula (X) is introduced into the functional group Q₁₀ of the compound (21), a compound (22) represented by the following formula (22) is produced.

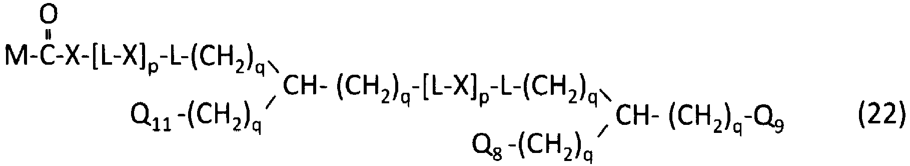

When p in formula (22) (p in M- CO-X-[L-X]ₚ-L-) is 0, a compound (22) in which M is M₀-NH- is produced: by reacting the functional group Q₁₀ of the compound (21) with the functional group A₁ of the compound (2) in accordance with a conventional method to produce a compound (22) in which M is HO-, and then reacting the carboxyl group of the compound (22) in which M is HO- with the amino group of the compound (1) in accordance with a conventional method; or by reacting the amino group of the compound (1) with the carboxyl group of the compound (2) in accordance with a conventional method, and then reacting the functional group A₁ of the obtained compound with the functional group Q₁₀ of the compound (21) in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. Specific examples of L formed by the reaction of two functional groups are the same as the specific examples of L formed by the reaction of the functional group E₁ and the functional group D₂.

When p in formula (22) (p in M -CO-X-[L-X]ₚ-L-) is 1 or more, a compound (22) in which M is M₀-NH- is produced: by reacting the functional group Q₁₀ of the compound (21) with the functional group Eₚ of the compound (3) in accordance with a conventional method, then reacting the functional group D₁ of the obtained compound with the functional group A₁ of the compound (2) in accordance with a conventional method to produce a compound (22) in which M is HO-, and then reacting the carboxyl group of the compound (22) in which M is HO- with the amino group of the compound (1) in accordance with a conventional method; or by reacting the amino group of the compound (1) with the carboxyl group of the compound (2) in accordance with a conventional method, then reacting the functional group A₁ of the obtained compound with the functional group D₁ of the compound (3) in accordance with a conventional method, and then reacting the functional group Eₚ of the obtained compound with the functional group Q₁₀ of the compound (21) in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. Specific examples of L formed by the reaction of two functional groups are the same as the specific examples of L formed by the reaction of the functional group E₁ and the functional group D₂.

When a straight chain represented by formula (XI) is introduced into the functional group Q₁₀ of the compound (21), a compound (23) represented by the following formula (23) is produced.

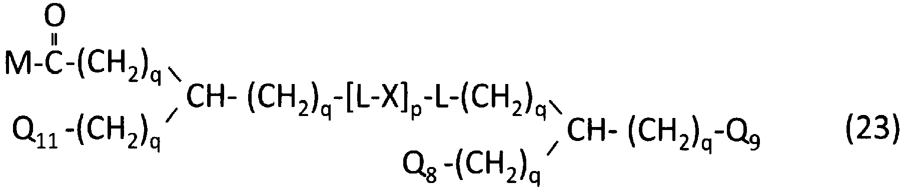

When a straight chain represented by formula (XI) is introduced into the functional group Q₁₀ of the compound (21), the functional group Q₁₀ of the compound (21) is a carboxyl group. When a straight chain represented by formula (XI) is introduced into the functional group Q₁₁ of the compound (21), the functional group Q₁₁ of the compound (21) is a carboxyl group. A compound (23) is produced by reacting the functional group Q₁₀ (carboxyl group) of the compound (21) with the amino group of the compound (1) in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method.

When a branched chain is introduced into the functional group Q₁₀ of the compound (21), a compound (24) represented by the following formula (24) is produced.

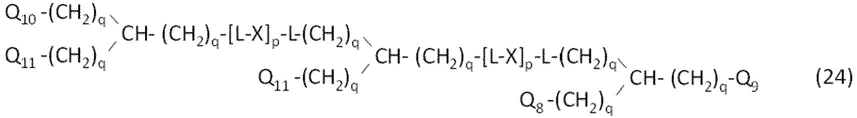

When p in formula (24) (p in (Q₁₀-(CH₂)_{q}-)(Q₁₁-(CH₂)_{q}-)CH-(CH₂)_{q}-[L-X]ₚ-L-) is 0, a compound (24) is produced by reacting the functional group Q₁₀ of the compound (21) with the functional group Q₁₂ of the compound (15). In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. Specific examples of L formed by the reaction of two functional groups are the same as the specific examples of L formed by the reaction of the functional group E₁ and the functional group D₂.

When p in formula (24) (p in (Q₁₀-(CH₂)_{q}-)(Q₁₁-(CH₂)_{q}-)CH-(CH₂)_{q}-[L-X]ₚ-L-) is 1 or more, a compound (24) is produced: by reacting the functional group Q₁₀ of the compound (21) with the functional group Kₚ of the compound (16) in accordance with a conventional method, and then reacting the functional group J₁ of the obtained compound with the functional group Q₁₂ of the compound (15) in accordance with a conventional method; or by reacting the functional group J₁ of the compound (16) with the functional group Q₁₂ of the compound (15) in accordance with a conventional method, and then reacting the functional group Kₚ of the obtained compound with the functional group Q₁₀ of the compound (21). In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. Specific examples of L formed by the reaction of two functional groups are the same as the specific examples of L formed by the reaction of the functional group E₁ and the functional group D₂.

A straight chain or a branched chain is introduced into newly introduced functional groups Q₁₀ and Q₁₁ in the same manner as mentioned above. By repeating this operation desired times, a branched chain having a desired number of branches can be introduced into the functional group Q₇ of the compound (14). A straight chain is introduced into lastly introduced functional groups Q₁₀ and Q₁₁ in the same manner as mentioned above. As a result, a compound (14-Z) is produced.

### (Step 10E)

When p in formula (V) is 0, a carboxylic acid-type lipid (V) is produced by reacting the functional group Q₉ of the compound (14-Z) with the functional group A₄ of the compound (18) in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. Specific examples of L formed by the reaction of two functional groups are the same as the specific examples of L formed by the reaction of the functional group E₁ and the functional group D₂.

When p in formula (V) is 1 or more, a carboxylic acid-type lipid (V) is produced: by reacting the functional group Q₉ of the compound (14-Z) with the functional group T₁ of the compound (17) in accordance with a conventional method, and then reacting the functional group Uₚ of the obtained compound with the functional group A₄ of the compound (18) in accordance with a conventional method; or by reacting the functional group Uₚ of the compound (17) with the functional group A₄ of the compound (18) in accordance with a conventional method, and then reacting the functional group T₁ of the obtained compound with the functional group Q₉ of the compound (14-Z) in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. Specific examples of L formed by the reaction of two functional groups are the same as the specific examples of L formed by the reaction of the functional group E₁ and the functional group D₂.

As mentioned above, the carboxylic acid-type lipid (V) can be produced by a method including step 1E to step 10E. In each step, the order of reaction can be appropriately changed as long as a desired compound can be produced.

### <Method for Producing Carboxylic Acid-Type Lipid (VI)>

One embodiment of a method for producing the carboxylic acid-type lipid (VI) will be described. When two or more same symbols (e.g., L, X, p, q, etc.) exist in a structure formula of one certain compound, the meanings of these same symbols may be the same or different as long as they are within the range of the definition of the symbols. When the same symbols (e.g., L, X, p, q, etc.) exist in structure formulas of two or more compounds, the meanings of these same symbols may be the same or different as long as they are within the range of the definition of the symbols.

### (Step 1F)

A compound (5-Y) is produced by introducing a straight chain or a branched chain into the functional groups Q₂ and Q₃ of the compound (5) in the same manner as in step 9C.

In the compound (5-Y) produced in step 1F, Q₁ is a functional group that can be reacted with Q₉ of a compound (14-Z) produced in step 2F or a functional group Wₚ of a compound (25) prepared in step 3F, and is selected from a carboxyl group, an amino group, a hydroxyl group and a thiol group. Specific examples of a combination of functional groups that can be reacted are the same as the specific examples of the combination of the functional group A₁ and the functional group D₁.

### (Step 2F)

A compound (14-Z) is produced by introducing a straight chain or a branched chain into the functional group Q₇ and Q₈ of the compound (14) in the same manner as in step 9E.

### (Step 3F)

A compound (25) represented by the following formula (25):

V₁-X-[L-X]ₚ₋₁-Wₚ (25)

wherein L and X are the same as defined above, and p represents an integer of 1 or more, and V₁ and Wₚ each independently represent a carboxyl group, an amino group, a hydroxyl group or a thiol group,
is provided, as necessary.

A functional group V₁ is a functional group that can be reacted with the functional group Q₉ of the compound (14-Z), and is selected from a carboxyl group, an amino group, a hydroxyl group and a thiol group. Specific examples of a combination of functional groups that can be reacted are the same as the specific examples of the combination of the functional group A₁ and the functional group D₁.

A functional group Wₚ is a functional group that can be reacted with the functional group Q₁ of the compound (5-Y), and is selected from a carboxyl group, an amino group, a hydroxyl group and a thiol group. Specific examples of a combination of functional groups that can be reacted are the same as the specific examples of the combination of the functional group A₁ and the functional group D₁.

The compound (25) can be produced in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. The compound (25) may be a commercially available product.

One embodiment of a method for producing the compound (25) is the same as one embodiment of a method for producing the compound (3).

### (Step 4F)

When p in formula (VI) is 0, a carboxylic acid-type lipid (VI) is produced by reacting the functional group Q₉ of the compound (14-Z) with the functional group Q₁ of the compound (5-Y) in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. Specific examples of L formed by the reaction of two functional groups are the same as the specific examples of L formed by the reaction of the functional group E₁ and the functional group D₂.

When p in formula (VI) is 1 or more, a carboxylic acid-type lipid (VI) is produced: by reacting the functional group Q₉ of the compound (14-Z) with the functional group V₁ of the compound (25) in accordance with a conventional method, and then reacting the functional group Wₚ of the obtained compound with the functional group Q₁ of the compound (5-Y) in accordance with a conventional method; or by reacting the functional group Wₚ of the compound (25) with the functional group Q₁ of the compound (5-Y) in accordance with a conventional method, and then reacting the functional group V₁ of the obtained compound with the functional group Q₉ of the compound (14-Z) in accordance with a conventional method. In this case, a functional group not involved in the reaction may be protected by a protecting group, as necessary. The protected functional group not involved in the reaction can be deprotected after reacting functional groups involved in the reaction with each other. Protection by a protecting group and deprotection can be performed in accordance with a conventional method. Specific examples of L formed by the reaction of two functional groups are the same as the specific examples of L formed by the reaction of the functional group E₁ and the functional group D₂.

As mentioned above, the carboxylic acid-type lipid (VI) can be produced by a method including step 1F to step 4F. In each step, the order of reaction can be appropriately changed as long as a desired compound can be produced.

### <Other Lipids>

The lipid particle, the lipid particle aggregate or the lipid membrane may include one or two or more lipids other than the carboxylic acid-type lipid according to the invention. Examples of the lipid other than the carboxylic acid-type lipid include a phospholipid, a glycolipid, a sterol and the like. The phospholipid, the glycolipid and the sterol will be described.

### < Phospholipid>

Examples of the phospholipid include a glycerophospholipid, a sphingophospholipid, a cardiolipin and the like. The phospholipid may be a phospholipid that is negatively charged at physiological pH, or may be a phospholipid that is amphoteric (i.e., has a moiety that is negatively charged and a moiety that is positively charged) at physiological pH. The phospholipid also includes a salt formed by a phosphoric acid group possessed by the phospholipid, and examples of the salt of a phosphoric acid group include a calcium salt, a magnesium salt, a potassium salt and the like. Regarding the phospholipid, one phospholipid may be used alone, or two or more phospholipids may be used in combination. The glycerophospholipid, the sphingophospholipid and the cardiolipin will be described.

### (Glycerophospholipid)

Examples of the glycerophospholipid include a lipid having a structure represented by the following formula (i). The glycerophospholipid may be a glycerophospholipid that is negatively charged at physiological pH, or may be a glycerophospholipid that is amphoteric at physiological pH. Examples of the glycerophospholipid that is negatively charged at physiological pH include a glycerophospholipid in which a group represented by X₁ in the following formula (i) is a group other than a cationic group (anionic group or electrically neutral group). Examples of the glycerophospholipid that is amphoteric at physiological pH include a glycerophospholipid in which a group represented by X₁ in the following formula (i) is a cationic group.

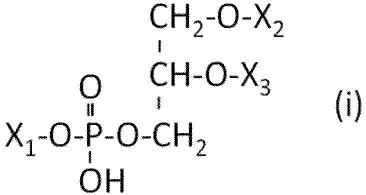

In formula (i), X₁ represents hydrogen, a choline residue, a serine residue, an inositol residue, a glycerol residue or an ethanolamine residue. A group represented by X₁ may be a cationic group, or may be a group other than a cationic group (anionic group or electrically neutral group). The choline residue is a cationic group, and the serine residue, the inositol residue and the glycerol residue are groups other than a cationic group.

In formula (i), X₂ and X₃ each independently represent hydrogen, a saturated or unsaturated acyl group (-CO-R, R is a hydrocarbon group) or a hydrocarbon group. Specific examples of the hydrocarbon group included in the acyl group represented by X₂ or X₃, and specific examples of the hydrocarbon group represented by X₂ or X₃ are the same as mentioned above. It is preferable that at least one of X₂ or X₃ is a saturated or unsaturated acyl group, and it is further preferable that both X₂ or X₃ are saturated or unsaturated acyl groups. When both X₂ or X₃ are acyl groups, two acyl groups may be the same or different.

Examples of the glycerophospholipid include phosphatidic acid, phosphatidylcholine, phosphatidylserine, phosphatidylinositol, phosphatidylglycerol, phosphatidylethanolamine and the like. Of these, phosphatidylserine and phosphatidylglycerol are preferable.

Examples of the phosphatidic acid include dipalmitoylphosphatidic acid, distearoylphosphatidic acid, dimyristoylphosphatidic acid, dioleylphosphatidic acid and the like.

Examples of the phosphatidylcholine include dipalmitoylphosphatidylcholine, distearoylphosphatidylcholine, dimyristoylphosphatidylcholine, dioleoylphosphatidylcholine, dilauroylphosphatidylcholine, didecanoylphosphatidylcholine, dioctanoylphosphatidylcholine, dihexanoylphosphatidylcholine, dibutyrylphosphatidylcholine, dielaidoylphosphatidylcholine, dilinoleoylphosphatidylcholine, diarachidonoylphosphatidylcholine, diicosenoylphosphatidylcholine, diheptanoylphosphatidylcholine, dicaproylphosphatidylcholine, diheptadecanoylphosphatidylcholine, dibehenoylphosphatidylcholine, eleostearoylphosphatidylcholine, hydrogenated egg phosphatidylcholine, hydrogenated soy phosphatidylcholine, 1-palmitoyl-2-arachidonoylphosphatidylcholine, 1-palmitoyl-2-oleoylphosphatidylcholine, 1-palmitoyl-2-linoleoylphosphatidylcholine, 1-palmitoyl-2-myristoylphosphatidylcholine, 1-palmitoyl-2-stearoylphosphatidylcholine, 1-stearoyl-2-palmitoylphosphatidylcholine, 1,2-dimyristoylamide-1,2-deoxyphosphatidylcholine, 1-myristoyl-2-palmitoylphosphatidylcholine, 1-myristoyl-2-stearoylphosphatidylcholine, di-O-hexadecylphosphatidylcholine, transdielaidoylphosphatidylcholine, dipalmitelaidoyl-phosphatidylcholine, n-octadecyl-2-methylphosphatidylcholine, n-octadecylphosphatidylcholine, 1-laurylpropanediol-3-phosphocholine, erythro-N-lignoceroylsphingophosphatidylcholine, palmitoyl-(9-cis-octadecenoyl)-3-sn-phosphatidylcholine and the like.

Examples of the phosphatidylserine include distearoylphosphatidylserine, dimyristoylphosphatidylserine, dilauroylphosphatidylserine, dipalmitoylphosphatidylserine, dioleoylphosphatidylserine, eleostearoylphosphatidylserine, 1,2-di-(9-cis-octadecenoyl)-3-sn-phosphatidylserine and the like.

Examples of the phosphatidylinositol include dipalmitoylphosphatidylinositol, distearoylphosphatidylinositol, dilauroylphosphatidylinositol and the like.

Examples of the phosphatidylglycerol include dipalmitoylphosphatidylglycerol, distearoylphosphatidylglycerol, dioleoylphosphatidylglycerol, dilauroylphosphatidylglycerol, dimyristoylphosphatidylglycerol, hydrogenated soy phosphatidylglycerol, hydrogenated egg phosphatidylglycerol and the like.

Examples of the phosphatidylethanolamine include dipalmitoylphosphatidylethanolamine, distearoylphosphatidylethanolamine, dioleoylphosphatidylethanolamine, dilauroylphosphatidylethanolamine, dimyristoylphosphatidylethanolamine, didecanoylphosphatidylethanolamine, N-glutarylphosphatidylethanolamine, N-(7-nitro-2,1,3-benzoxydiazol-4-yl)-1,2-dioleoyl-sn-phosphatidyle thanolamine, eleostearoylphosphatidylethanolamine, N-succinyldioleoylphosphatidylethanolamine, 1-hexadecyl-2-palmitoylglycerophosphatidylethanolamine and the like.

In phosphatidic acid, phosphatidylcholine, phosphatidylserine, phosphatidylinositol, phosphatidylglycerol and phosphatidylethanolamine, the number of carbon atoms of the hydrocarbon group included in the acyl group represented by X₂ or X₃, and the number of carbon atoms of the hydrocarbon group represented by X₂ or X₃ is preferably 10 to 24, more preferably 12 to 22, and still more preferably 14 to 18.

The glycerophospholipid is preferably dipalmitoylphosphatidylcholine, dipalmitoylphosphatidylserine, dipalmitoylphosphatidylglycerol, dipalmitoylphosphatidylethanolamine and the like.

### (Sphingophospholipid)

Examples of the sphingophospholipid include a lipid having a structure represented by the following formula (ii). The sphingophospholipid may be a sphingophospholipid that is negatively charged at physiological pH, or may be a sphingophospholipid that is amphoteric at physiological pH. Examples of the sphingophospholipid that is negatively charged at physiological pH include a sphingophospholipid in which a group represented by X₄ in the following formula (ii) is a group other than a cationic group (anionic group or electrically neutral group). Examples of the sphingophospholipid that is amphoteric at physiological pH include a sphingophospholipid in which a group represented by X₄ in the following formula (ii) is a cationic group.

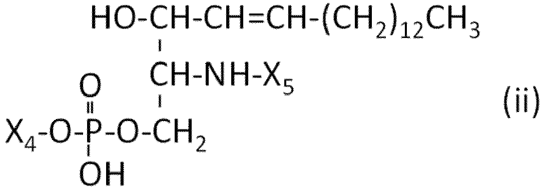

In formula (ii), X₄ represents hydrogen, a choline residue, a serine residue, an inositol residue, a glycerol residue or an ethanolamine residue. A group represented by X₄ may be a cationic group, or may be a group other than a cationic group (anionic group or electrically neutral group). The choline residue is a cationic group, and the serine residue, the inositol residue and the glycerol residue are groups other than a cationic group.

In formula (ii), X₅ represents hydrogen or a saturated or unsaturated acyl group. X₅ represents preferably a saturated or unsaturated acyl group. Specific example of the hydrocarbon group included in the acyl group are the same as mentioned above. The number of carbon atoms of the hydrocarbon group included in the acyl group is preferably 10 to 24, more preferably 12 to 22, and still more preferably 14 to 18.

Examples of the sphingophospholipid include sphingomyelin, dipalmitoyl sphingomyelin, distearoyl sphingomyelin, ceramide ciliatine, ceramide phosphorylethanolamine, ceramide phosphorylglycerol and the like.

### (Cardiolipin)

Examples of the cardiolipin include a lipid having a structure represented by the following formula (iii). The cardiolipin may be a cardiolipin that is negatively charged at physiological pH, or may be a cardiolipin that is amphoteric at physiological pH.

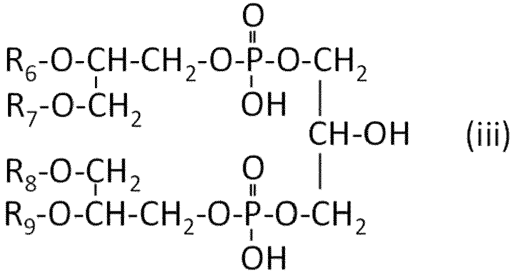

In formula (iii), R₆ to R₉ each independently represent hydrogen or a saturated or unsaturated acyl group, at least one of R₆ to R₉ is a saturated or unsaturated acyl group. It is preferable that two to four of R₆ to R₉ are acyl groups, it is more preferable that three to four of R₆ to R₉ are acyl groups, and it is still more preferable that all of R₆ to R₉ are acyl groups. When two or more of R₆ to R₉ are acyl groups, two or more acyl groups may be the same or different. Specific example of the hydrocarbon group included in the acyl group are the same as mentioned above. The number of carbon atoms of the hydrocarbon group included in the acyl group is preferably 10 to 24, more preferably 12 to 22, and still more preferably 14 to 18.

### <Glycolipid>

Examples of the glycolipid include a glyceroglycolipid, a sphingoglycolipid and the like. When two or more acyl groups are included in the glycolipid, two or more acyl groups may be the same or different. Specific example of the hydrocarbon group included in the acyl group are the same as mentioned above. The number of carbon atoms of the hydrocarbon group included in the acyl group is preferably 1 to 4, more preferably 1 to 2, and still more preferably 2. Regarding the glycolipid, one glycolipid may be used alone, or two or more glycolipids may be used in combination.

Examples of the glyceroglycolipid include diglycosyl diglyceride, glycosyl diglyceride, digalactosyl diglyceride, galactosyl diglyceride, sulfoxyribosyl diglyceride, (1,3)-D-mannosyl(1,3)diglyceride, digalactosyl glyceride, digalactosyl dilauroyl glyceride, digalactosyl dimyristoyl glyceride, digalactosyl dipalmitoyl glyceride, digalactosyl distearoyl glyceride, galactosyl glyceride, galactosyl dilauroyl glyceride, galactosyl dimyristoyl glyceride, galactosyl dipalmitoyl glyceride, galactosyl distearoyl glyceride, digalactosyldiacylglycerol and the like.

Examples of the sphingoglycolipid include ceramide (cerebroside), galactosylceramide, lactosylceramide, digalactosylceramide, ganglioside GM1, ganglioside GM2, ganglioside GM3, sulfatide, ceramide oligohexoside, globoside and the like.

### <Sterol>

Examples of the sterol include cholesterol, cholesterol succinate, dihydrocholesterol, lanosterol, dihydrolanosterol, desmosterol, stigmasterol, sitosterol, campesterol, brassicasterol, zymosterol, ergosterol, campesterol, fucosterol, 22-ketosterol, 20-hydroxysterol, 7-hydroxycholesterol, 19-hydroxycholesterol, 22-hydroxycholesterol, 25-hydroxycholesterol, 7-dehydrocholesterol, 5α-cholest-7-en-3β-ol, epicholesterol, dehydroergosterol, cholesterol sulfate, cholesterol hemisuccinate, cholesterol phthalate, cholesterol phosphate, cholesterol valerate, cholesterol hemisuccinate, 3βN-(N',N'-dimethylaminoethane)-carbamoyl cholesterol, cholesterol acetate, cholesteryl oleate, cholesteryl linoleate, cholesteryl myristate, cholesteryl palmitate, cholesteryl arachidate, coprostanol, cholesterol ester, cholesteryl phosphorylcholine, 3,6,9-trioxaoctan-1-ol-cholesteryl-3e-ol and the like. Regarding the sterol, one sterol may be used alone, or two or more sterols may be used in combination.

### < Fatty acid>

The lipid particle, the lipid particle aggregate or the lipid membrane may include fatty acid. The fatty acid may be saturated fatty acid or unsaturated fatty acid. The number of carbon atoms of fatty acid is not particularly limited, and is 10 to 24, more preferably 12 to 22, and still more preferably 14 to 18. Examples of the fatty acid include caprylic acid, pelargonic acid, capric acid, undecylenic acid, lauric acid, tridecylenic acid, myristic acid, pentadecylenic acid, palmitic acid, margaric acid, stearic acid, nonadecylenic acid, arachidic acid, dodecenoic acid, tetradecenoic acid, oleic acid, linoleic acid, linoleic acid, eicosenoic acid, erucic acid, docosapentaenoic acid and the like. Regarding the fatty acid, one fatty acid may be used alone, or two or more fatty acids may be used in combination.

Other lipids such as the phospholipid, the glycolipid and the sterol may be modified by a hydrophilic polymer, etc. Examples of the hydrophilic polymer include polyethylene glycol (PEG), polyglycerin, polypropylene glycol, polyvinyl alcohol, styrene-maleic anhydride alternating copolymer, polyvinylpyrrolidone, synthetic polyamino acid and the like. Regarding these hydrophilic polymers, one hydrophilic polymer may be used alone, or two or more hydrophilic polymers may be used in combination.

In the lipid particle, the lipid particle aggregate or the lipid membrane, the content of a phospholipid is preferably 0 to 95 mol%, more preferably 0 to 50 mol%, and still more preferably 0 to 30 mol%, based on the total lipid amount included in the lipid particle, the lipid particle aggregate or the lipid membrane. In the lipid particle, the lipid particle aggregate or the lipid membrane, the molar ratio of the content of a phospholipid to the content of the carboxylic acid-type lipid of the present invention (the content of a phospholipid : the content of the carboxylic acid-type lipid of the present invention) is preferably 0:1 to 19:1, more preferably 0:1 to 10:1, and still more preferably 0:1 to 1:1.

In the lipid particle, the lipid particle aggregate or the lipid membrane, the content of a sterol is preferably 0 to 50 mol%, more preferably 0 to 40 mol%, and still more preferably 0 to 30 mol%, based on the total lipid amount included in the lipid particle, the lipid particle aggregate or the lipid membrane. In the lipid particle, the lipid particle aggregate or the lipid membrane, the molar ratio of the content of a sterol to the content of the carboxylic acid-type lipid of the present invention (the content of a sterol : the content of the carboxylic acid-type lipid of the present invention) is preferably 0:1 to 9:1, more preferably 0:1 to 5:1, and still more preferably 0:1 to 1:1.

Specific combinations of a carboxylic acid-type lipid, a phospholipid and a sterol can be appropriately selected from the carboxylic acid-type lipids, the phospholipids and the sterols mentioned above.

When the lipid particle, the lipid particle aggregate or the lipid membrane comprises the carboxylic acid-type lipid of the present invention and a phospholipid, preferably, the carboxylic acid-type lipid of the present invention is a carboxylic acid-type lipid in which M in formulas (III) to (VI) is an aspartic acid residue, a glutamic acid residue, an AG residue or a salt thereof, and the phospholipid is at least one glycerophospholipid selected from the group consisting of dipalmitoylphosphatidylcholine, dipalmitoylphosphatidylserine, dipalmitoylphosphatidylglycerol and dipalmitoylphosphatidylethanolamine. More preferably, the carboxylic acid-type lipid of the present invention is a carboxylic acid-type lipid in which M in formulas (III) to (VI) is an aspartic acid residue, a glutamic acid residue, an AG residue or a salt thereof, and the phospholipid is at least one glycerophospholipid selected from the group consisting of dipalmitoylphosphatidylcholine, dipalmitoylphosphatidylserine and dipalmitoylphosphatidylglycerol. Still more preferably, the carboxylic acid-type lipid of the present invention is a carboxylic acid-type lipid in which M in formulas (III) to (VI) is an aspartic acid residue, a glutamic acid residue, an AG residue or a salt thereof, and the phospholipid is at least one glycerophospholipid selected from the group consisting of dipalmitoylphosphatidylserine and dipalmitoylphosphatidylglycerol.

When the lipid particle, the lipid particle aggregate or the lipid membrane comprises a carboxylic acid-type lipid and a sterol, whereby the carboxylic acid-type lipid of the present invention is a carboxylic acid-type lipid in which M in formulas (III) to (VI) is HO- or M₀-NH-, preferably, M₀ is an aspartic acid residue, a glutamic acid residue, an AG residue or a salt thereof, and the sterol is cholesterol.

When the lipid particle, the lipid particle aggregate or the lipid membrane comprises a carboxylic acid-type lipid, a phospholipid and a sterol, preferably, the carboxylic acid-type lipid of the present invention is a carboxylic acid-type lipid in which M in formulas (III) to (VI) is an aspartic acid residue, a glutamic acid residue, an AG residue or a salt thereof, the phospholipid is at least one glycerophospholipid selected from the group consisting of dipalmitoylphosphatidylcholine, dipalmitoylphosphatidylserine, dipalmitoylphosphatidylglycerol and dipalmitoylphosphatidylethanolamine, and the sterol is cholesterol. More preferably, the carboxylic acid-type lipid of the present invention is a carboxylic acid-type lipid in which M in formulas (III) to (VI) is an aspartic acid residue, a glutamic acid residue, an AG residue or a salt thereof, the phospholipid is at least one glycerophospholipid selected from the group consisting of dipalmitoylphosphatidylcholine, dipalmitoylphosphatidylserine and dipalmitoylphosphatidylglycerol, and the sterol is cholesterol. Still more preferably, the carboxylic acid-type lipid of the present invention is a carboxylic acid-type lipid in which M in formulas (III) to (VI) is an aspartic acid residue, a glutamic acid residue, an AG residue or a salt thereof, the phospholipid is at least one glycerophospholipid selected from the group consisting of dipalmitoylphosphatidylserine and dipalmitoylphosphatidylglycerol, and the sterol is cholesterol.

### Method for Producing Hemostatic Material

The hemostatic material of the present invention can be produced by, for example, a method including the following steps:
(a) a step of providing a water-insoluble base;
(b) a step of providing a lipid comprising an anionic lipid; and
(c) a step of supporting the lipid provided in the step (b) on the base provided in the step (a).

The description on the base to be provided in the step (a) is the same as mentioned above.

The description on the lipid to be provided in the step (b) is the same as mentioned above.

When the form of the lipid supported on the base is a lipid particle and/or an aggregate of a lipid particle, a dispersion liquid of a lipid particle comprising an anionic lipid is provided in the step (b).

When the form of the lipid supported on the base is a lipid membrane, a lipid solution comprising an anionic lipid or a lipid dispersion liquid comprising an anionic lipid is provided in the step (b).

When the form of the lipid supported on the base is a lipid particle and/or an aggregate of a lipid particle and/or a lipid membrane, in the step (c), the method for supporting the lipid particle and/or the lipid membrane on the base is not particularly limited, and examples thereof include physical adsorption, covalent bond, hydrogen bond, coordinate bond, electrostatic interaction, van der Waals force, hydrophobic interaction and the like. The method for supporting the lipid particle and/or the lipid membrane on the base is not particularly limited, and, for example, by immersing the base in a dispersion liquid of a lipid particle and then freeze-drying the base, it is possible to support the lipid particle. By spraying a dispersion liquid of a lipid particle or a lipid solution on the base with a spray, etc., and then freeze-drying the base, it is also possible to support a lipid particle and/or an aggregate of a lipid particle and/or a lipid membrane. Examples of the dispersion medium include physiological saline, a phosphate buffer, alcohols and the like. Examples of the alcohol include tert-butyl alcohol. Freeze-drying is performed by, for example, allowing to stand at 20 to 40 Pa for 12 hours.

The dispersion liquid of a lipid particle used in supporting the lipid particle on the base may include, in addition to the lipid particle, a pharmaceutically acceptable additive. Examples of the additive include an isotonizing agent, a stabilizer, an antioxidant, a pH adjuster, an excipient, a diluent, a humectant, an emulsifier, a binder, a disintegrant, a lubricant, an expander, a dispersant, a suspending agent, an osmotic pressure adjuster, an antiseptic, a coloring agent, an ultraviolet absorber, a moisturizer, a thickener, a brightener, a preservative, a corrigent, a fragrance, a film forming agent, a flavoring agent, a bacterial inhibitor and the like. Regarding these additives, one additive may be used alone, or two or more additives may be used in combination.

When a support member that supports the base is used, supporting a lipid particle on the base may be performed before or after combining the base with the support member, and is preferably performed after combining the base with the support member.

### EXAM PLES

The present invention will be described in more detail by way of Examples.

### [Synthesis of Lipid]

The following lipids were synthesized and used in Examples.

### (1) Synthesis of Carboxylic Acid-Type Lipid Represented by Formula (a2) (DHSG: 1,5-Dihexadecyl-N-succinyl-L-glutamate)

In accordance with the following procedures, DHSG was synthesized. DHSG can be used as a starting material when the carboxylic acid-type lipids of the present invention in which M is M₀-NH- (e.g., Glu-DHSG, Asp-DHSG, AG-DHSG and the like) are synthesized.

Glutamic acid (2.96 g, 20 mmol), p-toluenesulfonic acid (4.56 g, 24 mmol) and hexadecyl alcohol (10.65 g, 44 mmol) were dissolved in benzene (150 mL) and mixed, and the mixture was refluxed at 100°C for 14 hours while dehydrating. Then, the solvent was removed under reduced pressure, and the residue thus obtained was redissolve in chloroform, washed with a saturated aqueous solution of sodium hydrogen carbonate three times, and further washed with water three times. Then, the chloroform layer was dehydrated using sodium sulfate, and after filtration, the solvent of the obtained solution was removed under reduced pressure. The residue thus obtained was dissolved in methanol (400 mL) at 60°C, and after the obtained solution was cooled to 4°C and recrystallized, the crystal was filtered and dried to obtain a glutamic acid derivative represented by the following formula (a1) (Glu2C16) as a white solid (9.5 g, yield of 80%).

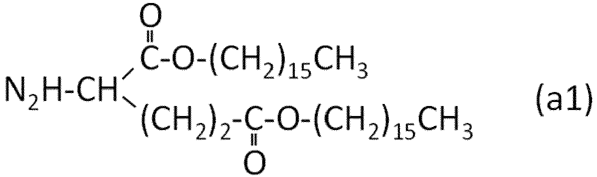

The obtained Glu2C16 (1.49 g, 2.5 mmol) was dissolved in a mixed solution (mixing ratio of 1: 1 (v/v)) of chloroform (7.5 mL) and THF (7.5 mL) and mixed in a recovery flask with a volume of 50 mL, and anhydrous succinic acid (0.374 g, 3.74 mmol) was added to the mixture, followed by stirring at 23°C for 12 hours to obtain a reaction solution. The solvent of the obtained reaction solution was removed under reduced pressure, and after the residue was dissolved in a mixed solution (mixing ratio of 1:5 (v/v)) of ethanol and acetone, and the solution thus obtained was cooled at 4°C for 3 hours and recrystallized. The crystal thus obtained was filtered through a glass filter (G4), and the filtered product was dissolved in chloroform. After the solvent of the obtained solution was removed under reduced pressure, the residue was redissolved in tert-butyl alcohol, and the solution thus obtained was freeze-dried to obtain DHSG represented by the following formula (a2) as a white powder (1376 mg, 1.98 mmol, yield of 79%).

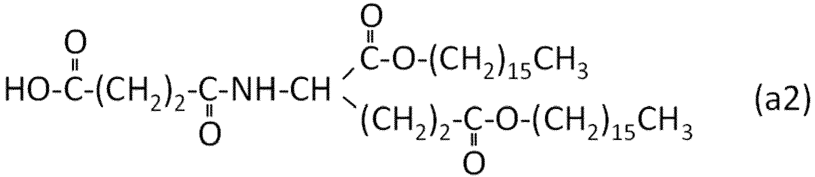

### (2) Synthesis of Carboxylic Acid-Type Lipid Represented by Formula (b2) (Asp-DHSG)

In accordance with the following procedures, an aspartic acid residue was introduced into the hydrophilic moiety (carboxyl group) of DHSG to synthesize a carboxylic acid-type lipid (Asp-DHSG).

In a recovery flask with a volume of 50 mL, DHSG (197 mg, 0.28 mmol), Asp(-OtBu)(-OtBu)·HCl (L-aspartic acid di-tert-butyl ester hydrochloride) (120 mg, 0.42 mmol), PyBOP (1H-benzotriazol-1-yloxytris[pyrrolidin-1-yl]phosphonium-hexafluor ophosphate) (177 mg, 0.34 mmol) and triethylamine (TEA) (57.4 µL, 0.42 mmol) were dissolved in dichloromethane (4 mL), followed by stirring at 23°C for 24 hours to obtain a reaction solution. The reaction solution thus obtained was separated twice using dichloromethane and a saturated aqueous solution of sodium carbonate, and further separated twice using dichloromethane and a saturated aqueous solution of sodium chloride, thus removing water-soluble impurities and acidic impurities to obtain a crude product. After the crude product was dehydrated using sodium sulfate, the product was purified by silica gel column chromatography (developing solvent: hexane/ethyl acetate = 1/1). The purified product thus obtained was redissolved in tert-butyl alcohol, and the solution thus obtained was freeze-dried to obtain Asp(-OtBu)(-OtBu)-DHSG represented by the following formula (b1) as a white powder (160 mg, 0.17 mmol, yield of 61.8%).

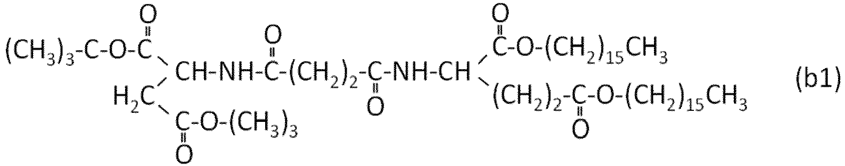

The obtained Asp(-OtBu)(-OtBu)-DHSG (40 mg, 0.044 mmol) was dissolved in a mixture of trifluoroacetic acid (4 mL) and dichloromethane (2 mL) in a recovery flask with a volume of 50 mL, followed by stirring at 23°C for 3 hours, and the reaction solution thus obtained was filtered under reduced pressure using an acid-proof pump. The filtered product was redissolved in tert-butyl alcohol, and the solution thus obtained was freeze-dried to obtain Asp-DHSG represented by the following formula (b2) as a white powder (32 mg, 0.040 mmol, yield of 92.4%).

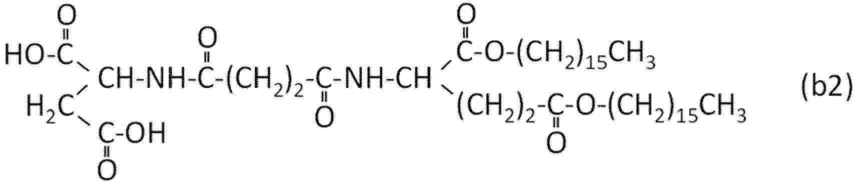

### (3) Synthesis of Carboxylic Acid-Type Lipid Represented by Formula (c2) (Glu-DHSG)

In accordance with the following procedures, a glutamic acid residue was introduced into the hydrophilic moiety (carboxyl group) of DHSG to synthesize a carboxylic acid-type lipid (Glu-DHSG).

In a recovery flask with a volume of 50 mL, DHSG (197 mg, 0.28 mmol), Glu(-OtBu)(-OtBu)·HCl (L-glutamic acid di-tert-butyl ester hydrochloride) (127 mg, 0.43 mmol), PyBOP (182 mg, 0.35 mmol) and TEA (58.8 µL, 0.43 mmol) were dissolved in dichloromethane (4 mL), followed by stirring at 23°C for 24 hours to obtain a reaction solution. The reaction solution thus obtained was separated twice using dichloromethane and a saturated aqueous solution of sodium carbonate, and further separated twice using dichloromethane and a saturated aqueous solution of sodium chloride, thus removing water-soluble impurities and acidic impurities to obtain a crude product. After the crude product was dehydrated using sodium sulfate, the product was purified by silica gel column chromatography (developing solvent: hexane/ethyl acetate = 1/1). The purified product thus obtained was redissolved in tert-butyl alcohol, and the solution thus obtained was freeze-dried to obtain Glu(-OtBu)(-OtBu)-DHSG represented by the following formula (c1) as a white powder (216.4 mg, 0.23 mmol, yield of 79.8%).

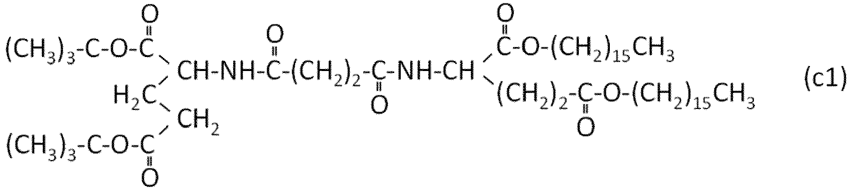

The obtained Glu(-OtBu)(-OtBu)-DHSG (40 mg, 0.043 mmol) was dissolved in a mixture of trifluoroacetic acid (4 mL) and dichloromethane (2 mL) in a recovery flask with a volume of 50 mL, followed by stirring at 23°C for 3 hours, and the reaction solution thus obtained was filtered under reduced pressure using an acid-proof pump. The filtered product was redissolved in tert-butyl alcohol, and the solution thus obtained was freeze-dried to obtain Glu-DHSG represented by the following formula (c2) as a white powder (35 mg, 0.042 mmol, yield of 87.6%).

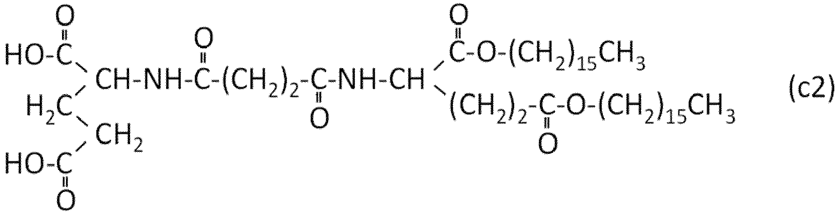

### (4) Synthesis of Carboxylic Acid-Type Lipid Represented by Formula (d2) (AG-DHSG)

In accordance with the following procedures, a peptide residue (AG residue) composed of two aspartic acid residues and one glutamic acid residue was introduced into the hydrophilic moiety (carboxyl group) of DHSG to synthesize a carboxylic acid-type lipid (AG-DHSG).

In a recovery flask with a volume of 50 mL, Glu-DHSG (57.4 mg, 0.069 mmol), Asp(-OtBu)(-OtBu)·HCl (58.9 mg, 0.209 mmol), PyBOP (86.9 mg, 0.167 mmol) and TEA (30 µL, 0.209 mmol) were dissolved in dichloromethane (4 mL), followed by stirring at 23°C for 72 hours to obtain a reaction solution. The reaction solution thus obtained was separated twice using dichloromethane and a saturated aqueous solution of sodium carbonate, and further separated twice using dichloromethane and a saturated aqueous solution of sodium chloride, thus removing water-soluble impurities and acidic impurities to obtain a crude product. After the crude product was dehydrated using sodium sulfate, the product was purified by silica gel column chromatography (developing solvent: hexane/ethyl acetate = 1/2). The purified product thus obtained was redissolved in tert-butyl alcohol, and the solution thus obtained was freeze-dried to obtain Asp(-OtBu)(-OtBu)-Glu-DHSG represented by the following formula (d1) as a white powder (38.9 mg, 0.03 mmol, yield of 44.0%).

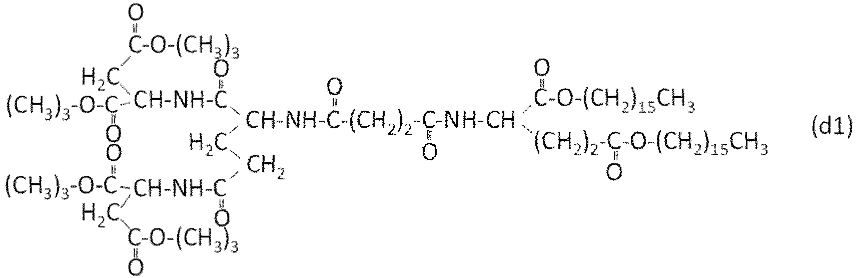

The obtained Asp(-OtBu)(-OtBu)-Glu-DHSG (35 mg, 0.027 mmol) was dissolved in a mixture of trifluoroacetic acid (4 mL) and dichloromethane (2 mL) in a recovery flask with a volume of 50 mL, followed by stirring at 23°C for 3 hours, and the reaction solution thus obtained was filtered under reduced pressure using an acid-proof pump. The filtered product was redissolved in tert-butyl alcohol, and the solution thus obtained was freeze-dried to obtain AG-DHSG represented by the following formula (d2) as a white powder (23 mg, 0.021 mmol, yield of 80.0%).

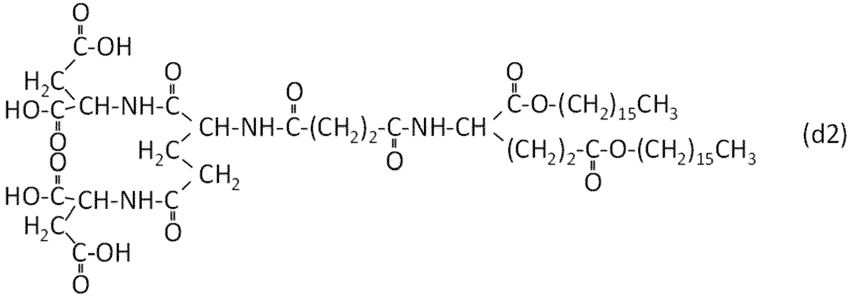

### (5) Synthesis of Carboxylic Acid-Type Lipid Represented by Formula (e2)

In accordance with the following procedures, a carboxylic acid-type lipid represented by formula (e2) was synthesized. This carboxylic acid-type lipid can be used as a starting material when the carboxylic acid-type lipid of the present invention in which M is M₀-NH- is synthesized. In other words, by introducing an amino acid residue, such as aspartic acid residue and a glutamic acid residue, and a peptide residue, such as an AG residue, into the hydrophilic moiety (carboxyl group) of this carboxylic acid-type lipid in the same manner as mentioned above, the carboxylic acid-type lipid of the present invention in which M is M₀-NH- can be synthesized.

L-glutamic acid (1.47 g, 10 mmol) and t-butoxycarbonyl anhydride (2.62 g, 12 mmol) were dissolved in a mixed solution of dioxane (20 mL), water (10 mL) and 1N NaOH (10 mL), followed by stirring at 25°C for 6 hours to obtain a reaction solution. The obtained reaction solution was concentrated under reduced pressure to 10 mL, and after an aqueous 5% potassium hydrogen sulfate solution was added until pH became 2.4, the solution was washed with ethyl acetate three times, and further washed with water three times. After the ethyl acetate layer was dehydrated with sodium sulfate, the solvent was removed under reduced pressure, the residue was dissolved in hexane, and the solution thus obtained was cooled at 4°C and recrystallized. The crystal thus obtained was filtered, and the filtered product was dried to obtain a branched compound 1 in which the amino group is protected by a protecting group (t-butoxycarbonyl group (Boc group)) as a white solid (1.85 g, yield of 75%).

The obtained branched compound 1 (0.49 g, 2 mmol) and N,N'-dicyclohexylcarbodiimide (DCC) (0.82 g, 4 mmol) were dissolved in chloroform, followed by stirring at 4°C for 1 hour to obtain a mixture. The obtained mixture was added dropwise to a chloroform solution in which a glutamic acid derivative (Glu2C16) (2.98 g, 5 mmol) and triethylamine (0.20 g, 2 mmol) were dissolved to obtain a reaction solution. The obtained reaction solution was stirred at 25°C for 6 hours, followed by filtration through a glass filter (G4), and the filtrate was concentrated under reduced pressure, and reprecipitated using methanol and purified. After the precipitate was filtered, the filtered product was purified by silica gel column chromatography (developing solvent: chloroform/methanol = 6/1 (v/v)) to obtain a branched compound 2 (1.40 g, yield of 50%).

The obtained branched compound 2 (1.40 g, 1 mmol) was dissolved in trifluoroacetic acid (TFA), followed by stirring for 1 hour to remove the protecting group (Boc group). The solution thus obtained was dissolved in methanol, followed by cooling at 4°C and recrystallization. The crystal thus obtained was filtered, and the filtered product was dried to obtain a branched compound 3 represented by the following formula (e1) (1.17 g, yield of 90%).

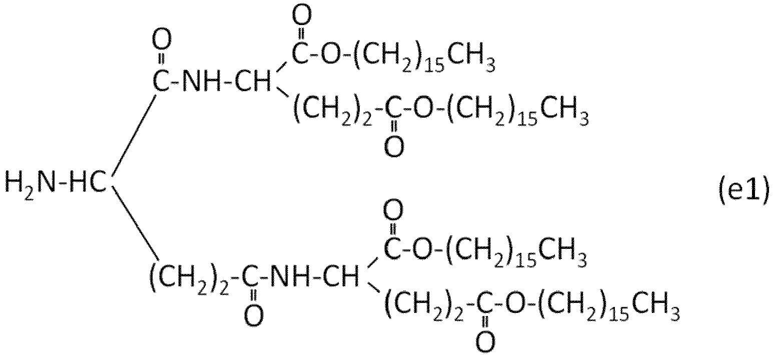

The obtained branched compound 3 (1.17 g, 0.9 mmol) was dissolved in a mixed solution (mixing ratio of 1: 1 (v/v)) of chloroform and tetrahydrofuran and mixed, and anhydrous succinic acid (130 g, 1.35 mmol) was added to the mixture, followed by stirring for 5 hours to obtain a reaction solution. The solvent of the obtained reaction solution was removed under reduced pressure, and after the residue was dissolved in a mixed solution (mixing ratio of 1:5 (v/v)) of ethanol and acetone, and the solution thus obtained was cooled at 4°C and recrystallized. The crystal thus obtained was filtered, and the filtered product was dried to obtain a carboxylic acid-type lipid represented by the following formula (e2) as a white solid (0.95 g, yield of 75%).

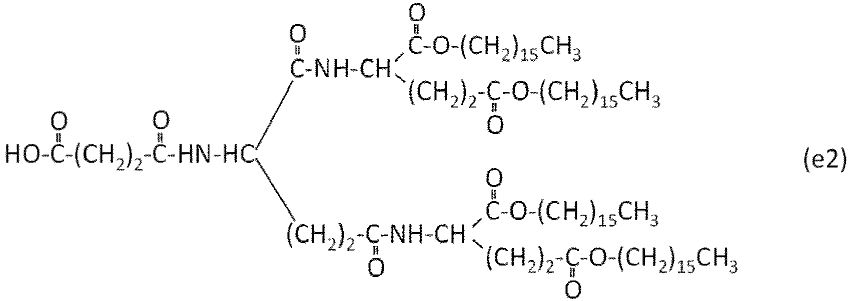

### [Examples 1 to 6 and 9 to 10, Reference Examples 7 to 8 and Comparative Example 1]

### (1) Preparation of Liposome

In accordance with the following procedures, a liposome was prepared. In this case, together with the carboxylic acid-type lipid obtained by the synthesis method mentioned above, the following lipids (commercially available products) were used. Cholesterol is hereinafter sometimes expressed as "Choi."
DPPC (1,2-dipalmitoyl-sn-glycero-3-phosphatidylcholine, manufactured by NIPPON FINE CHEMICAL CO., LTD.)
DPPG (1,2-dipalmitoyl-sn-glycero-3-phosphatidylglycerol, manufactured by NOF CORPORATION)
DPPS (1,2-dipalmitoyl-sn-glycero-3-phosphatidylserine, manufactured by NOF CORPORATION)
Cholesterol (manufactured by NIPPON FINE CHEMICAL CO., LTD.)
   PEG-DSPE (1,2-distearoyl-sn-glycero-3-phosphatidylethanolamine-N-[monom ethoxypoly(ethyleneglycol)], manufactured by NOF CORPORATION)

The lipids were mixed in the molar ratio shown in Table 1 to obtain a lipid mixture. The obtained lipid mixture was dissolved in tert-butyl alcohol, and the solution thus obtained was freeze-dried for 12 hours to obtain a lipid powder. In Examples 1 to 8 and Comparative Example 1, the obtained lipid powder was hydrated using DPBS (Dulbecco's PBS, 3wt%) at 25°C for 12 hours, and the particle diameter of the liposome was controlled by the extrusion method (pore diameter of 450 nm × 2, pore diameter of 220 nm × 2, pore diameter of 200 nm × 1) to obtain a liposome dispersion liquid. In Examples 9 and 10, the obtained lipid powder was sonicated using an HEPES buffer (20 mM) at 50°C for 1 hour to obtain a liposome dispersion liquid.

In Example 1, using DPPC, cholesterol, DHSG and PEG-DSPE (DPPC:cholesterol:DHSG:PEG-DSPE = 5:5:1:0.033 (molar ratio)), a liposome of the inventive example (L551DHSG) was obtained. In Example 2, using DPPC, cholesterol, DHSG and PEG-DSPE (DPPC:cholesterol:DHSG:PEG-DSPE = 5:5:5:0.045 (molar ratio)), a liposome of the inventive example (L555DHSG) was obtained. In Example 3, using DHSG (not using a lipid other than DHSG), a liposome of the inventive example (LDHSG) was obtained. In Example 4, using DPPC, cholesterol, Asp-DHSG and PEG-DSPE (DPPC:cholesterol:Asp-DHSG:PEG-DSPE = 5:5:5:0.045 (molar ratio)), a liposome of the inventive example (L555Asp-DHSG) was obtained. In Example 5, using DPPC, cholesterol, Glu-DHSG and PEG-DSPE (DPPC:cholesterol:Glu-DHSG:PEG-DSPE = 5:5:5:0.045 (molar ratio)), a liposome of the inventive example (L555Glu-DHSG) was obtained. In Example 6, using DPPC, cholesterol, AG-DHSG and PEG-DSPE (DPPC:cholesterol:AG-DHSG:PEG-DSPE = 5:5:5:0.045 (molar ratio)), a liposome of the inventive example (L555AG-DHSG) was obtained. In Reference Example 7, using DPPC, cholesterol, DPPG and PEG-DSPE (DPPC: cholesterol: DPPG: PEG-DSPE = 5:5:5:0.045 (molar ratio)), a liposome of the reference example (L555DPPG) not according to the invention was obtained. In Reference Example 8, using DPPC, cholesterol, DPPS and PEG-DSPE (DPPC: cholesterol: DPPS: PEG-DSPE = 5:5:5:0.045 (molar ratio)), a liposome of the reference example (L555DPPS) not according to the invention was obtained. In Example 9, using cholesterol and Asp-DHSG (cholesterol:Asp-DHSG = 5:5 (molar ratio)), a liposome of the inventive example (L055(Asp)) was obtained. In Example 10, using cholesterol and AG-DHSG (DPPC:cholesterol:AG-DHSG = 5:10 (molar ratio)), a liposome of the inventive example (L05[10](AG)) was obtained. In Comparative Example 1, using DPPC, cholesterol and PEG-DSPE (DPPC:cholesterol:PEG-DSPE = 5:5:5:0.045 (molar ratio)), a liposome of the comparative example not including a carboxylic acid-type lipid (L555DHSG) was obtained.

**[Table 1]**

| | DPPC | Chol | DHSG | Glu-DHSG | Asp-DHSG | AG-DHSG | DPPG | DPPS | PEG-DSPE |
|---|---|---|---|---|---|---|---|---|---|
| Example 1 (L551DHSG) | 5 | 5 | 1 | 0 | 0 | 0 | 0 | 0 | 0.033 |
| Example 2 (L555DHSG) | 5 | 5 | 5 | 0 | 0 | 0 | 0 | 0 | 0.045 |
| Example 3 (LDHSG) | 0 | 0 | 5 | 0 | 0 | 0 | 0 | 0 | 0 |
| Example 4 (L555Asp-DHSG) | 5 | 5 | 0 | 0 | 5 | 0 | 0 | 0 | 0.045 |
| Example 5 (L555Glu-DHSG) | 5 | 5 | 0 | 5 | 0 | 0 | 0 | 0 | 0.045 |
| Example 6 (L555AG-DHSG) | 5 | 5 | 0 | 0 | 0 | 5 | 0 | 0 | 0.045 |
| Reference Example 7 (L555DPPG) | 5 | 5 | 0 | 0 | 0 | 0 | 5 | 0 | 0.045 |
| Reference Example 8 (L555DPPS) | 5 | 5 | 0 | 0 | 0 | 0 | 0 | 5 | 0.045 |
| Example 9 (L055(Asp)) | 0 | 5 | 0 | 0 | 5 | 0 | 0 | 0 | 0 |
| Example 10 (L05[10](AG)) | 0 | 5 | 0 | 0 | 0 | 10 | 0 | 0 | 0 |
| Comparative Example 1 (L550DHSG) | 5 | 5 | 0 | 0 | 0 | 0 | 0 | 0 | 0.033 |

### (2) Measurement of Mean Particle Diameter of Liposome

In accordance with the following procedures, the mean particle diameter of a liposome was measured.

1 mL of a 0.1 mg/mL liposome dispersion liquid filtered through a 0.2 µm filter was put in a disposable cell in which dust, etc., was removed by an air duster, and using Zetasizer nano (manufactured by Malvern Panalytical Ltd.), the mean particle diameter was measured (25°C, n = 3). The mean particle diameter of a liposome is shown in Table 2.

### (3) Measurement of Zeta Potential of Liposome

In accordance with the following procedures, the zeta potential of a liposome was measured.

In cells for zeta potential measurement (folded capillary cells) (DTS1061, manufactured by Malvern Panalytical Ltd.), 1 mL of a 0.1 mg/mL liposome dispersion liquid was put using a 2.5 mL syringe, and after bubbles in the cells were removed, using Zetasizer nano (manufactured by Malvern Panalytical Ltd.), the zeta potential was measured at pH 7.4 and 25°C (n = 3). The mean zeta potential of a liposome is shown in Table 2.

**[Table 2]**

| | Particle diameter (nm) | Zeta potential (mV) |
|---|---|---|
| Example 1 (L551DHSG) | 259±99 | -10.9±0.1 |
| Example 2 (L555DHSG) | 226±80 | -18.9±1.3 |
| Example 3 (LDHSG) | 305±135 (97%) | -41.6±1.2 |
| | 4840±706 (3%) | |
| Example 4 (L555Asp-DHSG) | 247±97 | -22.0±0.2 |
| Example 5 (L555GIu-DHSG) | 261±115 | -20.2±1.0 |
| Example 6 (L555AG-DHSG) | 219±86 | -35.9±0.2 |
| Reference Example 7 (L555DPPG) | 227±60 | -20.4±0.3 |
| Reference Example 8 (L555DPPS) | 225±52 | -15.2±0.4 |
| Example 9 (L055(Asp)) | 316±262 | -75.8±2.9 |
| Example 10 (L05[10](AG)) | 211±133 | -79.6±8.5 |
| Comparative Example 1 (L550DHSG) | 253±94 | -2.8±1.1 |

### (4) Evaluation of Activated Platelet Aggregation Capacity

In accordance with the following procedures, a platelet sample used for evaluation of the activated platelet aggregation capacity was prepared.

Using a 18G winged needle and a 20 mL syringe, about 15 mL of blood was collected from a guinea pig (Hartley, male, 8 weeks old, body weight of 450 g, manufactured by Japan SLC, Inc.) by cardiopuncture, and was divided into two equal parts, which were contained in two 14 mL tubes, respectively. Then, 3.8% sodium citrate was added and mixed so that the volume thereof was 1/10 of the volume of whole blood, followed by slowly stirring twice using a polyethylene dropper to obtain a mixture. Then, the obtained mixture was centrifuged (600 rpm, room temperature, 15 minutes) to recover the platelet-rich plasma (PRP) of the supernatant. Then, the blood after recovery of PRP was centrifuged again (2000 rpm, room temperature, 10 minutes) to recover the platelet-poor plasma (PPP) of the supernatant. Using an automated hematology analyzer, the platelet count of the recovered PRP and PPP was measured, and PRP, PPP and an HEPES-Tyrode buffer were mixed so that the platelet concentration was 2.0 × 10⁵/µL to obtain a platelet sample.

Using the obtained platelet sample and a liposome dispersion liquid, observation and fluorescence quantitative determination of a fluorescently labeled liposome in a platelet aggregate were performed in accordance with the following procedures.

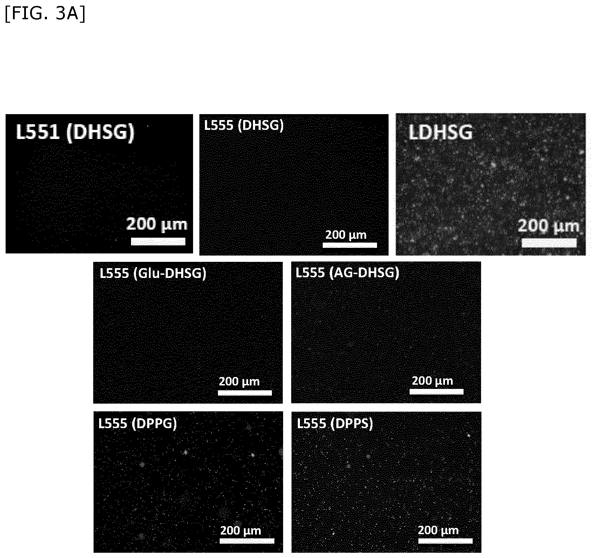

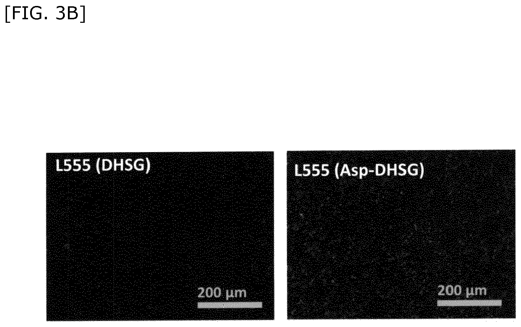

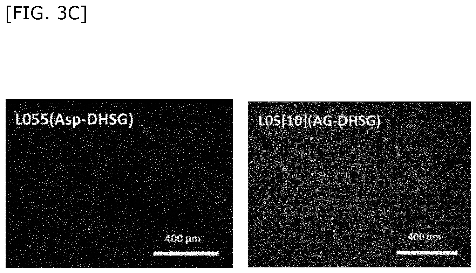

In a 96-well glass bottom plate, 50 µL of a guinea pig-derived platelet sample (platelet concentration: 2.0 × 10⁵/µL) prepared by the abovementioned method and a liposome dispersion liquid (200 µM, 5 µL) containing a liposome labeled with a fluorescence substance DiO (3,3'-dioctadecyloxacarbocyanine perchlorate ) or DiD (1,1'-dioctadecyl-3,3,3',3'-tetramethylindodicarbocyanine perchlorate) were mixed, followed by allowing to stand at room temperature for 2 minutes. If necessary, ADP (1 µM, 5 µL) that activates platelets was added, followed by allowing to stand at room temperature for 4 minutes, and then, using 8% formalin (60 µL, final concentration of 4%), the mixture was fixed at room temperature for 30 minutes, and after completion of fixation, the fixed mixture was washed with an HEPES-Tyrode buffer (100 µL) three times to obtain a platelet aggregate. Using a fluorescence microscope (20-fold or 60-fold), the fluorescently labeled liposome in the obtained platelet aggregate was observed. The results of observation (fluorescence micrographs) of the platelet aggregate obtained by using the liposome dispersion liquid are shown in FIGS. 3A, 3B and 3C. In Example 1 (L551DHSG), Example 3 (LDHSG), Example 5 (L555Glu-DHSG), Example 6 (L555AG-DHSG), Reference Example 7 (L555DPPG) not according to the invention, Reference Example 8 (L555DPPS) not according to the invention, Example 9 (L055(Asp)) and Example 10 (L05[10](AG)), DiD was used as a label (FIG. 3A, FIG. 3C). In Example 2 (L555DHSG), DiO and DiD were used as labels (FIG. 3A, FIG. 3B). In Example 4 (L555Asp-DHSG), DiO was used as a label (FIG. 3B). Using ImageJ, the fluorescence intensity of the fluorescently labeled liposome in each of platelet aggregates shown in FIGS. 3A, 3B and 3C was measured. The results of measurement of the fluorescence intensity are shown in Table 3. The value of the fluorescence intensity in Table 3 is a relative value when the fluorescence intensity in the case of using Example 2 (L555DHSG) is regarded as 1.

**[Table 3]**

| | Fluorescence intensity (mean ± standard deviation) (relative value when the value of Example 2 is regarded as 1) |
|---|---|
| Example 1 (L551DHSG) | 0.17±0.18 |
| Example 2 (L555DHSG) | 1^{<}**^{>} |
| Example 3 (LDHSG) | 8.23±3.39** |
| Example 4 (L555Asp-DHSG) | 2.11±0.63 ^{a)} |
| Example 5 (L555GIu-DHSG) | 1.81±0.31** |
| Example 6 (L555AG-DHSG) | 2.95±0.60** |
| Reference Example 7 (L555DPPG) | 4.36±2.75** |
| Reference Example 8 (L555DPPS) | 2.41±1.99** |
| Example 9 (L055(Asp)) | 1.48±0.06 ^{a)} |
| Example 10 (L05[10](AG)) | 19.91±2.54 ^{a)} |
| Comparative Example 1 (L550DHSG) | 0.06±0.12** |

| | |
|---|---|
| * represents the fact that the fluorescence intensity is statistically significantly higher than that of Comparative Example 1 (L550DHSG) (P < 0.05, t-test). ** represents the fact that the fluorescence intensity is statistically significantly higher than that of Comparative Example 1 (L550DHSG) (P < 0.01, t-test). <**> represents the fact that the fluorescence intensity is statistically significantly higher than that of Comparative Example 1 (L550DHSG) (P < 0.01, paired t-test). a) represents the mean ± standard deviation when one sample was measured three times. | |

As shown in Table 3, the liposomes of Examples 1 to 6 and 9 to 10 and of Reference Examples 7 to 8 not according to the invention had higher fluorescence intensity in the platelet aggregate than that of the liposome of Comparative Example 1. This represents the fact that the liposomes of Examples 1 to 6 and 9 to 10 and of Reference Examples 7 to 8 not according to the invention have superior platelet aggregation accelerating capacity to that of the liposome of Comparative Example 1. Particularly, the liposomes of Examples 2 to 5, and of Reference Examples 7 and 8 not according to the invention had significantly higher fluorescence intensity in the platelet aggregate than that of the liposome of Comparative Example 1. This represents the fact that the liposomes of Examples 2 to 5, and of Reference Examples 7 and 8 not according to the invention have significantly superior platelet aggregation accelerating capacity to that of the liposome of Comparative Example 1.

### (5) Evaluation of Hemostatic Capacity of Poly-L-lactic Acid Resin Hemostatic Material

In accordance with the following procedures, the hemostatic capacity of a poly-L-lactic acid resin hemostatic material was evaluated.

### (Fabrication of Base Complex)

In accordance with the following procedures, a base complex having a cellulose sponge (an example of the support member) and a fiber sheet made of a poly-L-lactic acid resin formed on the cellulose sponge (an example of the base) was fabricated.

A thermoplastic resin composition consisting of 80% by mass of a poly-L-lactic acid resin (PLLA resin, weight average molecular weight (Mw): 80,000, melting point (Tm): 169°C, melt flow rate (MFR): 78 g/10 minutes) vacuum-dried at 80°C for 24 hours and 20% by mass of polyethylene glycol (manufactured by Wako Pure Chemical Industries, Ltd., weight average molecular weight (Mw): 6,000) was supplied to an electrically grounded extruder, melt-kneaded at a spinning temperature of 300°C, and extruded from a spinning nozzle. At this time, assist air at 380°C was blown toward the resin fluid ejected from the spinning nozzle, and a voltage of 10 kV was applied by an electrode independent from the side of the nozzle, followed by blowing a molten product of the thermoplastic resin composition mentioned above on a cellulose sponge (manufactured by Toray Fine Chemicals Co., Ltd., thickness of 0.5 mm) for 10 seconds, thus obtaining a base complex having the cellulose sponge (an example of the support member) and a fiber sheet made of a PLLA resin formed on the cellulose sponge (an example of the base).

### (Measurement of Mean Fiber Diameter)

The mean diameter of a fiber constituting the fiber sheet was measured by the following procedures.

A sample measuring 5 mm per side collected from the fiber sheet was photographed with a scanning electron microscope (model S-3500N, manufactured by Hitachi, Ltd.) at 3,000-fold magnification. Using image processing software (WINROOF (registered trademark)), the diameter of 50 single fibers randomly extracted from the photographs of the sample obtained was measured to one decimal place in a unit of µm, and the value was rounded off to the nearest integer to calculate the fiber diameter. Sampling was performed five times, and the diameter of 50 single fibers for each sampling was calculated. Then, the sum of the diameter of a total of 250 single fibers was divided by the total number (250), thus calculating the mean fiber diameter (µm) as a simple mean. The mean fiber diameter of the above fiber sheet fabricated was 1.4 µm.

### (Measurement of Basis Weight)

The basis weight of the fiber sheet was measured by the procedure in accordance with JIS L 1913: 1998 6.2.

The base complex was allowed to stand under conditions of 20°C and a relative humidity of 65% for 24 hours, and the humidity was controlled. Then, a sample measuring 2 cm per side was collected from a plurality of points of the base complex, and fiber sheet pieces were detached from the sample. The weight (g) of each fiber sheet piece was measurement, and the basis weight (weight per 1 m² (g/m²)) of each fiber sheet piece was calculated. Sampling was performed 10 times, and the mean of the basis weight of 10 fiber sheet pieces was calculated, and this mean was regarded as a basis weight (g/m²) of the fiber sheet. The basis weight of the fiber sheet was 20 g/m².

### (Fabrication of Hemostatic Material)

The base complex fabricated in accordance with the abovementioned procedures was die-cut with a metal punch to obtain a cylindrical base complex with a diameter of 13 mm. On the base part (fiber sheet part) of the obtained cylindrical base complex, 66.7 µL each of the tert-butyl alcohol solutions with a concentration 30 mg/mL of Examples 1 to 4 and 7 to 8 was sprayed, followed by freeze-drying at -40°C for 12 hours, thus fabricating a hemostatic material. The hemostatic materials fabricated using the tert-butyl alcohol solutions of Examples 1 to 4 and Reference Examples 7 to 8 not according to the invention are hereinafter referred to as hemostatic materials A1 to A4 of the inventive example and as hemostatic materials A7 to A8 of the reference example not according to the invention, respectively. A hemostatic material fabricated in the same manner as mentioned above except that 66.7 µL of tert-butyl alcohol is sprayed in place of the tert-butyl alcohol solution is referred to as control hemostatic material.

### (Evaluation of Hemostatic Capacity)

A guinea pig (Slc: Hartley, 8 weeks old, male, manufactured by Japan SLC, Inc.) under 3% isoflurane anesthesia was fixed in a supine position, and the abdominal wall was incised at the midline to expose the left lobe of liver. A part of the incised marginal region was removed with scissors so that the width of the section was 10 mm, thus making bleeding from the entire wound surface. A hemostatic material was attached to cover the wound surface, and astriction was performed with fingers. The hemostatic material was detached every 2 minutes, and the condition of an issue of blood from the wound surface was confirmed. When an issue of blood was observed within 5 seconds after detachment, the detached hemostatic material was attached to the wound surface again. At the time point at which no issue of blood was observed during 5 seconds after detachment, hemostasis was regarded as successful, and the time from attachment of the hemostatic material to hemostasis (hemostasis time) was measured. A nonwoven fabric was laid around the liver before removal of the liver to absorb blood issued during hemostasis, and the amount of bleeding was calculated from the difference in weight of the hemostatic material and the nonwoven fabric that absorbed blood between before and after surgery. The hemostasis time (min) is shown in Table 4, and the amount of bleeding (mg) is shown in Table 5. Hemostasis was performed three times for each of hemostatic materials, and the mean of the hemostasis time and the amount of bleeding for three times was regarded as the hemostasis time and the amount of bleeding of each hemostatic material.

**[Table 4]**

| | Hemostasis time (min) (mean ± standard deviation) |
|---|---|
| Control hemostatic material (Dulbecco's PBS) | 10.7±0.94 |
| Hemostatic material A1(L551DHSG) | 10.0±1.63 |
| Hemostatic material A2 (L555DHSG) | 8.0±0.0 |
| Hemostatic material A3 (LDHSG) | 4.7±0.94 |
| Hemostatic material A4 (L555Asp-DHSG) | 7.3±0.94 |
| Reference Hemostatic material A7 (L555DPPG) | 7.3±0.94 |
| Reference Hemostatic material A8 (L555DPPS) | 6.7±0.94 |

As shown in Table 4, when the hemostatic materials A1 to A4of the inventive examples and the Reference hemostatic materials A7 and A8 were used, the hemostasis time was significantly shortened compared with when the control hemostatic material was used (hemostatic material A2: p < 0.05, hemostatic material A3: p < 0.01, hemostatic material A4: p < 0.01, Reference hemostatic material A7: p < 0.01, Reference hemostatic material A8: p < 0.01, t-test for all). This represents the fact that the hemostatic material of the inventive example has superior hemostatic capacity to that of the control hemostatic material.

**[Table 5]**

| | Amount of bleeding (mg) (mean ± standard deviation) |
|---|---|
| Control hemostatic material (Dulbecco's PBS) | 196.16±54.45 |
| Hemostatic material A1 (L551DHSG) | 157.61±51.48 |
| Hemostatic material A2 (L555DHSG) | 137.21±31.36 |
| Hemostatic material A3 (LDHSG) | 41.50±19.75 |
| Hemostatic material A4 (L555Asp-DHSG) | 100.93±25.18 |
| Reference Hemostatic material A7 (L555DPPG) | 124.62±53.22 |
| Reference Hemostatic material A8 (L555DPPS) | 51.17±11.62 |

As shown in Table 5, when the hemostatic materials A1 to A4 of the inventive examples and the Reference hemostatic materials A7 and A8 were used, the amount of bleeding was suppressed compared with when the control hemostatic material was used. Particularly, when the hemostatic materials A3 and A4 of the inventive examples and the Reference hemostatic material A8 were used, the amount of bleeding was significantly suppressed compared with when the control hemostatic material was used (hemostatic material A3: p < 0.01, hemostatic material A4: p < 0.05, Reference hemostatic material A8: p < 0.01, t-test for all). This represents the fact that the hemostatic material of the inventive example has superior hemostatic capacity to that of the control hemostatic material.

### (6) Evaluation of Hemostatic Capacity of Collagen Hemostatic Material

In accordance with the following procedures, the hemostatic capacity of a collagen hemostatic material was evaluated.

### (Fabrication of Collagen Hemostatic Material)

In accordance with the following procedures, a hemostatic material was fabricated.

A commercially available collagen-based absorbable topical hemostatic material Integran (registered trademark)(KOKEN CO., LTD.) was die-cut with a metal punch to obtain a circular fiber sheet made of collagen with a diameter of 13 mm. On the obtained fiber sheet, 100 µL each of the liposome dispersion liquids with a concentration of 20 mg/mL of Examples 1 and 2 and the liposome dispersion liquid of Comparative Example 1 was sprayed, followed by freeze-drying at -40°C for 12 hours, thus fabricating a hemostatic material having a fiber sheet made of collagen (an example of the base) and a liposome supported on the fiber sheet made of collagen. The hemostatic material fabricated using the liposome dispersion liquid of Example 1 is hereinafter referred to as "hemostatic material B1 of the inventive example," the hemostatic material fabricated using the liposome dispersion liquid of Example 2 is hereinafter referred to as "hemostatic material B2 of the inventive example," and the hemostatic material fabricated using the liposome dispersion liquid of Comparative Example 1 is hereinafter referred to as "hemostatic material of the comparative example." As a control, a hemostatic material fabricated in the same manner as mentioned above except that 100 µL of Dulbecco's PBS is sprayed in place of the liposome dispersion liquid (hereinafter referred to as "control hemostatic material") was used. As a control, an untreated gauze and an untreated fiber sheet made of collagen were used.

### (Evaluation of Hemostatic Capacity)

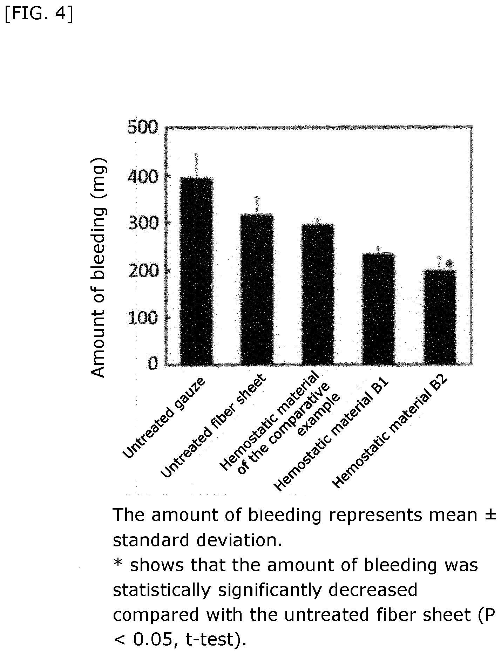

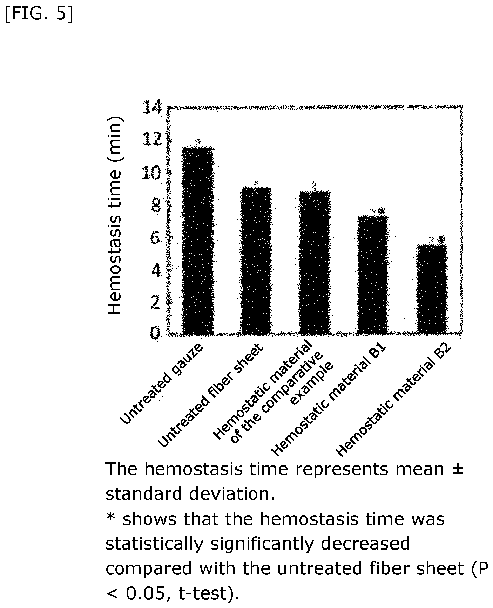

The hemostasis time and the amount of bleeding of each hemostatic material were evaluated in accordance with the same procedures as mentioned above. The amount of bleeding and the hemostasis time of each hemostatic material are shown in FIG. 4 and FIG. 5, respectively. The amount of bleeding and the hemostasis time were calculated as mean values of the hemostasis time and the amount of bleeding in 8 guinea pigs after hemostasis was performed in 8 guinea pigs for each hemostatic material.

For each hemostatic material, the platelet attachment rate was evaluated in accordance with the following procedures.

The platelet attachment rate was evaluated as follows. The hemostatic material was put in a sheet holder, and 1.5 mL of blood was added from the top of a syringe. After the blood passed through the hemostatic material by a free fall, the passed blood was recovered. Platelet counts in the added blood and the recovered blood were measured using an automated hematology analyzer, and the platelet counts before and after passing were compared to calculate the platelet attachment rate (%) of the hemostatic material (platelet attachment rate (%) = (platelet count before passing - platelet count after passing/platelet count before passing) × 100).

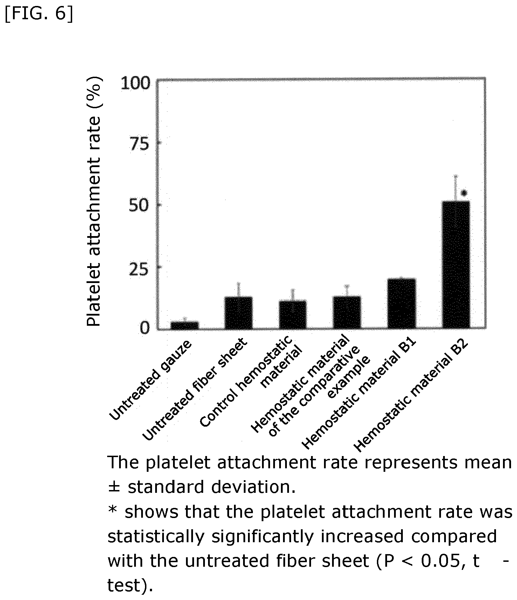

The platelet attachment rate of each hemostatic material is shown in FIG. 6. The platelet attachment rate was calculated as a mean value of the platelet attachment rate in 3 guinea pigs after hemostasis was performed in 3 guinea pigs for each hemostatic material.

As shown in FIG. 4, the hemostatic materials B1 and B2 were able to suppress the amount of bleeding compared with the untreated gauze, the untreated fiber sheet made of collagen and the hemostatic material of the comparative example. Particularly, the hemostatic materials B1 and B2 were able to significantly suppress the amount of bleeding compared with the untreated fiber sheet made of collagen (P < 0.05, t-test).

As shown in FIG. 5, the hemostatic materials B1 and B2 were able to shorten the hemostasis time compared with the untreated gauze, the untreated fiber sheet made of collagen and the hemostatic material of the comparative example. Particularly, the hemostatic material B2 was able to significantly shorten the hemostasis time compared with the untreated fiber sheet made of collagen (P < 0.05, t-test).

As shown in FIG. 6, the hemostatic materials B1 and B2 were able to increase the platelet attachment rate compared with the untreated gauze, the untreated fiber sheet made of collagen, the control hemostatic material and the hemostatic material of the comparative example. Particularly, the hemostatic material B2 was able to significantly increase the platelet attachment rate compared with the untreated fiber sheet made of collagen (P < 0.05, t-test).

### [Reference Examples 11 and 12 not according to the invention]

### (1) Obtainment of Phospholipid

DPPA sodium salt (1,2-dipalmitoyl-sn-glycero-3-phosphatidic acid, sodium salt) was purchased from NOF CORPORATION (COATSOME MA-6060LS). In accordance with the following methods, -O-P(=O)(-OH)(-O⁻Na⁺) in the DPPA sodium salt was converted to -P(=O)(-OH)(-OH), thus fabricating acidic DPPA. Anionic DPPA has higher solubility in t-butyl alcohol than that of the DPPA sodium salt.

After 150 mg of the DPPA sodium salt and 15 mL of a mixed solution of chloroform and methanol (volume of chloroform:volume of methanol = 6:4) were mixed, 56.0 µL of 4M hydrochloric acid was added, followed by sonication at 50°C for 30 minutes. After sonication, the solvent was evaporated, followed by addition of 5 mL of t-butyl alcohol, and then NaCl was removed by filtration using a filter to obtain acidic DPPA. By reacting the DPPA sodium salt with equimolar hydrochloric acid, it is possible to obtain acidic DPPA.

### (2) Fabrication of Hemostatic Material

In the same manner as mentioned above, a base complex having a cellulose sponge and a fiber sheet made of a poly-L-lactic acid resin formed on the cellulose sponge was fabricated, and the fabricated base complex was die-cut with a metal punch to obtain a cylindrical base complex with a diameter of 13 mm. On the base part (fiber sheet part) of the obtained cylindrical base complex, 66.7 µL each of a tert-butyl alcohol dispersion liquid with a concentration of 30 mg/mL of the DPPA sodium salt and a tert-butyl alcohol solution with a concentration of 30 mg/mL of acidic DPPA of the DPPA sodium salt was sprayed, followed by drying, thus fabricating a hemostatic material. In Reference Example 11, a hemostatic material was fabricated using a tert-butyl alcohol dispersion liquid of DPPA sodium salt (hereinafter referred to as "hemostatic material C1 of the reference example"), and in Reference Example 12, a hemostatic material was fabricated using a tert-butyl alcohol solution of acidic DPPA (hereinafter referred to as "hemostatic material C2 of the reference example").

### (3) In Vivo Hemostasis Study using Guinea Pigs

In the same manner as mentioned above, an *in vivo* hemostasis study using guinea pigs was performed, and by quantitatively determining the hemostasis time and the amount of bleeding, the hemostatic capacity of the hemostatic materials C1 and C2 was evaluated. As a control, the hemostasis time and the amount of bleeding of a base complex before supporting a lipid were also quantitatively determined. The results are shown in Table 6 and Table 7. As shown in Table 6 and Table 7, the hemostasis time of the hemostatic materials C1 and C2 was significantly shorter than the hemostasis time of the base complex before supporting a lipid, and the amount of bleeding of the hemostatic materials C1 and C2 was lower than the amount of bleeding of the base complex before supporting a lipid.

**[Table 6]**

| Hemostasis time (min) | Base complex before supporting a lipid | Hemostatic material C1 (DPPA sodium salt) | Hemostatic material C2 (acidic DPPA) |
|---|---|---|---|
| Mean | 10.7 | 8.7 | 7.3 |
| SD | 0.94 | 2.5 | 0.9 |

**[Table 7]**

| Amount of bleeding (mg) | Base complex before supporting a lipid | Hemostatic material C1 (DPPA sodium salt) | Hemostatic material C2 (acidic DPPA) |
|---|---|---|---|
| Mean | 196.2 | 135.4 | 128.2 |
| SD | 54.5 | 79.6 | 64.8 |

### [Examples 13 to 16]

### (1) Synthesis of Lipid

DHSG was synthesized in the same manner as mentioned above, and using the synthesized DHSG, Asp-DHSG, Glu-DHSG and AG-DHSG were synthesized in the same manner as mentioned above.

### (2) Fabrication of Hemostatic Material

DHSG, Asp-DHSG, Glu-DHSG and AG-DHSG were fabricated in the same manner as mentioned above. In the same manner as mentioned above, a base complex having a cellulose sponge and a fiber sheet made of a poly-L-lactic acid resin formed on the cellulose sponge was fabricated, and the fabricated base complex was die-cut with a metal punch to obtain a cylindrical base complex with a diameter of 13 mm. On the base part (fiber sheet part) of the obtained cylindrical base complex, 66.7 µL each of tert-butyl alcohol solutions with a concentration 30 mg/mL of DHSG, Asp-DHSG, Glu-DHSG and AG-DHSG was sprayed, followed by drying, thus fabricating a hemostatic material. In Example 13, using a tert-butyl alcohol solution of DHSG, a hemostatic material (hereinafter referred to as "hemostatic material D1 of the inventive example") was fabricated; in Example 14, using a tert-butyl alcohol solution of Asp-DHSG, a hemostatic material (hereinafter referred to as "hemostatic material D2 of the inventive example") was fabricated; in Example 15, using a tert-butyl alcohol solution of Glu-DHSG, a hemostatic material (hereinafter referred to as "hemostatic material D3 of the inventive example") was fabricated; and in Example 16, using a tert-butyl alcohol solution of AG-DHSG, a hemostatic material (hereinafter referred to as "hemostatic material D4 of the inventive example") was fabricated.

### (3) Evaluation of Platelet Aggregation Capacity of Hemostatic Material (In Vitro)

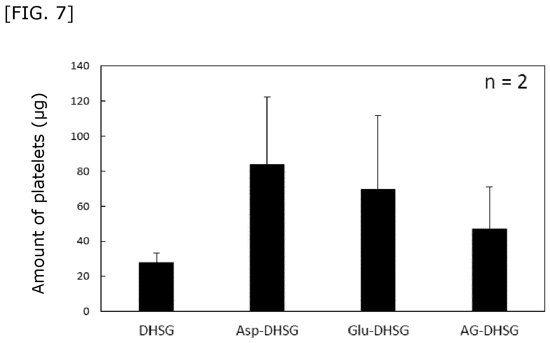

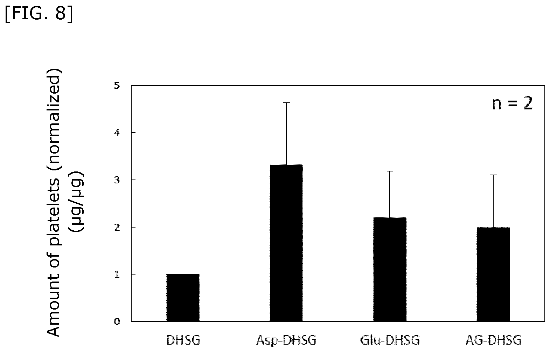

From the hemostatic materials D1, D2, D3 and D4, a base part (fiber sheet part) supporting DHSG, Asp-DHSG, Glu-DHSG and AG-DHSG, respectively, was taken out. To a 12-well plate, the base part (fiber sheet part) that was taken out and 1 mL of guinea pig-derived PRP (2.0 × 10⁵/µL) prepared by the abovementioned method were added. Thereafter, ADP (1 µM, 100 µL) that activates platelets was added, and after allowing to stand at room temperature for 5 minutes, the mixture was washed with 1 mL of DPBS twice. Thereafter, in order to dissolve platelets attached to the base part (fiber sheet part), 500 µL of 0.5% Triton X was added, followed by allowing to stand at room temperature for 1 hour to obtain a platelet lysate. To a 96-well plate, 10 µL of the platelet lysate prepared by the abovementioned method and 150 µL of Pierce (trademark) 660 nm Protein Assay Kit were added, followed by allowing to stand for 5 minutes. By measuring absorbance at 660 nm using a microplate reader, proteins were quantitatively determined, and the determination results were used as an index of the count of platelets which were not washed away and were attached to the fiber sheet. The results are shown in FIG. 7 and FIG. 8. The results in FIG. 8 are relative values when the platelet count in the case of using the hemostatic material D1 is regarded as 1. In FIG. 7 and FIG. 8, "DHSG" represents results on the hemostatic material D1, "Asp-DHSG" represents results on the hemostatic material D2, "Glu-DHSG" represents results on the hemostatic material D3, and "AG-DHSG" represents results on the hemostatic material D4.

As shown in FIG. 7 and FIG. 8, it was shown that each of the hemostatic material D2 supporting Asp-DHSG, the hemostatic material D3 supporting Glu-DHSG, and the hemostatic material D4 supporting AG-DHSG has a higher count of tightly attached platelets than that of the hemostatic material D1 supporting DHSG.

In both cases of when guinea pig-derived PRP was added to the base part (fiber sheet part) before supporting a lipid and when guinea pig-derived PRP was not added to the base part (fiber sheet part) before supporting a lipid, no proteins were detected, and as a result of washing, no platelets were detected. When DPPC in place of DHSG, Asp-DHSG, Glu-DHSG and AG-DHSG was supported on the base part (fiber sheet part), as a result of washing, no platelets were detected. This is considered to be due to the fact that DPPC does not show a negative charge *in vivo.*

### (4) In Vivo Hemostasis Study using Guinea Pigs

In the same manner as mentioned above, an *in vivo* hemostasis study using guinea pigs was performed, and by quantitatively determining the hemostasis time and the amount of bleeding, the hemostatic capacity of the hemostatic materials D2, D3 and D4 were evaluated. As a control, the hemostasis time and the amount of bleeding of a base complex before supporting a lipid were also quantitatively determined. The results are shown in Table 8 and Table 9. As shown in Table 8 and Table 9, the hemostasis time of the hemostatic materials D2, D3 and D4 was significantly shorter than the hemostasis time of the base complex before supporting a lipid, and the amount of bleeding of the hemostatic materials D2, D3 and D4 was lower than the amount of bleeding of the base complex before supporting a lipid.

**[Table 8]**

| Hemostasis time (min) | Base complex before supporting a lipid | Hemostatic material D2 (Asp-DHSG) | Hemostatic material D3 (Glu-DHSG) | Hemostatic material D4 (AG-DHSG) |
|---|---|---|---|---|
| Mean | 10.7 | 6.8 | 6.0 | 4.8 |
| SD | 0.9 | 2.7 | 2.2 | 1.0 |

**[Table 9]**

| Amount of bleeding (mg) | Base complex before supporting a lipid | Hemostatic material D2 (Asp-DHSG) | Hemostatic material D3 (Glu-DHSG) | Hemostatic material D4 (AG-DHSG) |
|---|---|---|---|---|
| Mean | 196.2 | 92.7 | 63.7 | 39.3 |
| SD | 54.5 | 52.7 | 26.7 | 23.3 |

### [Examples 19 to 22 and Reference Examples 17, 18, 23 and 24 not according to the invention]

In the same manner as mentioned above, a base complex having a cellulose sponge and a fiber sheet made of a poly-L-lactic acid resin formed on the cellulose sponge was fabricated, and the fabricated base complex was die-cut with a metal punch to obtain a cylindrical base complex with a diameter of 13 mm. On the base part (fiber sheet part) of the obtained cylindrical base complex, 66.7 µL of a lipid solution with a concentration 30 mg/mL was sprayed, followed by drying, thus fabricating a hemostatic material. As the lipid solution, a tert-butyl alcohol dispersion liquid of DPPA sodium salt (Reference Example 17 not according to the invention), a tert-butyl alcohol solution of acidic DPPA (Reference Example 18 not according to the invention), a tert-butyl alcohol solution of DHSG (Example 19), a tert-butyl alcohol solution of Asp-DHSG (Example 20), a tert-butyl alcohol solution of Glu-DHSG (Example 21), a tert-butyl alcohol solution of AG-DHSG (Example 22), a tert-butyl alcohol dispersion liquid of DMPS sodium salt (Reference Example 23 not according to the invention) and a tert-butyl alcohol dispersion liquid of DSPG sodium salt (Reference Example 24 not according to the invention) were used. The DPPA sodium salt, acidic DPPA, DHSG, Asp-DHSG, Glu-DHSG and AG-DHSG were prepared in the same manner as mentioned above. As DMPS (1,2-dimyristoyl-sn-glycero-3-phospho-L-serine, sodium salt), one manufactured by NOF CORPORATION was used, and as DSPG (1,2-disteanoyl-sn-glycero-3-[phospho-rac-(1-glycerol)] (sodium salt)), one manufactured by NIPPON FINE CHEMICAL CO., LTD. was used. A hemostatic material fabricated using the tert-butyl alcohol solution of DPPA sodium salt is hereinafter referred to as "hemostatic material E1 of the reference example," a hemostatic material fabricated using the tert-butyl alcohol solution of acidic DPPA is hereinafter referred to as "hemostatic material E2 of the reference example," a hemostatic material fabricated using the tert-butyl alcohol solution of DHSG is hereinafter referred to as "hemostatic material E3 of the inventive example," a hemostatic material fabricated using the tert-butyl alcohol solution of Asp-DHSG is hereinafter referred to as "hemostatic material E4 of the inventive example," a hemostatic material fabricated using the tert-butyl alcohol solution of Glu-DHSG is hereinafter referred to as "hemostatic material E5 of the inventive example," a hemostatic material fabricated using the tert-butyl alcohol solution of AG-DHSG is hereinafter referred to as "hemostatic material E6 of the inventive example," a hemostatic material fabricated using the tert-butyl alcohol solution of DMPS sodium salt is hereinafter referred to as "hemostatic material E7 of the reference example," and a hemostatic material fabricated using the tert-butyl alcohol solution of DSPG sodium salt is hereinafter referred to as "hemostatic material E8 of the reference example."

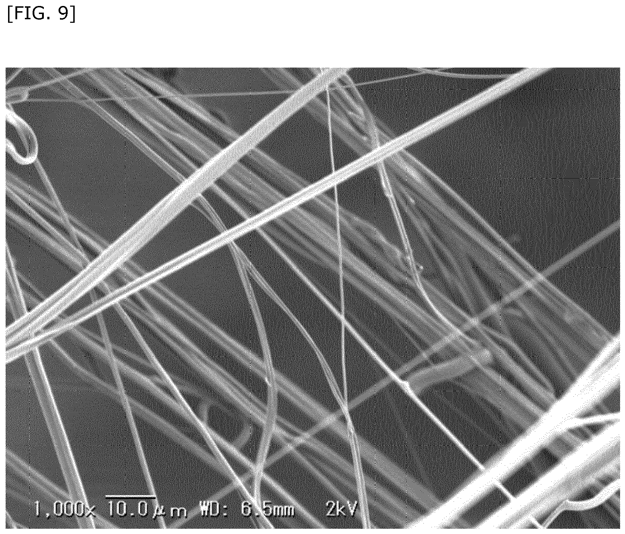

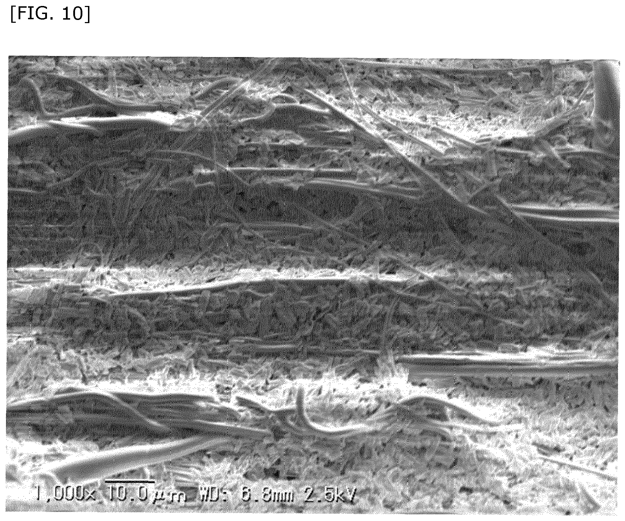

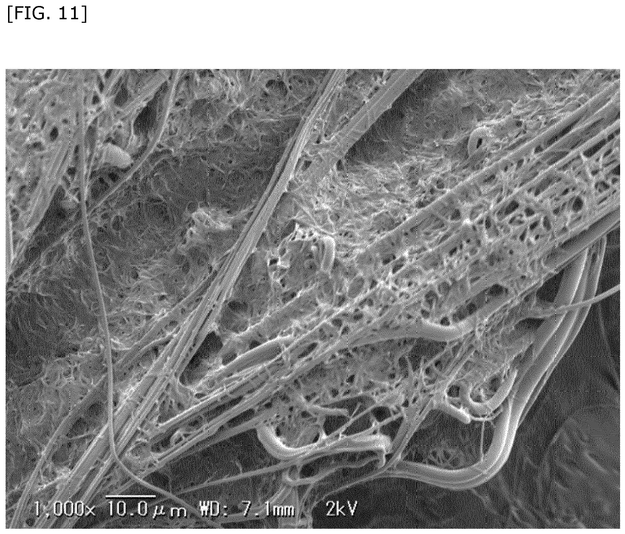

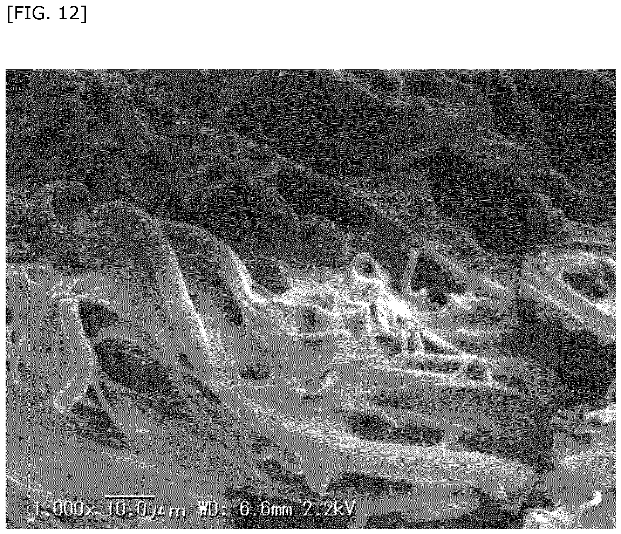

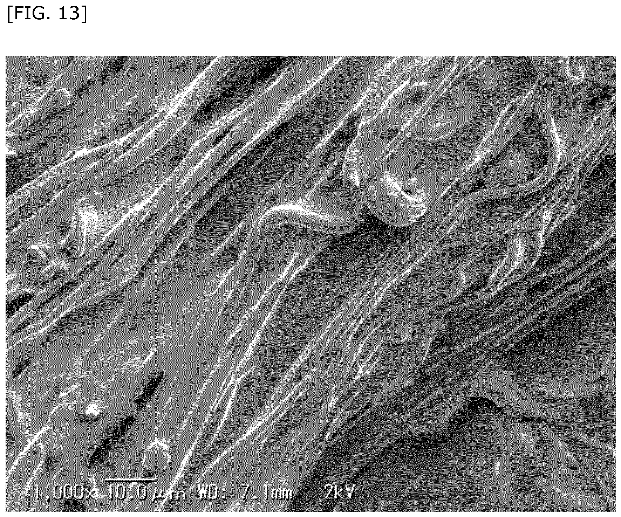

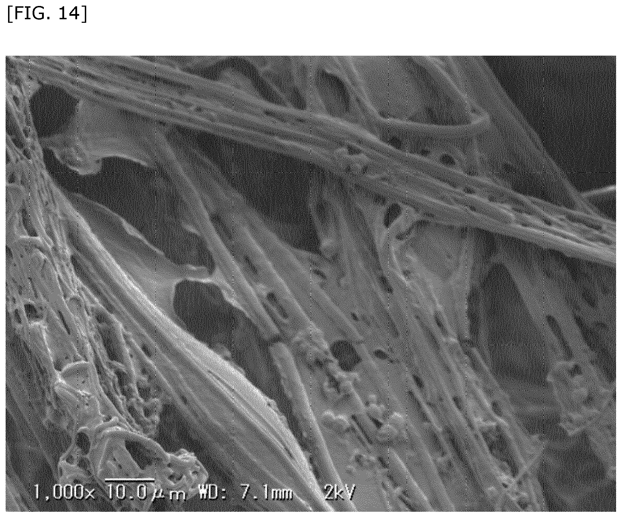

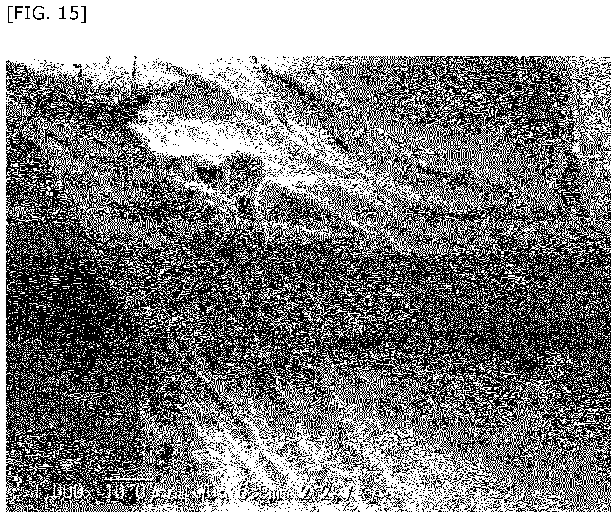

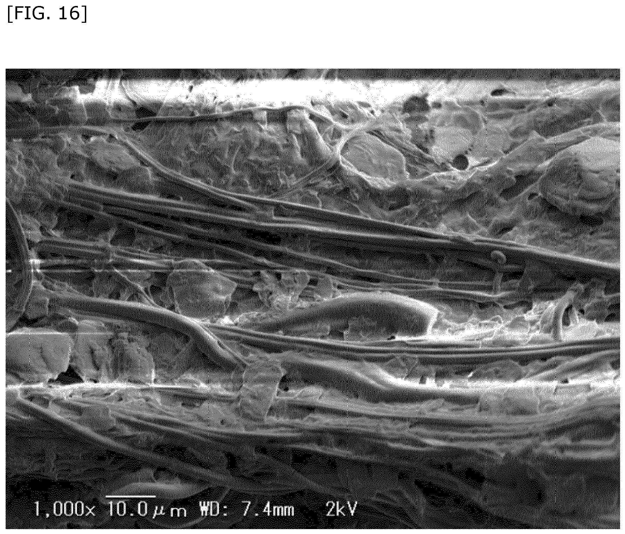

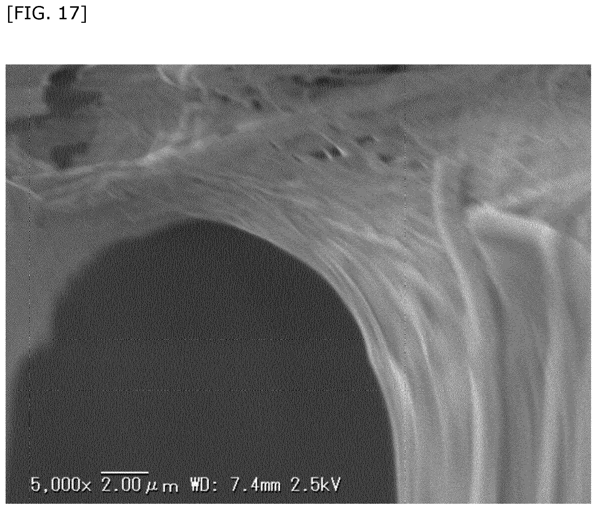

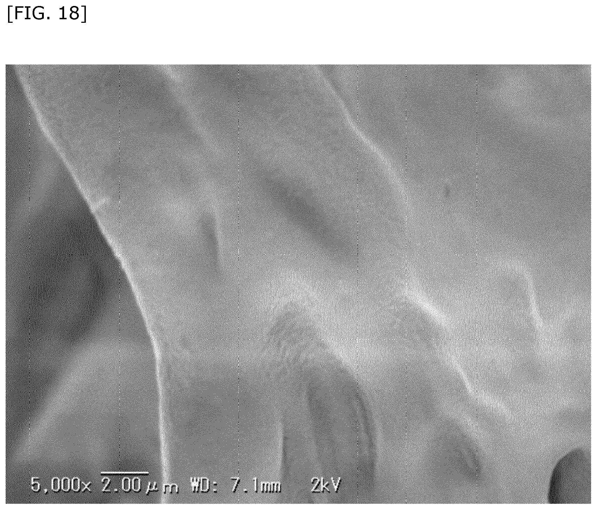

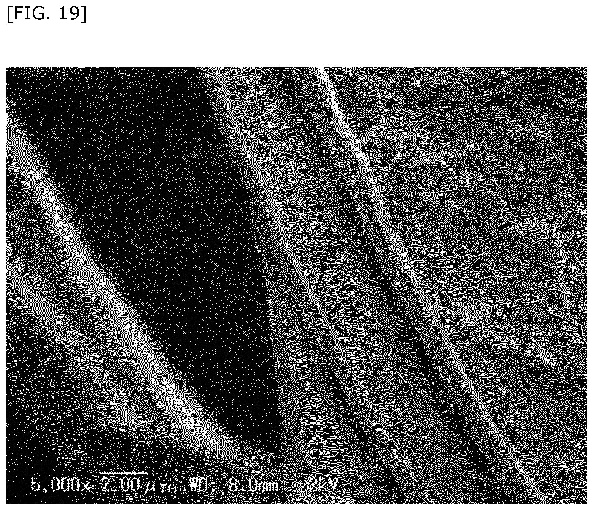

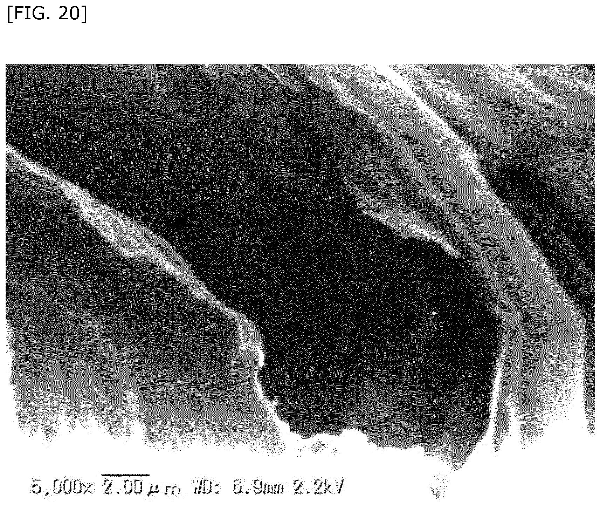

When a base complex before supporting a lipid and the base parts (fiber sheet parts) of the hemostatic materials E1 to E8 were subjected to ion sputtering treatment (target: Au) and observed with a scanning electron microscope (SEM), in the hemostatic material E1, the lipid was supported on a void of the base in a form of a lipid particle with a diameter of several tens of µm, but in the hemostatic materials E2 to E8, the majority of lipids had a membranous form spreading between fibers. An SEM observation image (× 1,000) of the base complex before supporting a lipid is shown in FIG. 9, and SEM observation images (× 1,000) of the hemostatic materials E2 to E8 are shown in FIG. 10 to FIG. 16, respectively. SEM observation images (× 5,000) of the hemostatic materials E3 to E6 are shown in FIG. 17 to FIG. 20, respectively. The SEM observation images shown in FIG. 17 to FIG. 20 are enlarged views of parts of the SEM observation images shown in FIG. 11 to FIG. 14, respectively. The thickness of the lipid membrane (DHSG) calculated from the SEM observation image shown in FIG. 17 was 131 nm. The thickness of the lipid membrane (Asp-DHSG) calculated from the SEM observation image shown in FIG. 18 was 153 nm. The thickness of the lipid membrane (Glu-DHSG) calculated from the SEM observation image shown in FIG. 19 was 187 nm. The thickness of the lipid membrane (AG-DHSG) calculated from the SEM observation image shown in FIG. 20 was 124 nm.

### REFERENCE SIGNS LIST

- 1:: Base
- 2:: Lipid particle
- 3:: Support member
- 10:: Hemostatic material

## Claims

1. A hemostatic material, comprising a water-insoluble base and a lipid supported on a surface of the base,
wherein the lipid comprises one or two or more anionic lipids,
wherein the one or two or more anionic lipids comprise one or two or more carboxylic acid-type lipids selected from carboxylic acid-type lipids represented by the following formulas (III) to (VI): 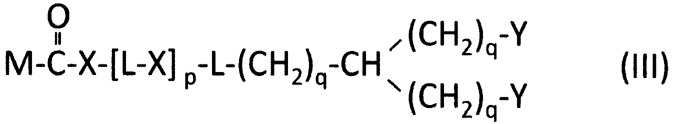 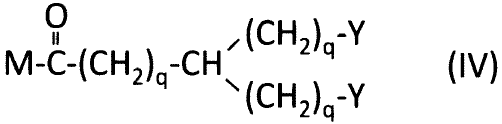 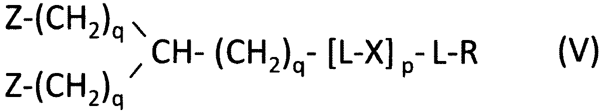 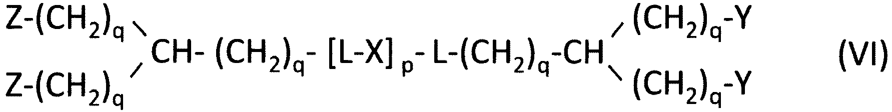
wherein, in formulas (III) to (VI),
M is the same or different and represents HO- or M₀-NH-,
M₀ is the same or different and represents an amino acid residue, an amino acid derivative residue, a peptide residue or a salt thereof, wherein the amino acid residue, the amino acid derivative residue, the peptide residue and the salt thereof can be negatively charged at physiological pH,
R is the same or different and represents a hydrocarbon group having 8 to 30 carbon atoms,
L is the same or different and represents -CO-O-, -O-CO-, - CO-NH-, -NH-CO-, -CO-S-, -S-CO- or -S-S-,
X is the same or different and represents a hydrocarbon group having 1 to 6 carbon atoms,
p is the same or different and represents an integer of 0 to 4,
q is the same or different and represents an integer of 0 to 8,
Y is the same or different and represents a branched chain composed of a branched chain body and one or more groups Y2 that are bonded to the branched chain body, or represents a straight chain composed of one group Y2, wherein the branched chain body is composed of one or more units Y1, wherein each unit Y1 is represented by the following formula (VII): 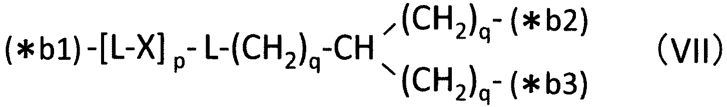 and wherein each group Y2 is represented by the following formula (VIII):
(*b4)-[L-X]ₚ-L-R (VIII)
wherein, in formulas (VII) and (VIII),
R, L, X, p and q are the same as defined above,
(*b1), (*b2) and (*b3) represent a bond of each unit Y1,
(*b4) represents a bond of each group Y2,
the bond (*b1) of each unit Y1 is bonded to (CH₂)_{q} in formula (III), (IV) or (VI), or is bonded to a bond (*b2) or (*b3) of another unit Y1 constituting the branched chain body, and
the bond (*b4) of each group Y2 is bonded to (CH₂)_{q} in formula (III), (IV) or (VI), or is bonded to a bond (*b2) or (*b3) of any unit Y1 constituting the branched chain body,
Z is the same or different and represents a branched chain composed of a branched chain body and one or more groups Z2 that are bonded to the branched chain body, or represents a straight chain composed of one group Z2, wherein the branched chain body is composed of one or more units Z1, wherein each unit Z1 is represented by the following formula (IX): 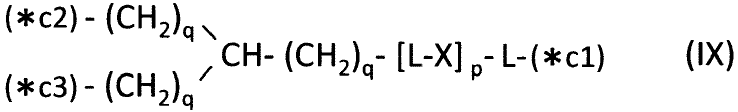 and wherein each group Z2 is selected from groups represented by the following formulas (X) and (XI): 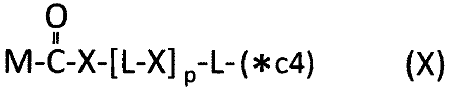 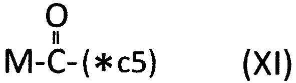
wherein, in formulas (IX), (X) and (XI),
M, L, X, p and q are the same as defined above,
(*c1), (*c2) and (*c3) represent a bond of each unit Z1,
(*c4) and (*c5) represent a bond of each group Z2,
the bond (*c1) of each unit Z1 is bonded to (CH₂)_{q} in formula (V) or (VI), or is bonded to a bond (*c2) or (*c3) of another unit Z1 constituting the branched chain body, and
the bond (*c4) or (*c5) of each group Z2 is bonded to (CH₂)_{q} in formula (V) or (VI), or is bonded to a bond (*c2) or (*c3) of any unit Z1 constituting the branched chain body.

2. The hemostatic material according to claim 1, wherein the base is a porous base, and the lipid is supported on a surface of a pore of the porous base.

3. The hemostatic material according to claim 2, wherein the lipid accounts for at least a part of the pore of the porous base.

4. The hemostatic material according to claim 2 or 3, wherein the porous base is a fiber base.

5. The hemostatic material according to any one of claims 1 to 4, wherein the amino acid residue represented by M₀ is an acidic amino acid residue or a neutral amino acid residue.

6. The hemostatic material according to claim 5, wherein the acidic amino acid residue is an aspartic acid residue or a glutamic acid residue.

7. The hemostatic material according to any one of claims 1 to 4, wherein the residue of the amino acid derivative represented by M₀ is a residue of a basic amino acid derivative, and an introduced derivatization that the basic amino acid derivative comprises is amidation of an amino group of a side chain of a basic amino acid to a group represented by the following formula: -NH-CO-R₁ wherein -NH- is derived from the amino group of the side chain of the basic amino acid, and R₁ represents a hydrocarbon group.

8. The hemostatic material according to any one of claims 1 to 4, wherein the peptide residue represented by M₀ is a peptide residue comprising one or two or more acidic amino acid residues.

9. The hemostatic material according to claim 8, wherein the peptide residue represented by M₀ is a peptide residue comprising two or more acidic amino acid residues selected from an aspartic acid residue and a glutamic acid residue.

10. The hemostatic material according to claim 9, wherein the peptide residue represented by M₀ is a peptide residue consisting of 2 to 12 acidic amino acid residues selected from an aspartic acid residue and a glutamic acid residue.

11. The hemostatic material according to claim 10, wherein the peptide residue represented by M₀ is a peptide residue represented by the following formula (XII): 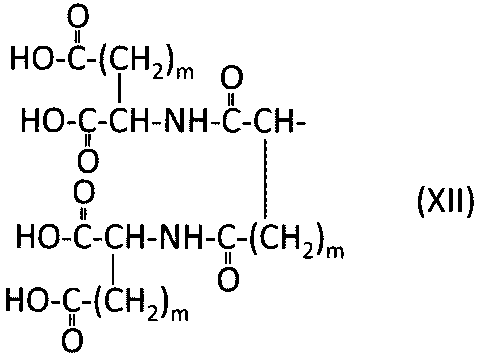 wherein m is the same or different and represents 1 or 2.

12. The hemostatic material according to any one of claims 1 to 11, wherein Y is selected from straight and branched chains represented by the following formulas (XIII), (XIV), (XV) and (XVI) :
(_{*}b)-Y2 (XIII) 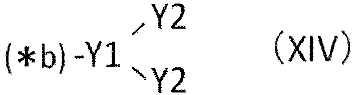 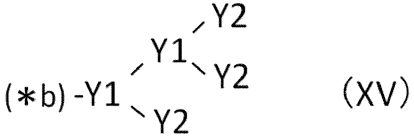 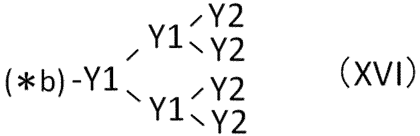
wherein, in formulas (XIII) to (XVI),
Y1 represents one unit Y1,
Y2 represents one group Y2, and
(*b) represents a bond of the unit Y1 bonded to (CH₂)_{q} in formula (III), (IV) or (VI).

13. The hemostatic material according to any one of claims 1 to 12, wherein Z is selected from straight and branched chains represented by the following formulas (XVII), (XVIII), (XIX) and (XX):
Z2-(*c) (XVII) 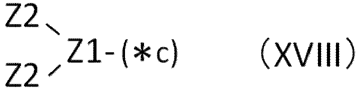 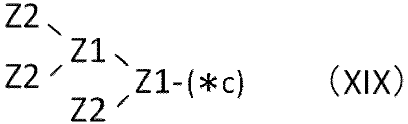 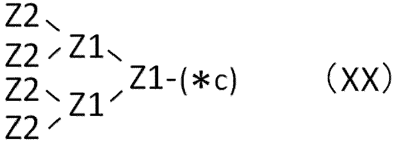
wherein, in formulas (XVII) to (XX),
Z1 represents one unit Z1,
Z2 represents one group Z2, and
(*c) represents a bond of the unit Z1 bonded to (CH₂)_{q} in formula (V) or (VI).

14. The hemostatic material according to any one of claims 1 to 13, wherein the one or two or more anionic lipids comprise one or two or more lipids selected from a phospholipid and a sterol.

15. The hemostatic material according to claims 1 to 14, wherein the lipid is supported on a surface of the base in one or two or more forms selected from a lipid particle, an aggregate of a lipid particle and a lipid membrane.

## Patentansprüche

1. Hämostatisches Material, das eine wasserunlösliche Basis und ein an einer Fläche der Basis gehaltenes Lipid umfasst,
wobei das Lipid ein oder zwei oder mehr anionische Lipide umfasst,
wobei das eine oder die zwei oder die mehr anionischen Lipide ein oder zwei oder mehr carboxylische Lipide des Säure-Typs umfassen, die aus carboxylischen Lipiden des Säure-Typs ausgewählt sind, die durch die folgenden Formeln (III) bis (VI) wiedergegeben werden: 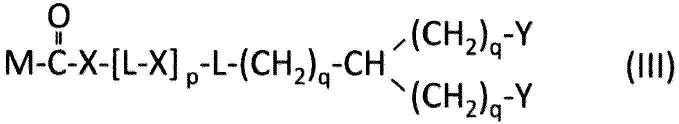 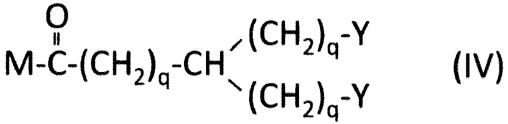 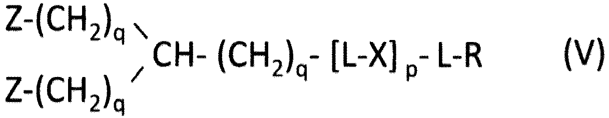 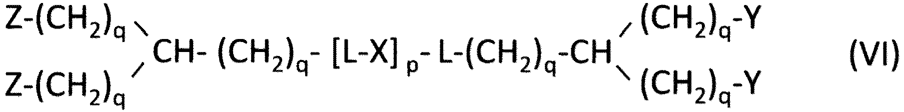
wobei in den Formeln (III) bis (VI):
M gleich oder verschieden ist und HO- oder M₀-NH- wiedergibt,
M₀ gleich oder verschieden ist und einen Aminosäurerest, einen Aminosäurederivatrest, einen Peptidrest oder ein Salz davon wiedergibt, wobei der Aminosäurerest, der Aminosäurederivatrest, der Peptidrest oder das Salz davon bei einem physiologischen pH-Wert negativ geladen werden können,
R gleich oder verschieden ist und eine Kohlenwasserstoffgruppe mit 8 bis 30 Kohlenstoffatomen wiedergibt,
L gleich oder verschieden ist und -CO-O-, -O-CO-, -CO-NH-, -NH-CO-, -CO-S-, -S-CO- oder -S-S- wiedergibt,
X gleich oder verschieden ist und eine Kohlenwasserstoffgruppe mit 1 bis 6 Kohlenstoffatomen wiedergibt.
p gleich oder verschieden ist und eine Ganzzahl von 0 bis 4 wiedergibt,
q gleich oder verschieden ist und eine Ganzzahl von 0 bis 8 wiedergibt,
Y gleich oder verschieden ist und eine verzweigte Kette, die aus einem verzweigten Kettenkörper und einer oder mehreren Gruppen Y2, die mit dem verzweigten Kettenkörper verbunden sind, besteht, wiedergibt oder eine gerade Kette, die aus einer einzelnen Gruppe Y2 besteht, wiedergibt, wobei der verzweigte Kettenkörper aus einer oder mehreren Einheiten Y1 besteht, wobei jede Einheit Y1 durch die folgende Formel (VII) wiedergegeben wird: 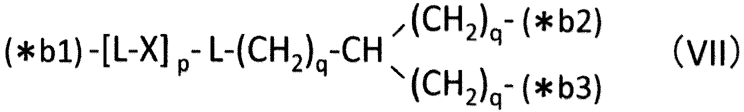
und wobei jede Gruppe Y2 durch die folgende Formel (VIII) wiedergegeben wird:
(*b4)-[L-X]ₚ-L-R (VIII)
wobei in den Formeln (VII) und (VIII):
R, L, X, p und q wie oben definiert sind,
(*b1), (*b2) und (*b3) eine Bindung jeder Einheit Y1 wiedergeben,
(*b4) eine Bindung jeder Gruppe Y2 wiedergibt,
die Bindung (*b1) jeder Einheit Y1 mit (CH₂)_{q} in der Formel (III), (IV) oder (VI) verbunden ist oder mit einer Bindung (*b2) oder (*b3) einer anderen Einheit Y1 des verzweigten Kettenkörpers verbunden ist, und
die Bindung (*b4) jeder Gruppe Y2 mit (CH₂)_{q} in der Formel (III), (IV) oder (VI) verbunden ist oder mit einer Bindung (*b2) oder (*b3) einer beliebigen Einheit Y1 des verzweigten Kettenkörpers verbunden ist,
Z gleich oder verschieden ist und eine verzweigte Kette, die aus einem verzweigten Kettenkörper und einer oder mehreren mit dem verzweigten Kettenkörper verbundenen Gruppen Z2 besteht, wiedergibt oder eine gerade Kette, die aus einer einzelnen Gruppe Z2 besteht, wiedergibt, wobei der verzweigte Kettenkörper aus einer oder mehreren Einheiten Z1 besteht, wobei jede Einheit Z1 durch die folgende Formel (IX) wiedergegeben wird: 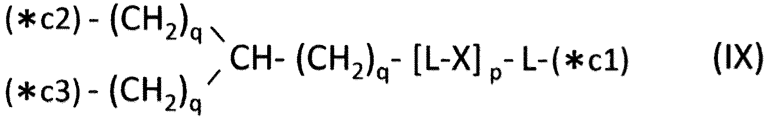
und wobei jede Gruppe Z2 aus Gruppen ausgewählt ist, die durch die folgenden Formeln (X) und (XI) wiedergegeben werden: 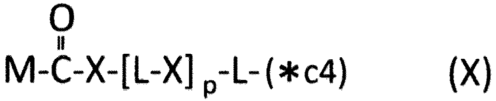 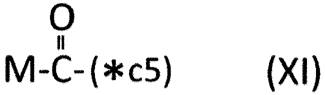
wobei in den Formeln (IX), (X) und (XI):
M, L, X, p und q wie oben definiert sind,
(*c1), (*c2) und (*c3) eine Bindung jeder Einheit Z1 wiedergeben,
(*c4) und (*c5) eine Bindung jeder Gruppe Z2 wiedergeben,
die Bindung (*c1) jeder Einheit Z1 mit (CH₂)_{q} in der Formel (V) oder (VI) verbunden ist oder mit einer Bindung (*c2) oder (*c3) einer anderen Einheit Z1 des verzweigten Kettenkörpers verbunden ist, und
die Bindung (*c4) oder (*c5) jeder Gruppe Z2 mit (CH₂)_{q} in der Formel (V) oder (VI) verbunden ist oder mit einer Bindung (*c2) oder (*c3) einer beliebigen Einheit Z1 des verzweigten Kettenkörpers verbunden ist.

2. Hämostatisches Material nach Anspruch 1, wobei die Basis eine poröse Basis ist und das Lipid an einer Fläche einer Pore der porösen Basis gehalten wird.

3. Hämostatisches Material nach Anspruch 2, wobei sich das Lipid über wenigstens einen Teil der Pore der porösen Basis erstreckt.

4. Hämostatisches Material nach Anspruch 2 oder 3, wobei die poröse Basis eine Faserbasis ist.

5. Hämostatisches Material nach einem der Ansprüche 1 bis 4, wobei der durch M₀ wiedergegebene Aminosäurerest ein saurer Aminosäurerest oder ein neutraler Aminosäurerest ist.

6. Hämostatisches Material nach Anspruch 5, wobei der saure Aminosäurerest ein Aspariginsäurerest oder ein Glutaminsäurerest ist.

7. Hämostatisches Material nach einem der Ansprüche 1 bis 4, wobei der durch M₀ wiedergegebene Rest des Aminosäurederivats ein Rest eines basischen Aminosäurederivats ist und eine eingeführte Derivatisierung in dem basischen Aminosäurederivat eine Amidierung einer Aminogruppe einer Seitenkette einer basischen Aminosäure zu einer durch die Formel -NH-CO-R₁ wiedergegebenen Gruppe ist, wobei -NH- von der Aminogruppe der Seitenkette der basischen Aminosäure abgeleitet ist und R₁ eine Kohlenwasserstoffgruppe wiedergibt.

8. Hämostatisches Material nach einem der Ansprüche 1 bis 4, wobei der durch M₀ wiedergegebene Peptidrest ein Peptidrest ist, der ein oder zwei oder mehr saure Aminosäurereste umfasst.

9. Hämostatisches Material nach Anspruch 8, wobei der durch M₀ wiedergegebene Peptidrest ein Peptidrest ist, der zwei oder mehr saure Aminosäurereste, die aus einem Aspariginsäurerest und einen Glutaminsäurerest ausgewählt sind, umfasst.

10. Hämostatisches Material nach Anspruch 9, wobei der durch M₀ wiedergegebene Peptidrest ein Peptidrest ist, der aus 2 bis 12 sauren Aminosäureresten, die aus einem Aspariginsäurerest und einen Glutaminsäurerest ausgewählt sind, besteht.

11. Hämostatisches Material nach Anspruch 10, wobei der durch M₀ wiedergegebene Peptidrest ein Peptidrest ist, der durch die folgende Formel (XII) wiedergegeben wird: 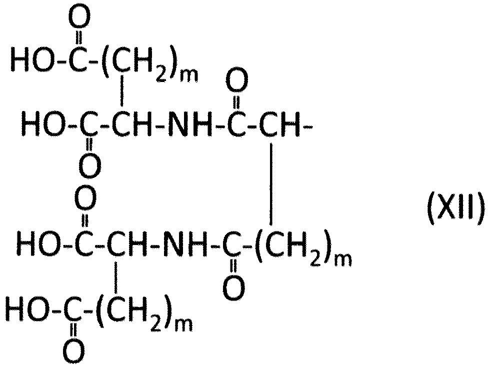 wobei m gleich oder verschieden ist und 1 oder 2 wiedergibt.

12. Hämostatisches Material nach einem der Ansprüche 1 bis 11, wobei Y aus geraden und verzweigten Ketten ausgewählt ist, die durch die folgenden Formeln (XIII), (XIV), (XV) und (XVI) wiedergegeben werden:
(* b) -Y2 (XIII) 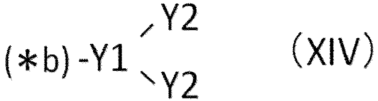 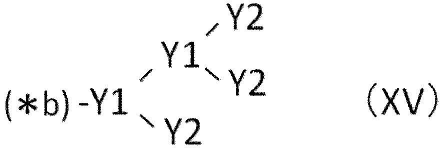 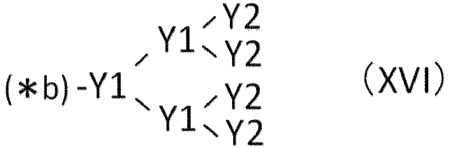
wobei in den Formeln (XIII) bis (XVI):
Y1 eine Einheit Y1 wiedergibt,
Y2 eine Gruppe Y2 wiedergibt, und
(*b) eine Bindung der Einheit Y1, die mit (CH₂)_{q} in der Formel (III), (IV) oder (VI) verbunden ist, wiedergibt.

13. Hämostatisches Material nach einem der Ansprüche 1 bis 12, wobei Z aus geraden und verzweigten Ketten ausgewählt ist, die durch die folgenden Formeln (XVII), (XVIII), (XIX) und (XX) wiedergegeben werden:
Z2-(_{*}c) (XVII) 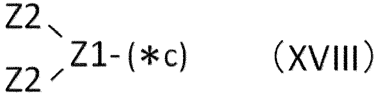 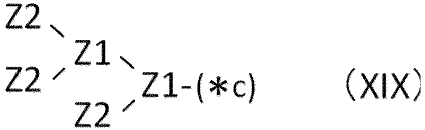 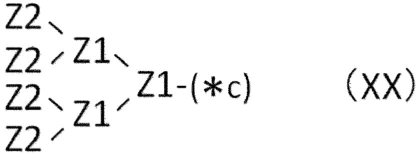
wobei in den Formeln (XVII) bis (XX):
Z1 eine Einheit Z1 wiedergibt,
Z2 eine Gruppe Z2 wiedergibt, und
(*c) eine Bindung der Einheit Z1, die mit (CH₂)_{q} in der Formel (V) oder (VI) verbunden ist, widergibt.

14. Hämostatisches Material nach einem der Ansprüche 1 bis 13, wobei das eine oder die zwei oder die mehr anionischen Lipide ein oder zwei oder mehr Lipide umfassen, die aus Phospholipid und einem Sterin ausgewählt sind.

15. Hämostatisches Material nach einem der Ansprüche 1 bis 14, wobei das Lipid an einer Fläche der Basis in einer oder zwei oder mehr Formen, die aus einem Lipidpartikel, einem Aggregat eines Lipidpartikels und einer Lipidmembran ausgewählt sind, gehalten wird.

## Revendications

1. Matériau hémostatique, comprenant une base insoluble dans l'eau et un lipide supporté sur une surface de la base,
dans lequel le lipide comprend un ou deux lipides anioniques ou plus,
dans lequel les un ou deux lipides anioniques ou plus comprennent un ou deux lipides de type acide carboxylique ou plus, choisis parmi les lipides de type acide carboxylique représentés par les formules (III) à (VI) suivantes : 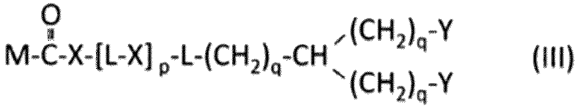 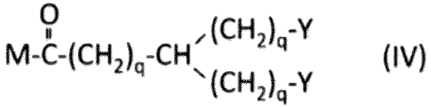 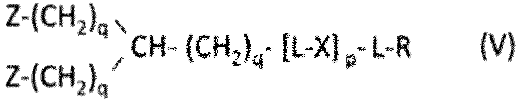 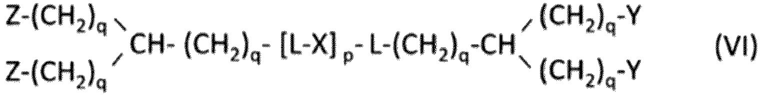
dans lequel, dans les formules (III) à (VI),
M est identique ou différent et représente HO- ou M₀-NH-,
M₀ est identique ou différent et représente un résidu d'acide aminé, un résidu de dérivé d'acide aminé, un résidu de peptide, ou un sel de celui-ci, le résidu d'acide aminé, le résidu de dérivé d'acide aminé, le résidu de peptide, et le sel de celui-ci pouvant être chargés négativement au pH physiologique,
R est identique ou différent et représente un groupe hydrocarboné ayant de 8 à 30 atomes de carbone,
L est identique ou différent et représente -CO-O-, -O-CO-, -CO-NH-, - NH-CO-, -CO-S-, -S-CO- ou -S-S-,
X est identique ou différent et représente un groupe hydrocarboné ayant de 1 à 6 atomes de carbone,
p est identique ou différent et représente un nombre entier de 0 à 4,
q est identique ou différent et représente un nombre entier de 0 à 8,
Y est identique ou différent et représente une chaîne ramifiée composée d'un corps de chaîne ramifiée et d'un ou plusieurs groupes Y2 qui sont liés au corps de chaîne ramifiée, ou représente une chaîne linéaire composée d'un groupe Y2, le corps de chaîne ramifiée étant composé d'une ou plusieurs unités Y1, chaque unité Y1 étant représentée par la formule (VII) suivante : et chaque groupe Y2 étant représenté par la formule (VIII) suivante : 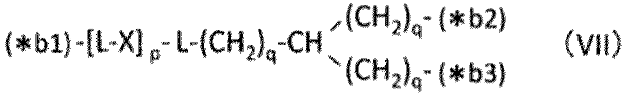
et chaque groupe Y2 étant représenté par la formule (VIII) suivante :
(*b4)-[L-X]ₚ-L-R (VIII)
dans lequel, dans les formules (VII) et (VIII),
R, L, X, p et q sont les mêmes que ceux définis ci-dessus,
(*b1), (*b2) et (*b3) représentent une liaison de chaque unité Y1,
(*b4) représente une liaison de chaque groupe Y2,
la liaison (*b1) de chaque unité Y1 est liée à (CH₂)_{q} dans la formule (III), (IV) ou (VI), ou est liée à une liaison (*b2) ou (*b3) d'une autre unité Y1 constituant le corps de chaîne ramifiée, et
la liaison (*b4) de chaque groupe Y2 est liée à (CH₂)_{q} dans la formule (III), (IV) ou (VI), ou est liée à une liaison (*b2) ou (*b3) de toute unité Y1 constituant le corps de chaîne ramifiée,
Z est identique ou différent et représente une chaîne ramifiée composée d'un corps de chaîne ramifiée et d'un ou plusieurs groupes Z2 qui sont liés au corps de chaîne ramifiée, ou représente une chaîne linéaire composée d'un groupe Z2, le corps de chaîne ramifiée étant composé d'une ou plusieurs unités Z1, chaque unité Z1 étant représentée par la formule (IX) suivante : 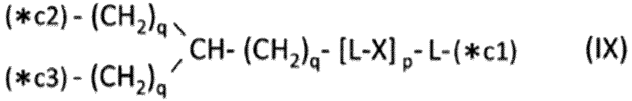 et chaque groupe Z2 étant choisi parmi les groupes représentés par les formules (X) et (XI) suivantes : 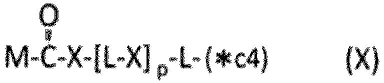 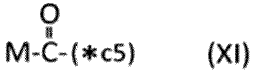
dans lequel, dans les formules (IX), (X) et (XI),
M, L, X, p et q sont les mêmes que ceux définis ci-dessus,
(*c1), (*c2) et (*c3) représentent une liaison de chaque unité Z1,
(*c4) et (*c5) représentent une liaison de chaque groupe Z2, la liaison (*c1) de chaque unité Z1 est liée à (CH₂)_{q} dans la formule (V) ou (VI), ou est liée à une liaison (*c2) ou (*c3) d'une autre unité Z1 constituant le corps de chaîne ramifiée, et
la liaison (*c4) ou (*c5) de chaque groupe Z2 est liée à (CH₂)_{q} dans la formule (V) ou (VI), ou est liée à une liaison (*c2) ou (*c3) de toute unité Z1 constituant le corps de chaîne ramifiée.

2. Matériau hémostatique selon la revendication 1, dans lequel la base est une base poreuse, et le lipide est supporté sur une surface d'un pore de la base poreuse.

3. Matériau hémostatique selon la revendication 2, dans lequel le lipide représente au moins une partie du pore de la base poreuse.

4. Matériau hémostatique selon la revendication 2 ou 3, dans lequel la base poreuse est une base fibreuse.

5. Matériau hémostatique selon l'une quelconque des revendications 1 à 4, dans lequel le résidu d'acide aminé représenté par M₀ est un résidu d'acide aminé acide ou un résidu d'acide aminé neutre.

6. Matériau hémostatique selon la revendication 5, dans lequel le résidu d'acide aminé acide est un résidu d'acide aspartique ou un résidu d'acide glutamique.

7. Matériau hémostatique selon l'une quelconque des revendications 1 à 4, dans lequel le résidu du dérivé d'acide aminé représenté par M₀ est un résidu d'un dérivé d'acide aminé basique, et une dérivatisation introduite que le dérivé d'acide aminé basique comprend est l'amidation d'un groupe amino d'une chaîne latérale d'un acide aminé basique en un groupe représenté par la formule suivante : -NH-CO-R₁ dans laquelle -NH- est dérivé du groupe amino de la chaîne latérale de l'acide aminé basique et R₁ représente un groupe hydrocarboné.

8. Matériau hémostatique selon l'une quelconque des revendications 1 à 4, dans lequel le résidu de peptide représenté par M₀ est un résidu de peptide comprenant un ou deux résidus d'acides aminés acides ou plus.

9. Matériau hémostatique selon la revendication 8, dans lequel le résidu de peptide représenté par M₀ est un résidu de peptide comprenant deux résidus d'acides aminés acides ou plus choisis parmi un résidu d'acide aspartique et un résidu d'acide glutamique.

10. Matériau hémostatique selon la revendication 9, dans lequel le résidu de peptide représenté par M₀ est un résidu de peptide constitué de 2 à 12 résidus d'acides aminés choisis parmi un résidu d'acide aspartique et un résidu d'acide glutamique.

11. Matériau hémostatique selon la revendication 10, dans lequel le résidu de peptide représenté par M₀ est un résidu de peptide représenté par la formule (XII) suivante : 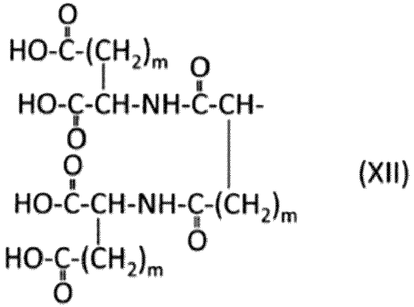 dans laquelle m est identique ou différent et représente 1 ou 2.

12. Matériau hémostatique selon l'une quelconque des revendications 1 à 11, dans lequel Y est choisi parmi les chaînes linéaires et ramifiées représentées par les formules (XIII), (XIV), (XV) et (XVI) suivantes :
(_{*}b)-Y2 (XIII) 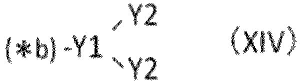 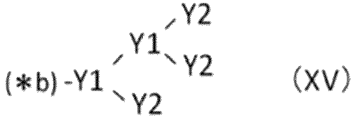 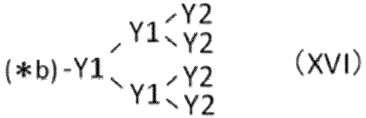
dans lequel, dans les formules (XIII) à (XVI),
Y1 représente une unité Y1,
Y2 représente un groupe Y2, et
(*b) représente une liaison de l'unité Y1 liée à (CH₂)_{q} dans la formule (III), (IV) ou (VI).

13. Matériau hémostatique selon l'une quelconque des revendications 1 à 12, dans lequel Z est choisi parmi les chaînes linéaires et ramifiées représentées par les formules (XVII), (XVIII), (XIX) et (XX) suivantes :
Z2-(*c) (XVII) 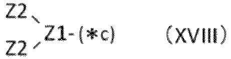 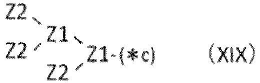 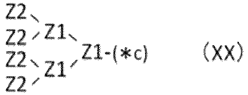
dans lequel, dans les formules (XVII) à (XX),
Z1 représente une unité Z1,
Z2 représente un groupe Z2, et
(*c) représente une liaison de l'unité Z1 liée à (CH₂)_{q} dans la formule (V) ou (VI).

14. Matériau hémostatique selon l'une quelconque des revendications 1 à 13, dans lequel les un ou deux lipides anioniques ou plus comprennent un ou deux lipides ou plus choisis parmi un phospholipide et un stérol.

15. Matériau hémostatique selon les revendications 1 à 14, dans lequel le lipide est supporté sur une surface de la base sous une ou deux formes ou plus choisies parmi une particule lipidique, un agrégat de particule lipidique et une membrane lipidique.
